# EUROPÄISCHE PATENTSCHRIFT

(11) **EP 3 772 337 B1**
(45) Veröffentlichungstag und Bekanntmachung des Hinweises auf die Patenterteilung: **18.10.2023**
(21) Anmeldenummer: 20189290.8
(22) Anmeldetag: 04.08.2020
(51) Int. Cl.: A61B 17/00, A61B 17/29, A61B 18/00, A61B 18/14, A61B 34/00, A61B 34/30, A61B 34/37, A61B 1/00, A61B 1/018

(54) **ENDOSKOPISCHE VORRICHTUNG**
ENDOSCOPIC DEVICE
DISPOSITIF ENDOSCOPIQUE

(30) Priorität: 05.08.2019 DE 102019121038
(43) Veröffentlichungstag der Anmeldung: 10.02.2021
(73) Patentinhaber: Karl Storz SE & Co. KG, 78532 Tuttlingen (DE)
(72) Erfinder: Grüner, Sven, 78532 Tuttlingen (DE); Stefan, Jochen, 78532 Tuttlingen (DE)

(56) Entgegenhaltungen:
- EP-A1- 3 087 944
- US-A1- 2016 066 939
- US-A1- 2016 074 028

## Beschreibung

### Stand der Technik

Die Erfindung betrifft eine endoskopische Vorrichtung nach dem Oberbegriff des Anspruchs 1, ein Endoskop mit einer endoskopischen Vorrichtung nach Anspruch 10, ein endoskopisches Instrument mit einer endoskopischen Vorrichtung nach Anspruch 11, einem Chirurgiesystem mit einer endoskopischen Vorrichtung nach Anspruch 12 sowie ein Verfahren zur Herstellung einer endoskopischen Vorrichtung nach Anspruch 13.

Es ist bereits eine endoskopische Vorrichtung mit wenigstens einem Schaft, welcher wenigstens einen in zumindest einer Ebene auslenkbaren Abschnitt aufweist, und mit wenigstens einer Auslenkmechanik, welche zur Auslenkung des auslenkbaren Abschnitts eingerichtet ist und in Reihe angeordnet zumindest ein erstes Verbindungsglied und zumindest ein zu einer Auslenkung mit dem ersten Verbindungsglied zusammenwirkendes zweites Verbindungsglied umfasst und wenigstens einen Steuerstrang, welcher zu einer Verstellung einer Auslenkung des auslenkbaren Abschnitts mit den Verbindungsgliedern gekoppelt und mit einem Endabschnitt des Schafts verbunden ist, vorgeschlagen worden.

Die Aufgabe der Erfindung besteht insbesondere darin, eine gattungsgemäße Vorrichtung mit verbesserten Eigenschaften hinsichtlich einer Funktionalität bereitzustellen. Die Aufgabe wird erfindungsgemäß durch die Merkmale der Patentansprüche 1, 10 bis 13 gelöst, während vorteilhafte Ausgestaltungen und Weiterbildungen der Erfindung den Unteransprüchen entnommen werden können.

Die Patentanmeldung EP 3 087 944 A1 offenbart ein handgehaltenes medizinisches Instrument mit einem abwinkelbaren distalen Abschnitt. Dieser besteht aus in Reihe angeordneten Wirbelelementen, an welche Zugseile zur Ansteuerung angelenkt.

Die Offenlegungsschrift US 2016/0066939 A1 lehrt ein System für medizinische Behandlungen mit einer abwinkelbaren Sonde und einem chirurgischen Werkzeug mit einem abwinkelbaren Schaft, die unabhängig voneinander abwinkelbar sind. Der abwinkelbare Abschnitt weist aneinandergereihte Abwinklungsglieder auf. Letztere weisen Kanäle für Abwinklungskabel zur Ansteuerung auf.

Aus der US-Offenlegungsschrift 2016/0074028 A1 ist ein handgehaltenes chirurgisches Instrument mit einem Steuermechanismus bekannt. Der abwinkelbare Bereich wird von mehreren Abwinklungsgliedern gebildet, die von Steuerkabeln angesteuert werden.

### Vorteile der Erfindung

Die Erfindung geht aus von einer endoskopischen Vorrichtung mit wenigstens einem Schaft, welcher wenigstens einen in zumindest einer Ebene auslenkbaren Abschnitt aufweist, und mit wenigstens einer Auslenkmechanik, welche zur Auslenkung des auslenkbaren Abschnitts eingerichtet ist und in Reihe angeordnet zumindest ein erstes Verbindungsglied und zumindest ein zu einer Auslenkung mit dem ersten Verbindungsglied zusammenwirkendes zweites Verbindungsglied umfasst und wenigstens einen Steuerstrang, welcher zu einer Verstellung einer Auslenkung des auslenkbaren Abschnitts mit den Verbindungsgliedern gekoppelt und mit einem Endabschnitt des Schafts verbunden ist.

In einem Aspekt der Erfindung, welcher insbesondere mit weiteren Aspekten der Erfindung in Kombination betrachtet werden kann, wird vorgeschlagen, dass wenigstens ein Teil des Steuerstrangs im Bereich des Endabschnitts des Schafts eine Umschlingung ausbildend angeordnet ist.

Hierdurch kann vorteilhaft eine Funktionalität der endoskopischen Vorrichtung verbessert werden. Insbesondere kann eine Anordnung des Steuerstrangs gesichert werden. Weiter vorteilhaft kann vermieden werden, dass sich der Steuerstrang im Bereich des Endabschnitts des Schafft löst, beschädigt oder zerstört wird. Es lässt sich weiter vorteilhaft eine Standzeit der endoskopischen Vorrichtung erhöhen. Ferner könnten Wartungsintervalle zu einem etwaigen Austausch des Steuerstrangs vermieden werden.

Unter einer "endoskopischen Vorrichtung" soll insbesondere ein, vorzugsweise funktionsfähiger Bestandteil, insbesondere eine Unterbaugruppe und/oder eine Konstruktions- und/oder eine Funktionskomponente eines endoskopischen Instruments und/oder eines Endoskops verstanden werden. Alternativ kann die endoskopische Vorrichtung ein Endoskop und/oder ein endoskopisches Instrument, zumindest teilweise, vorzugsweise zumindest zu einem Großteil und besonders bevorzugt vollständig ausbilden. Unter dem Begriff "endoskopisch" soll insbesondere auch minimalinvasiv verstanden werden. Unter dem Ausdruck "zumindest zu einem Großteil" soll dabei insbesondere zumindest zu 55 %, vorzugsweise zumindest zu 65 %, bevorzugt zumindest zu 75 %, besonders bevorzugt zumindest zu 85 % und ganz besonders bevorzugt zumindest zu 95 %, sowie vorteilhaft vollständig verstanden werden und zwar insbesondere mit Bezug auf ein Volumen und/oder eine Masse eines Objekts. Die endoskopische Vorrichtung ist beispielsweise dazu eingerichtet, zumindest teilweise und vorzugsweise zumindest zu einem Großteil in eine insbesondere künstliche und/oder natürliche Öffnung, insbesondere Körperöffnung, eingeführt zu werden, um sich dort eine Behandlung und/oder Untersuchung vorzugnehmen. Bei einem endoskopischen Instrument kann es sich beispielsweise um ein endoskopisches Zangeninstrument, ein endoskopisches Schereninstrument, ein endoskopisches Skalpellinstrument, ein endoskopisches Klemmeninstrument oder dergleichen handeln. Es ist denkbar, dass die endoskopische Vorrichtung zur Bereitstellung wenigstens eines, zweier oder mehrerer elektrischer Potentiale eingerichtet ist, um beispielsweise Gewebe zu schneiden, zu versiegeln, zu koagulieren und/oder dergleichen. Unter "eingerichtet" soll insbesondere speziell programmiert, vorgesehen, ausgelegt, ausgebildet und/oder ausgestattet verstanden werden. Darunter, dass ein Objekt zu einer bestimmten Funktion eingerichtet ist, soll insbesondere verstanden werden, dass das Objekt diese bestimmte Funktion in zumindest einem Anwendungs- und/oder Betriebszustand erfüllt und/oder ausführt. Weist die endoskopische Vorrichtung in etwa wenigstens einen Schaft auf, so ist dieser dazu eingerichtet, wenigstens teilweise und vorzugsweise wenigstens zu einem Großteil in eine insbesondere künstliche und/oder natürliche Öffnung, insbesondere Körperöffnung, eingeführt zu werden. Der Schaft umfasst beispielsweise wenigstens einen Endabschnitt und/oder weiteren Endabschnitt, wobei beispielsweise der Endabschnitt ein distaler Endabschnitt und/oder der weitere Endabschnitt ein proximaler Endabschnitt ist. Unter "distal" soll insbesondere bei einer Bedienung einem Patienten zugewandt und/oder einem Bediener abgewandt verstanden werden. Unter "proximal" soll insbesondere bei einer Bedienung einem Patienten abgewandt und/oder einem Bediener zugewandt verstanden werden. Der Schaft weist beispielsweise eine Haupterstreckungsachse auf. Unter einer Haupterstreckungsachse eines Objekts soll eine Achse verstanden werden, welche durch den geometrischen und/oder Massenmittelpunkt des Objekts verläuft und zumindest im Wesentlichen parallel zu einer Haupterstreckungsrichtung des Objekts ist. Unter einer "Haupterstreckungsrichtung" eines Objekts soll dabei insbesondere eine Richtung verstanden werden, welche parallel zu einer längsten Kante eines kleinsten gedachten Quaders verläuft, welcher das Objekt gerade noch vollständig umschließt. Eine Längserstreckung beispielsweise des Schafts ist mit dessen Haupterstreckungsrichtung identisch. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung und die Bezugsrichtung einen Winkel von 0° insbesondere unter Berücksichtigung einer maximalen Abweichung von kleiner als 8°, vorteilhaft von kleiner als 5° und besonders vorteilhaft von kleiner als 2° einschließt. Eine Breite kann zumindest im Wesentlichen senkrecht zur Längserstreckung gemessen sein. Unter "zumindest im Wesentlichen senkrecht" soll hier insbesondere eine Ausrichtung einer Richtung relativ zu einer Bezugsrichtung, insbesondere in einer Ebene verstanden werden, wobei die Richtung und die Bezugsrichtung einen Winkel von 90°, insbesondere unter Berücksichtigung einer maximalen Abweichung von kleiner als 8°, vorteilhaft von kleiner als 5° und besonders vorteilhaft von kleiner als 2° einschließt. Die endoskopische Vorrichtung kann mehrere Komponenten aufweisen, welche zumindest im Wesentlichen identisch zueinander ausgebildet sein können. Unter "zumindest im Wesentlichen identisch" soll bis auf Montage- und/oder Herstellungstoleranzen identisch oder identisch verstanden werden. Die endoskopische Vorrichtung kann wenigstens teilweise einstückig ausgebildet sein. Darunter, dass "ein Objekt und ein weiteres Objekt zumindest teilweise einteilig ausgebildet/verbunden sind", soll insbesondere verstanden werden, dass zumindest ein Element und/oder Teil des Objekts und zumindest ein Element und/oder Teil des weiteren Objekts einteilig ausgebildet/verbunden sind. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess. Ferner kann unter einstückig auch einteilig verstanden werden. Unter "einteilig" soll insbesondere in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Komponenten der endoskopischen Vorrichtung sollen wenigstens teilweise form- und/oder kraftschlüssig miteinander verbunden sein. Unter einer "kraft-und/oder formschlüssigen Verbindung" ist insbesondere zu verstehen, verbunden, vorzugsweise lösbar verbunden zu sein, wobei eine Haltekraft zwischen zwei Objekten vorzugsweise über einen geometrischen Eingriff der Strukturkomponenten ineinander und/oder über eine Reibungskraft, die vorzugsweise zwischen den Objekten wirkt, übertragen wird. Alternativ oder zusätzlich können Komponenten der endoskopischen Vorrichtung stoffschlüssig miteinander verbunden sein. Unter "stoffschlüssig verbunden" soll insbesondere verstanden werden, dass die Objekte durch atomare oder molekulare Kräfte zusammengehalten werden, wie beispielsweise beim Löten, Schweißen, Kleben und/oder Vulkanisieren. Ferner kann die endoskopische Vorrichtung Teil eines Chirurgiesystems sein. Unter einem Chirurgiesystem soll insbesondere ein für die Durchführung eines chirurgischen, beispielsweise endoskopischen und/oder minimalinvasiven Verfahrens eingerichtetes System verstanden werden, welches wenigstens einen Chirurgieroboter umfasst. Der Chirurgieroboter kann dabei wenigstens einen Chirurgieroboterarm oder mehrere Chirurgieroboterarme umfassen. Die endoskopische Vorrichtung kann mit dem Chirurgieroboter, insbesondere dem Chirurgieroboterarm steuerbar und/oder betätigbar sein. Die endoskopische Vorrichtung kann mit dem Chirurgieroboter lösbar koppelbar sein, um beispielsweise einen Austausch und/oder eine Reinigung der endoskopischen Vorrichtung zu ermöglichen. Ferner kann das Chirurgiesystem wenigstens ein Steuergerät umfassen, welches zu einer manuellen und/oder automatisierten Steuerung des Chirurgieroboters eingerichtet ist.

Der Schaft weist einen auslenkbaren Abschnitt auf. Zur Auslenkung des Schafts weist die endoskopische Vorrichtung wenigstens eine Auslenkmechanik auf. Die Auslenkmechanik ist insbesondere zu einer mechanischen Auslenkung des ablenkbaren Abschnitts des Schafts ausgebildet. Der Schaft ist in wenigstens einer weiteren Ebene, welche von der zumindest einen Ebene verschieden ist, auslenkbar. Beispielsweise kann die weitere Ebene senkrecht auf der Ebene stehen. Ferner ist denkbar, dass der Schaft entlang seines Umfangs in beliebigen Ebenen auslenkbar ist. Insbesondere umfasst die Auslenkmechanik wenigstens ein und vorzugsweise mehrere erste Verbindungsglieder, welche in etwa zumindest im Wesentlichen identisch zueinander ausgebildet sein können. Insbesondere kann die Auslenkmechanik wenigstens zwei und vorzugsweise mehrere zweite Verbindungsglieder umfassen, welche in etwa zumindest im Wesentlichen identisch zueinander ausgebildet seien können. Die ersten Verbindungsglieder und die zweiten Verbindungsglieder können alternierend in Reihe angeordnet sein. Außer an Randbereichen der Auslenkmechanik kann ein Verbindungsglied von zwei zweiten Verbindungsgliedern benachbart sein oder umgekehrt. Ferner ist denkbar, dass wenigstens ein zweites Verbindungsglied einen Randbereich der Auslenkmechanik definiert bzw. zwei zweite Verbindungsglieder gegenüberliegende Randbereiche der Auslenkmechanik definieren. Dabei kann ein zweites Verbindungsglied wenigstens teilweise einstückig mit einem Endabschnitt des Schafts und/oder dem Endeffektorkopf ausgebildet und/oder verbunden sein. Ein erstes Verbindungsglied ist insbesondere von zwei gegenüberliegenden Seiten jeweils von einem zweiten Verbindungsglied umgriffen. Ferner greifen jeweils zwei erste Verbindungsglieder von zwei gegenüberliegenden Seiten in ein zweites Verbindungsglied ein. Das erstes Verbindungsglied und das zweites Verbindungsglied können dabei kugelgelenkartig miteinander verbunden sein. Insbesondere weist das erste Verbindungsglied wenigstens einen Gelenkkopf auf und das zweite Verbindungsglied wenigstens eine Gelenkpfanne, welche gemeinsam nach Art eines Kugelgelenks zusammenwirken.

Das erste Verbindungsglied ist als ein Rotationskörper ausgebildet. Das erste Verbindungsglied weist eine erste Rotationssymmetrieachse auf. Das erste Verbindungsglied weist insbesondere eine olivenartige Form auf. Das zweite Verbindungsglied ist als ein Rotationskörper ausgebildet. Das zweite Verbindungsglied weist eine zweite Rotationssymmetrieachse auf. Das zweite Verbindungsglied weist insbesondere eine diskusartige Form auf. Unter einem "Geradenstellungsabstand" soll insbesondere eine Stellung wenigstens des ersten Verbindungsglieds und des zweiten Verbindungsglieds, insbesondere aller ersten und zweiten Verbindungsglieder verstanden werden, in welcher die erste Rotationssymmetrieachse und die zweite Rotationssymmetrieachse, insbesondere alle Rotationssymmetrieachsen der Verbindungsglieder, zueinander zumindest im Wesentlichen parallel ausgerichtet sind oder sogar miteinander identisch sind. Unter einer "Auslenkstellung" soll insbesondere eine Stellung wenigstens des ersten Verbindungsglieds und des zweiten Verbindungsglieds, insbesondere aller ersten und zweiten Verbindungsglieder verstanden werden, in welcher die erste Rotationssymmetrieachse und die zweite Rotationssymmetrieachse, insbesondere alle Rotationssymmetrieachsen der Verbindungsglieder, zueinander winklig angeordnet sind und vorzugsweise um einen gleichen Winkel zueinander versetzt sind. Unter "winklig" angeordnet soll insbesondere verschieden von zumindest im Wesentlichen parallel angeordnet verstanden werden.

Der Endeffektor und der Betätigungsstrang können zusätzlich elektrisch miteinander gekoppelt sein, um beispielsweise wenigstens ein elektrisches Potential von dem Betätigungsstrang auf den Endeffektor, insbesondere einem Werkzeugstück des Endeffektors zu übertragen. Der Betätigungsstrang weist insbesondere wenigstens eine Innentrosse auf, welcher vorzugsweise flexibel ausgebildet ist. Insbesondere kann die Innentrosse über eine gesamte Erstreckung des Betätigungsstrangs flexibel ausgebildet sein. Es ist denkbar, dass die Innentrosse elektrisch leitend ausgebildet sein kann, um beispielsweise ein elektrisches Potential zu übertragen. Ferner kann der Betätigungsstrang wenigstens eine Außentrosse aufweisen, welche vorteilhaft die Innentrosse koaxial umgebend angeordnet sein kann. Insbesondere kann die Außentrosse über wenigstens einen Großteil einer Erstreckung des Betätigungsstrangs flexibel ausgebildet sein. Es ist denkbar, dass die Außentrosse elektrisch leitend ausgebildet seien kann, um beispielsweise ein weiteres elektrisches Potential zu übertragen. Die Außentrosse könnte als ein Schlauch ausgebildet sein. Beispielsweise könnte die Außentrosse als ein Gewebe ausgebildet sein.

Der Steuerstrang der Auslenkmechanik ist insbesondere biegeschlaff ausgebildet. Unter einem "biegeschlaffen Bauteil" soll insbesondere ein Bauteil, vorzugsweise ein längliches Bauteil, verstanden werden, welches zumindest in einer Richtung senkrecht zu einer Haupterstreckungsrichtung biegeschlaffe Eigenschaften aufweist. Vorzugsweise soll darunter insbesondere ein forminstabiles Bauteil verstanden werden. Besonders bevorzugt soll darunter insbesondere ein Bauteil verstanden werden, welches in einem ausgestreckten Zustand einer parallel zu einer Haupterstreckungsrichtung wirkenden Druckkraft eine Gegenkraft aufbringt, die geringer ist als eine Gewichtskraft des Bauteils. Vorzugsweise beträgt die Gegenkraft maximal 70%, vorzugsweise maximal 50% und besonders bevorzugt maximal 30% einer Gewichtskraft. Dabei soll unter einem "länglichen Bauteil" insbesondere ein Bauteil verstanden werden, das eine Quererstreckung aufweist, die um ein Vielfaches geringer ist, als eine Längserstreckung. Dabei soll unter "um ein Vielfaches geringer" insbesondere zumindest 3-mal, vorzugsweise zumindest 5-mal und besonders bevorzugt zumindest 10-mal geringer verstanden werden.

Es wird ferner vorgeschlagen, dass die Umschlingung einen Umschlingungsradius aufweist, welcher größer ist als ein Durchmesser des Steuerstrangs. Es kann vorteilhaft ein Abknicken des Steuerstrangs im Bereich des Endabschnitts vermieden werden. Weiter vorteilhaft kann dadurch eine Standzeit weiter erhöht werden. Insbesondere ist der Umschlingungsradius wenigstens um einen Faktor zwei, vorzugswiese wenigstens um einen Faktor vier und besonders bevorzugt wenigstens um einen Faktor acht größer als der Durchmesser des Steuerstrangs.

Es wird ferner vorgeschlagen, dass der Endabschnitt des Schafts wenigstens eine Strangaufnahme aufweist, in welcher wenigstens ein die Umschlingung ausbildender Teil des Steuerstrangs angeordnet ist. Mittels der Strangaufnahme kann vorteilhaft eine Führung des Steuerstrangs erzielt werden. Es kann vorteilhaft ein Abknicken des Steuerstrangs im Bereich des Endabschnitts vermieden werden. Die Strangaufnahme, ist insbesondere dazu eingerichtet den Steuerstrang entlang dessen die Umschlingung ausbildenden Teils zu führen.

Die Erfindung umfasst ferner, dass die Strangaufnahme eine hufeisenartige und/oder schlüssellochartige Kontur aufweist. Hierdurch kann eine vorteilhafte Anordnung des Steuerstrangs entlang der Strangaufnahme erzielt werden, wobei insbesondere Knicke, scharfe Kanten oder dergleichen vermeiden werden können. Die Kontur des Strangaufnahme umfasst wenigstens einen Kreisabschnitt, sowie vorzugsweise zwei an den Kreisabschnitt angefügte Schenkel, welche sich vorzugsweise gegenüberliegen und besonders bevorzugt zumindest im Wesentlichen parallel zueinander orientiert sind.

Es wird ferner vorgeschlagen, dass die Strangaufnahme einen Umfangserstreckungswinkel von mehr als 180°, vorzugsweise mehr als 210° und besonders bevorzugt mehr als 240° und/oder von weniger als 360°, vorzugsweise weniger als 330° und besonders bevorzugt weniger als 330° beschreibt. Die auf den Steuerstrang wirkende Kraft kann auf einen großen Radius verteilt werden, wodurch ein Risiko einer Beschädigung des Steuerstrangs weiter verringert werden kann. Ferner kann weiterhin eine vorteilhafte Führung und/oder Entlanggleiten des Steuerstrangs entlang der Strangaufnahme, sichergestellt werden.

Es wird weiter vorgeschlagen, dass der Steuerstrang mittels der Strangaufnahme wenigstens abschnittsweise im Wesentlichen parallel zu einer Längserstreckungsrichtung des Schaftes geführt ist. Es kann vorteilhaft eine gleichmäßige Führung und/oder ein gutes Gleitverhalten des Steuerstrangs sichergestellt werden. Insbesondere ist der Steuerstrang entlang der beiden Schenkel der Kontur der Strangaufnahme im Wesentlichen parallel zur Längserstreckungsrichtung des Schafts geführt.

Weiterhin wird vorgeschlagen, dass der Steuerstrang mittels der Strangaufnahme in Richtung des Endabschnitts des Schafts geführt ist und nach der Umschlingung wieder entgegen dem Endabschnitts des Schafts geführt ist. Es kann vorteilhaft eine Führung und/oder ein gutes Gleitverhalten des Steuerstrangs weiter verbessert werden. Insbesondere führt einer der Schenkel der Kontur des Steuerstrangs den Steuerstrang in Richtung des Endabschnitts und der zweite der Schenkel den Steuerstrang entgegen dem Endabschnitt des Schafts, wobei die beiden Schenkel von einem Kreisabschnitt miteinander verbunden sind, welcher den Steuerstrang aus einer Richtung in Richtung des Endabschnitts in eine Richtung entgegen dem Endabschnitt umlenkt.

Es wird ferner vorgeschlagen, dass ein Abstand zwischen einem hingeführten Abschnitt und einem zurückgeführten Abschnitt des Steuerstrangs existiert, welcher kleiner ist als ein doppelter Umschlingungsradius der Umschlingung. Es kann vorteilhaft ein abknicken des Steuerstrangs vermieden werden. Die auf den Steuerstrang wirkende Kraft kann auf einen großen Radius verteilt werden, wodurch ein Risiko einer Beschädigung des Steuerstrangs weiter verringert werden kann. Der Abstand ist insbesondere um einen Faktor 1,5, vorzugsweise um einen Faktor 2 und besonders bevorzugt um einen Faktor 3 kleiner des doppelten Umschlingungsradius der Umschlingung.

Die Strangaufnahme könne nach innen offen sein oder zu einem Auffädeln des Steuerstrangs eingerichtet sein. Um jedoch vorteilhaft ein Einlegend es Steuerstrangs zu vereinfachen und insbesondere eine knickfreie Anordnung des Strangs zu gewährleisten, wird vorgeschlagen, dass die Strangaufnahme zu einem radialen Einlegen des Steuerstrangs zumindest teilweise radial nach außen geöffnet ist.

Es wird zudem vorgeschlagen, dass die endoskopische Vorrichtung wenigstens eine mit dem Endabschnitt des Schafts koppelbare Abdeckung umfasst, welche in einem gekoppelten Zustand die Strangaufnahme radial von außen zumindest teilweise bedeckt. Hierdurch kann insbesondere eine radial geöffnete Strangaufnahme gegen Verunreinigungen geschützt werden.

Weiterhin wird vorgeschlagen, dass die endoskopische Vorrichtung wenigstens einen weiteren Steuerstrang umfasst, welcher parallel und entlang eins Umfangs des Schafts versetzt zum Steuerstrang angeordnet ist und im Bereich des Endabschnitts des Schafts wenigstens eine weitere Umschlingung ausbildend angeordnet ist. Hierdurch kann vorteilhaft eine bei einer Ansteuerung auf die Auslenkmechanik wirkende Kraft gleichmäßig verteil werden. Der weitere Steuerstrang ist im Wesentlichen identisch zu dem Steuerstrang angeordnet. Der weitere Steuerstrang ist in einer Umfangsrichtung des Endabschnitts des Schafts versetzt zu dem Steuerstrang angeordnet. Insbesondre weist der Endabschnitt des Schaftes wenigstens eine weitere Strangaufnahme auf, welche zur Aufnahme des weiteren Steuerstrangs eingerichtet ist. Die weitere Strangaufnahme ist insbesondere in Umfangsrichtung des Endabschnitts des Schafts versetzt zur Strangaufnahme angeordnet.

Ein Gegenstand der vorliegenden Offenbarung sollen/soll hierbei nicht auf die oben beschriebene Anwendung und Ausführungsform beschränkt sein. Insbesondere kann der Gegenstand der vorliegenden Offenbarung zu einer Erfüllung einer hierin beschriebenen Funktionsweise eine von einer hierin genannten Anzahl von einzelnen Elementen, Bauteilen und Einheiten, sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Falls von einem bestimmten Objekt mehr als ein Exemplar vorhanden ist, ist nur eines davon in den Figuren und in der Beschreibung mit einem Bezugszeichen versehen. Die Beschreibung dieses Exemplars kann entsprechend auf die anderen Exemplare von dem Objekt übertragen werden.

### Zeichnungen

Weitere Vorteile ergeben sich aus der folgenden Zeichnungsbeschreibung. In den Zeichnungen sind offenbarungsgemäße Ausführungsbeispiele dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### Es zeigen:

- Fig. 1: eine schematische Darstellung eines Chirurgiesystems mit einer endoskopischen Vorrichtung in einer perspektivischen Ansicht,
- Fig. 2: eine schematische Darstellung eines sich in einer Geradenstellung befindlichen Teils der endoskopischen Vorrichtung in einer Seitenansicht,
- Fig. 3: eine schematische Darstellung eines sich in einer Auslenkungsstellung befindlichen Teils der endoskopischen Vorrichtung in einer Seitenansicht,
- Fig. 4: eine schematische Darstellung eines sich in einer Geradenstellung befindlichen Teils der endoskopischen Vorrichtung in einer Schnittansicht,
- Fig. 5: eine schematische Darstellung eines sich in einer Auslenkungsstellung befindlichen Teils der endoskopischen Vorrichtung in einer Schnittansicht,
- Fig. 6: eine schematische Darstellung eines Teils der endoskopischen Vorrichtung in einem teilweise demontierten Zustand in einer perspektivischen Ansicht,
- Fig. 7: eine schematische Darstellung wenigstens eines Teils einer weiteren endoskopischen Vorrichtung in einer Schnittansicht längs eines Schafts der endoskopischen Vorrichtung,
- Fig. 8: eine schematische Darstellung wenigstens eines Teils der endoskopischen Vorrichtung aus Fig. 7 in einer Schnittansicht quer zu einem Schaft der endoskopischen Vorrichtung,
- Fig. 9: eine schematische Darstellung eines Teils der endoskopischen Vorrichtung aus Fig. 7 in einer perspektivischen Ansicht,
- Fig. 10: eine schematische Darstellung wenigstens eines Teils einer alternativen endoskopischen Vorrichtung in einer Schnittansicht längs eines Schafts der endoskopischen Vorrichtung in einer Geradenstellung,
- Fig. 11: eine schematische Darstellung wenigstens eines Teils der endoskopischen Vorrichtung aus Fig. 10 in einer Schnittansicht längs des Schafts der endoskopischen Vorrichtung in einer Auslenkungsstellung,
- Fig. 12: eine schematische Darstellung wenigstens eines Teils einer weiteren endoskopischen Vorrichtung in einer perspektivischen Ansicht,
- Fig. 13: eine schematische Darstellung wenigstens eines Teils einer zusätzlichen endoskopischen Vorrichtung in einer perspektivischen Ansicht in einem Montagezustand,
- Fig. 14: eine schematische Darstellung wenigstens eines Teils der endoskopischen Vorrichtung aus Fig. 13 in einer perspektivischen Ansicht in einem weiteren Montagezustand,
- Fig. 15: eine schematische Darstellung wenigstens eines Teils der endoskopischen Vorrichtung aus Fig. 13 und Fig. 14 in einer perspektivischen Ansicht in einem zusätzlichen Montagezustand,
- Fig. 16: eine schematische Darstellung wenigstens eines Teils einer weiteren endoskopischen Vorrichtung in einer Draufsicht,
- Fig. 17: eine schematische Darstellung wenigstens eines Teils einer alternativen endoskopischen Vorrichtung in einer perspektivischen Ansicht,
- Fig. 18: eine schematische Darstellung wenigstens eines Teils einer alternativen endoskopischen Vorrichtung in einer perspektivischen Ansicht in einem Montagezustand,
- Fig. 19: eine schematische Darstellung wenigstens eines Teils der endoskopischen Vorrichtung aus Fig. 18 in einer perspektivischen Ansicht in einem montierten Zustand,
- Fig. 20: eine schematische Darstellung wenigstens eines Teils der endoskopischen Vorrichtung aus Fig. 18 in einer perspektivischen Ansicht in einem Montagezustand,
- Fig. 21: eine schematische Darstellung wenigstens eines Teils der endoskopischen Vorrichtung aus Fig. 18 in einer perspektivischen Ansicht in einem weiteren Montagezustand,
- Fig. 22: eine schematische Darstellung wenigstens eines Teils der endoskopischen Vorrichtung aus Fig. 18 in einer perspektivischen Ansicht in einem montierten Zustand,
- Fig. 23: eine schematische Darstellung wenigstens eines Teils einer alternativen endoskopischen Vorrichtung in einer Seitenansicht in einer Geradenstellung,
- Fig. 24: eine schematische Darstellung wenigstens eines Teils der endoskopischen Vorrichtung aus Fig. 23 in einer Schnittansicht längs eines Schafts der endoskopischen Vorrichtung in der Geradestellung,
- Fig. 25: eine schematische Darstellung wenigstens eines Teils der endoskopischen Vorrichtung aus Fig. 23 und 24 in einer Seitenansicht in einer Auslenkungsstellung,
- Fig. 26: eine schematische Darstellung wenigstens eines Teils der endoskopischen Vorrichtung aus Fig. 23, 34 und 25 in einer Schnittansicht längs des Schafts der endoskopischen Vorrichtung in der Auslenkungsstellung,
- Fig. 27: eine schematische Darstellung wenigstens eines Teils einer alternativen endoskopischen Vorrichtung in einer perspektivischen Ansicht in einem Montagezustand.

### Beschreibung der Ausführungsbeispiele

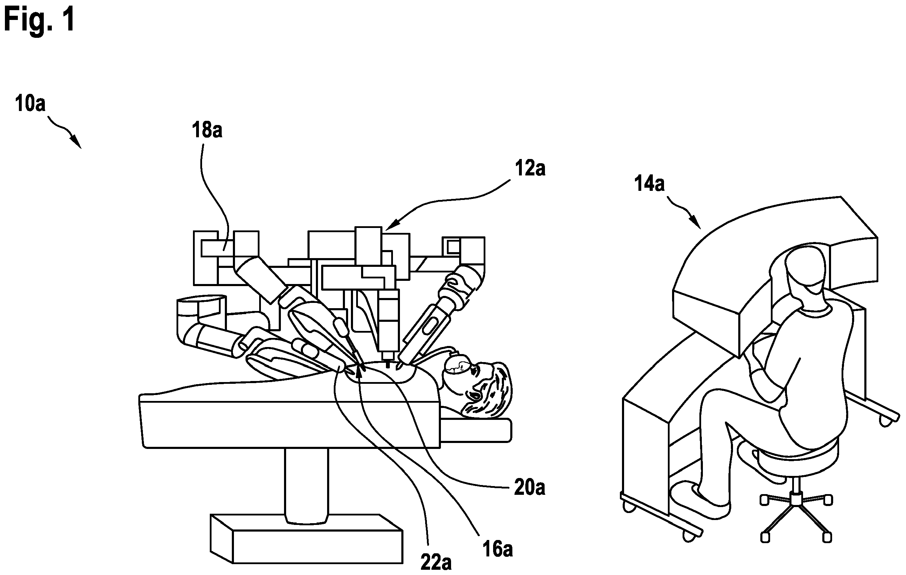

Fig. 1 zeigt eine schematische Darstellung eines Chirurgiesystems 10a in einer perspektivischen Ansicht. Das Chirurgiesystem 10a umfasst wenigstens einen Chirurgieroboter 12a. Ferner umfasst das Chirurgiesystem 10a wenigstens ein Steuergerät 14a. Das Steuergerät 14a ist zu einer Steuerung des Chirurgieroboters 12a eingerichtet.

Der Chirurgieroboter 12a ist dazu eingerichtet, wenigstens eine endoskopische Vorrichtung 16a des Chirurgiesystems 10a zu führen. Dazu weist der Chirurgieroboter 12a wenigstens einen Roboterarm 18a auf. In einem Betriebszustand ist die endoskopische Vorrichtung 16a mit dem Roboterarm 18a gekoppelt. Die endoskopische Vorrichtung 16a kann lösbar mit dem Roboterarm 18a verbunden sein, beispielsweise um diese auszutauschen, zu modifizieren, zu sterilisieren oder dergleichen. Im vorliegenden Fall weist der Chirurgieroboter 12a mehrere Roboterarme auf. Von den Roboterarmen ist der Übersichtlichkeit halber nur der Roboterarm 18a mit einem Bezugszeichen versehen ist.

Das Chirurgiesystem 10a umfasst wenigstens eine endoskopische Vorrichtung 16a. Im vorliegenden Fall umfasst das Chirurgiesystem 10a mehrerer endoskopische Vorrichtungen. Der Chirurgieroboter 12a weist je einen Roboterarm 18a pro endoskopischer Vorrichtung 16a auf. Der Übersichtlichkeit halber ist von den endoskopischen Vorrichtungen nur die endoskopische Vorrichtung 16a mit einem Bezugszeichen versehen. Die mehreren endoskopischen Vorrichtungen könnten zueinander im Wesentlichen identisch ausgebildet sein. Im Wesentlichen identisch kann bis auf Herstellungs- und/oder Montagetoleranzen gleich bedeuten. Allerdings ist denkbar, dass die mehreren endoskopischen Vorrichtungen wenigstens teilweise voneinander verschieden ausgebildet sein könnten und sich beispielswiese durch eine Art eines Endeffektors und/oder eine Funktionsweise voneinander unterscheiden. Auch würde ein Fachmann die mehreren endoskopischen Vorrichtungen zu verschiedenen chirurgischen Anwendungen gemäß seinem Fachwissen in naheliegender Weise anpassen.

Die endoskopische Vorrichtung 16a bildet wenigstens teilweise ein endoskopisches Instrument 20a aus. Im vorliegenden Fall bildet die endoskopische Vorrichtung 16a ein endoskopisches Instrument 20a vollständig aus. Eine endoskopische Vorrichtung könnte jedoch nur Bestandteil eines endoskopischen Instruments sein. Ferner könnte eine endoskopische Vorrichtung, beispielsweise eine der mehreren endoskopischen Vorrichtungen, wenigstens teilweise oder vollständig ein Endoskop 22a ausbilden. Eine endoskopische Vorrichtung könnte jedoch auch nur ein Bestandteil eines Endoskops sein.

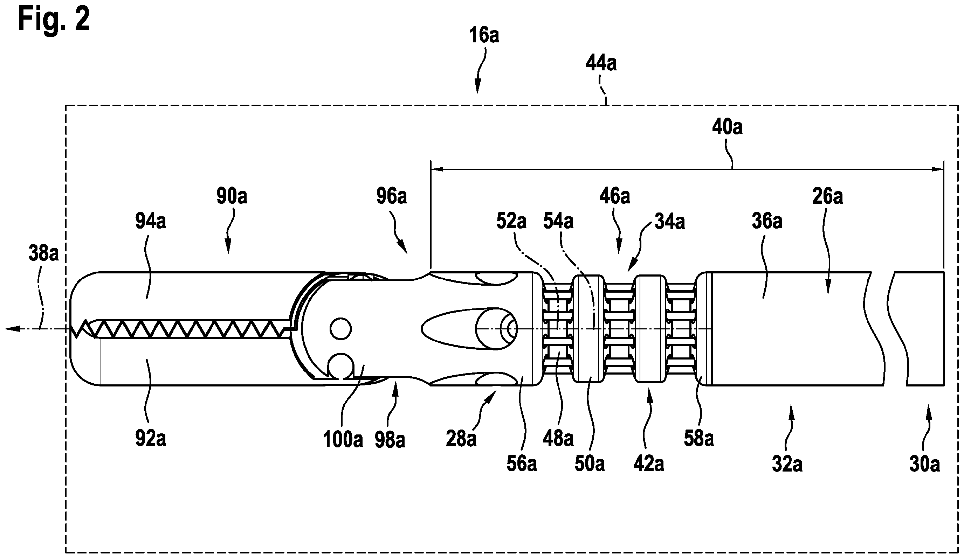

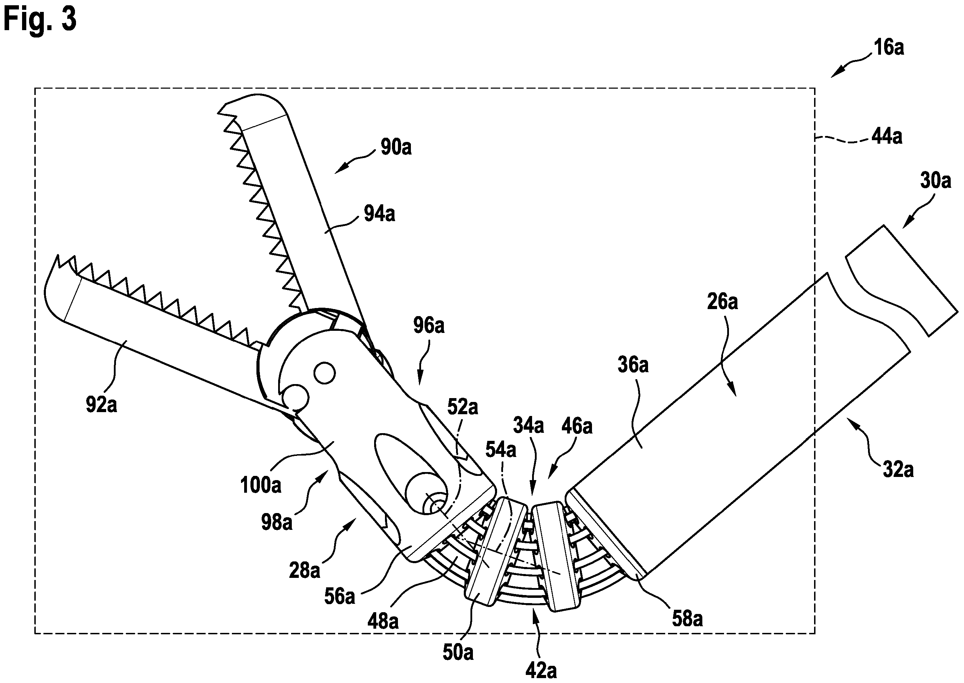

Fig. 2 zeigt eine schematische Darstellung eines sich in einer Geradenstellung befindlichen Teils der endoskopischen Vorrichtung 16a in einer Seitenansicht. Ferner ist in Fig. 3 eine schematische Darstellung eines sich in einer Auslenkungsstellung befindlichen Teils der endoskopischen Vorrichtung 16a in einer Seitenansicht gezeigt.

Die endoskopische Vorrichtung 16a weist wenigstens einen Schaft 26a auf. Im vorliegenden Fall weist die endoskopische Vorrichtung 16a genau einen Schaft 26a auf. Der Schaft 26a weist eine Längserstreckungsrichtung 38a auf. Die Längserstreckungsrichtung 38a entspricht einer Haupterstreckungsrichtung des Schafts 26a in der Geradenstellung. Entlang der Längserstreckungsrichtung 38a des Schafts 26a erstreckt sich eine Längserstreckung 40a des Schafts 26a.

Der Schaft 26a umfasst wenigstens einen Endabschnitt 28a. Der Endabschnitt 28a ist ein distaler Endabschnitt. Der Endabschnitt 28a ist zu einer Behandlung eines Patienten eingerichtet. Ferner weist der Schaft 26a einen weiteren Endabschnitt 30a auf. Der weitere Endabschnitt 30a ist ein proximaler Endabschnitt. Der weitere Endabschnitt 30a ist zu einer Kopplung mit dem Chirurgieroboter 12a, beispielsweise mit dessen Roboterarm 18a, eingerichtet. Der Endabschnitt 28a und der weitere Endabschnitt 30a liegen einander gegenüber. Ferner weist der Schaft 26a einen Mittelabschnitt 32a auf. Der Mittelabschnitt 32a verbindet den Endabschnitt 28a und den weiteren Endabschnitt 30a miteinander. Der Mittelabschnitt 32a ist zwischen den Endabschnitt 28a und dem weiteren Endabschnitt 30a angeordnet.

Der Schaft 26a weist ein Grundgerüst 34a auf. Das Grundgerüst 34a erstreckt sich von dem Endabschnitt 28a zum weiteren Endabschnitt 30a des Schafts 26a. Ferner weist der Schaft 26a einen Schaftmantel 36a auf. Der Schaftmantel 36a umgibt das Grundgerüst 34a wenigstens teilweise. Im vorliegenden Fall umgibt der Schaftmantel 36a das Grundgerüst 34a wenigstens zu einem Großteil. Der Schaftmantel 36a ist koaxial zum Grundgerüst 34a angeordnet. Der Schaftmantel 36a umgibt den Mittelabschnitt 32a zumindest teilweise. Im vorliegenden Fall umgibt der Schaftmantel 36a den Mittelabschnitt 32a wenigstens zu einem Großteil. Ferner kann der Schaft 26a eine Schafthülle aufweisen. Eine Schafthülle ist der Übersichtlichkeit halber in den Figuren nicht dargestellt, um vorteilhaft den Aufbau des Grundgerüsts 34a besser darstellen zu können. Eine Schafthülle kann dazu eingerichtet sein, den Schaft 26a nach außen hin abzudichten.

Der Schaft 26a weist wenigstens einen auslenkbaren Abschnitt 42a auf. Der auslenkbare Abschnitt 42a ist zwischen dem Endabschnitt 28a und dem weiteren Endabschnitt 30a angeordnet. Der auslenkbare Abschnitt 42a ist Teil des mit Mittelabschnitts 32a. Der auslenkbare Abschnitt 42a schließt sich unmittelbar an den Endabschnitt 28a an. Der auslenkbare Abschnitt 42a ist von dem weiteren Endabschnitt 30a beabstandet. Alternativ ist denkbar, dass ein auslenkbarer Abschnitt einen Endabschnitt, beispielsweise einen distalen Endabschnitt, wenigstens teilweise ausbildet. Vorteilhaft könnte der auslenkbare Abschnitt von einer Schafthülle umgeben sein. Die Schafthülle kann wenigstens teilweise elastisch und/oder flexibel ausgebildet sein. Beispielsweise kann es sich bei der Schafthülle um einen Gummischlauch handeln.

Der auslenkbare Abschnitt 42a ist in zumindest einer Ebene 44a auslenkbar. Die Ebene 44a stimmt in Fig.2 mit einer Bildebene der Figur überein. Der auslenkbare Abschnitt 42a im vorliegenden Fall ist sogar in mehreren Ebenen auslenkbar, von welchen der Übersichtlichkeit halber nur die Ebene 44a mit einem Bezugszeichen versehen und in den Figuren dargestellt ist. Im vorliegenden Fall ist der auslenkbare Abschnitt 42a sogar entlang eines gesamten Umfangs des Schafts 26a auslenkbar. Der auslenkbare Abschnitt 42a ist wenigstens teilweise flexibel ausgebildet.

Das Grundgerüst 34a des Schafts 26a weist eine Manschette 56a auf. Die Manschette 56a bildet wenigstens teilweise den Endabschnitt 28a des Schafts 26a aus. Die Manschette 56a schließt sich distalseitig an den auslenkbaren Abschnitt 42a an. Ferner weist das Grundgerüst 34a des Schafts 26a eine weitere Manschette 58a auf. Die weitere Manschette 58a bildet wenigstens teilweise den Mittelabschnitt 32a des Schafts 26a aus. Die weitere Manschette 58a schließt sich proximalseitig an den auslenkbaren Abschnitt 42a an.

Die endoskopische Vorrichtung 16a weist wenigstens eine Auslenkmechanik 46a auf. Die Auslenkmechanik 46a ist zur Auslenkung des auslenkbaren Abschnitts 42a des Schafts 26a eingerichtet. Im Bereich des auslenkbaren Abschnitts 42a bildet die Auslenkmechanik 46a zumindest teilweise das Grundgerüst 34a des Schafts 26a aus.

Die Auslenkmechanik 46a weist wenigstens ein erstes Verbindungsglied 48a auf. Im vorliegenden Fall weist die Auslenkmechanik 46a mehrere erste Verbindungglieder, beispielsweise drei erste Verbindungsglieder, auf. Von den mehreren ersten Verbindungsgliedern ist der Übersichtlichkeit halber nur das erste Verbindungsglied 48a mit einem Bezugszeichen versehen ist. Die mehreren ersten Verbindungsglieder sind dabei im Wesentlichen identisch ausgebildet. Eine Beschreibung des ersten Verbindungsglieds 48a ist auf die mehreren ersten Verbindungsglieder übertragbar. Alternativ könnten die mehreren ersten Verbindungsglieder jedoch auch voneinander zumindest teilweise verschieden ausgebildet sein.

Das erste Verbindungsglied 48a ist symmetrisch. Das erste Verbindungsglied 48a ist im Wesentlichen als ein Rotationskörper ausgebildet. Das erste Verbindungsglied 48a weist eine erste Rotationssymmetrieachse 52a auf. Um die erste Rotationssymmetrieachse 52a weist das erste Verbindungsglied 48a wenigstens eine zweifache Rotationssymmetrie auf. Beispielsweise könnte eine Anzahl einer ersten Rotationssymmetrie mit einer Anzahl von Ebenen, in welcher der auslenkbare Abschnitt auslenkbar ist übereinstimmen. In einer Geradenstellung entspricht die Längserstreckungsrichtung 38a des Schafts 26a mit der ersten Rotationssymmetrieachse überein. Ferner weist die Auslenkmechanik 46a wenigstens ein zweites Verbindungsglied 50a auf. Im vorliegenden Fall weist die Auslenkmechanik 46a mehrere zweite Verbindungglieder, beispielsweise vier zweite Verbindungsglieder, auf. Von den mehreren zweiten Verbindungsgliedern ist der Übersichtlichkeit halber nur das zweite Verbindungsglied 50a mit einem Bezugszeichen versehen. Die mehreren zweiten Verbindungsglieder sind, soweit nicht weiter darauf hingewiesen, im Wesentlichen identisch ausgebildet. Eine Beschreibung bezüglich des zweien Verbindungsglieds 50a ist somit auf die mehreren zweiten Verbindungsglieder übertragbar. Alternativ könnten die mehreren zweiten Verbindungsglieder auch voneinander zumindest teilweise verschieden ausgebildet sein.

Das zweite Verbindungsglied 50a ist dem ersten Verbindungsglied 48a zumindest teilweise koaxial umgebend angeordnet. Das zweite Verbindungsglied 50a weist einen Außendurchmesser auf, welcher größer ist als ein Außendurchmesser des ersten Verbindungsglieds 48a. Das zweite Verbindungsglied 50a weist eine diskusartige und/oder linsenartige Form auf. Das erste Verbindungsglied 48a weist eine olivenartige Form auf.

Das zweite Verbindungsglied 50a ist symmetrisch. Das zweite Verbindungsglied 50a ist im Wesentlichen als ein Rotationskörper ausgebildet. Das zweite Verbindungsglied 50a weist eine zweite Rotationssymmetrieachse 54a auf. Um die zweite Rotationssymmetrieachse 54a weist das zweite Verbindungsglied 50a wenigstens eine zweifache Rotationssymmetrie auf. Beispielsweise könnte eine Anzahl einer ersten Rotationssymmetrie mit einer Anzahl von Ebenen, in welcher der auslenkbare Abschnitt auslenkbar ist, übereinstimmen. Ferner kann eine Rotationssymmetrie des zweiten Verbindungsglieds 50a mit der des ersten übereinstimmen. In einer Geradenstellung entspricht die Längserstreckungsrichtung 38a des Schafts 26a mit der zweiten Rotationssymmetrieachse 54a überein. Ferner stimmt in der Geradenstellung die zweite Rotationssymmetrieachse 54a mit der ersten Rotationssymmetrieachse 52a überein.

Eine Differenz einer Anzahl der mehreren ersten Verbindungsglieder und einer Anzahl der mehreren zweiten Verbindungsglieder ist von Null verschieden. Im vorliegenden Fall entspricht die Differenz dem Wert eins, so dass immer die mehreren zweiten Verbindungsglieder ein zweites Verbindungsglied 50a mehr umfassen, als die mehreren ersten Verbindungsglieder erste Verbindungsglieder umfassen. Eine Anzahl der mehreren ersten Verbindungsglieder ist ungerade. Eine Anzahl der mehreren zweiten Verbindungsglieder ist gerade. Im vorliegenden Fall umfassen die mehreren ersten Verbindungsglieder insgesamt drei erste Verbindungsglieder. Zudem umfassen im vorliegenden Fall die mehreren zweiten Verbindungsglieder insgesamt vier zweite Verbindungsglieder.

Zwei der mehreren zweiten Verbindungsglieder schließen den auslenkbaren Abschnitt 42a des Schafts 26a ab. Eines der mehreren zweiten Verbindungsglieder, vorteilhaft ein distalseitiges, ist mit der Manschette 56a verbunden. Im vorliegenden Fall ist das distalseitige zweite Verbindungsglied 50a einstückig mit der Manschette 56a verbunden. Dieses zweite Verbindungsglied 50a verbindet die Auslenkmechanik 46a wenigstens teilweise einstückig mit dem Endabschnitt 28a des Schafts 26a.

Ein weiteres der mehreren zweiten Verbindungsglieder, vorteilhaft ein proximalseitiges, ist mit der weiteren Manschette 56a verbunden. Im vorliegenden Fall ist das proximalseitige zweite Verbindungsglied 50a einstückig mit der weiteren Manschette 58a verbunden. Dieses zweite Verbindungsglied 50a verbindet die Auslenkmechanik 46a wenigstens teilweise einstückig mit dem Mittelabschnitt 32a des Schafts 26a.

Das erste Verbindungsglied 48a und das zweite Verbindungsglied 50a sind dazu eingerichtet, zu einer Auslenkung des Schafts 26a miteinander zusammenzuwirken. Das erste Verbindungsglied 48a und das zweite Verbindungsglied 50a sind in Reihe angeordnet.

Die mehreren ersten Verbindungsglieder und die mehreren zweiten Verbindungsglieder sind in Reihe angeordnet. Die mehreren ersten Verbindungsglieder und die mehreren zweiten Verbindungsglieder sind alternierend angeordnet. Die mehreren ersten Verbindungsglieder und die mehreren zweiten Verbindungsglieder sind derart angeordnet, dass auf ein erstes Verbindungsglied der mehreren ersten Verbindungsglieder ein zweites Verbindungsglied der mehreren zweiten Verbindungsglieder folgt. Ferner folgt auf ein zweites Verbindungsglied der mehreren zweiten Verbindungsglieder ein erstes Verbindungsglied der mehreren ersten Verbindungsglieder.

Ein erstes Verbindungsglied der mehreren ersten Verbindungsglieder ist von wenigstens einem zweiten Verbindungsglied der mehreren zweiten Verbindungsglieder benachbart. Ferner ist ein erstes Verbindungsglied der mehreren ersten Verbindungsglieder von zwei einander gegenüberliegenden zweiten Verbindungsgliedern der mehreren zweiten Verbindungsglieder benachbart angeordnet. Jedes der mehreren ersten Verbindungsglieder ist von zwei zweiten Verbindungsgliedern der mehreren zweiten Verbindungsglieder benachbart.

Ein zweites Verbindungsglied der mehreren zweiten Verbindungsglieder ist von wenigstens einem ersten Verbindungsglied der mehreren zweiten Verbindungsglieder benachbart. Ferner ist ein zweites Verbindungsglied der mehreren zweiten Verbindungsglieder von zwei einander gegenüberliegenden ersten Verbindungsgliedern der mehreren zweiten Verbindungsglieder benachbart angeordnet. Bis auf die Auslenkmechanik abschließende zweite Verbindungsglieder ist jedes der mehreren zweiten Verbindungsglieder von zwei ersten Verbindungsgliedern der mehreren ersten Verbindungsglieder benachbart.

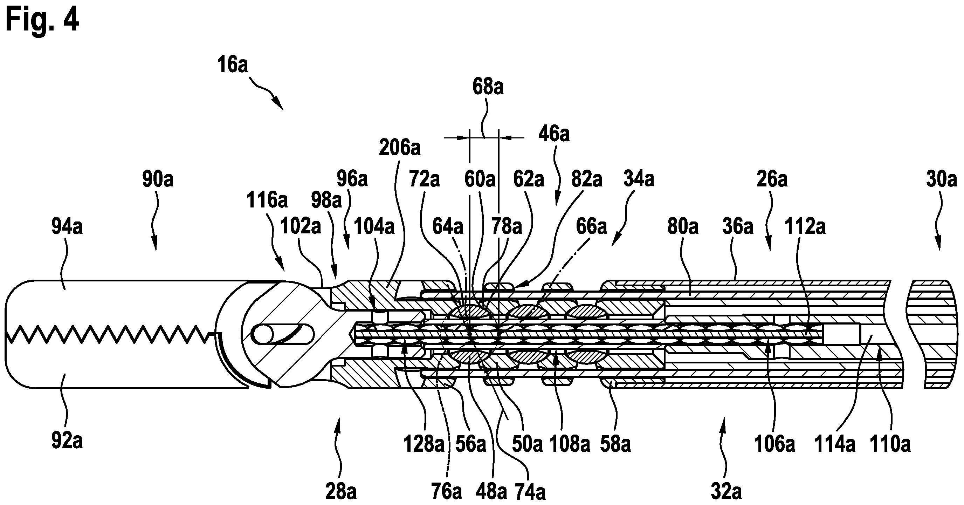

Fig. 4 zeigt eine schematische Darstellung eines sich in einer Geradenstellung befindlichen Teils der endoskopischen Vorrichtung 16a in einer Schnittansicht. Ferner ist in Fig. 3 eine schematische Darstellung eines sich in einer Auslenkungsstellung befindlichen Teils der endoskopischen Vorrichtung 16a in einer Schnittansicht gezeigt.

Das erste Verbindungsglied 48a und das zweite Verbindungsglied 50a wirken nach Art eines Kugelgelenks und/oder Wirbelkörpern zusammen. Das erste Verbindungsglied 48a weist wenigstens einen Gelenkkopf 60a auf. Das zweite Verbindungsglied 50a weist wenigstens eine Gelenkpfanne 62a auf. Die Gelenkpfanne 62a ist zu dem Gelenkkopf 60a korrespondierend ausgebildet. Derart greifen der Gelenkkopf 60a des ersten Verbindungsglieds 48a und die Gelenkpfanne 62a des zweiten Verbindungsglieds 50a ineinander ein, sodass das erste Verbindungsglied 40a und das zweite Verbindungsglied 50a zueinander beweglich gelagert sind. Denkbar ist auch eine umgekehrte Ausgestaltung, bei welcher ein erstes Verbindungsglied eine Gelenkpfanne aufweist und das zweite Verbindungsglied einen Gelenkkopf 60a.

Im vorliegenden Fall weist das erste Verbindungsglied 40a zwei gegenüberliegender Gelenkköpfe 60a auf. Der Übersichtlichkeit halber ist von den Gelenkköpfen nur der Gelenkkopf 60a mit einem Bezugszeichen versehen. Die Gelenkköpfe sind im Wesentlichen identisch miteinander ausgebildet. Im vorliegenden Fall weist das zweite Verbindungsglied 50a zwei gegenüberliegender Gelenkpfannen 62a auf. Der Übersichtlichkeit halber ist von den Gelenkpfannen nur die Gelenkpfanne 62a mit einem Bezugszeichen versehen. Die Gelenkpfannen 62a sind im Wesentlichen identisch miteinander ausgebildet. Lediglich die die Auslenkmechanik 46a abschließenden zweiten Verbindungsglieder der mehreren zweiten Verbindungsglieder weisen nur jeweils eine einzige Gelenkpfanne 62a auf.

Ein erstes Verbindungsglied 48a der mehreren ersten Verbindungsglieder ist stets von zwei gegenüberliegenden Seiten von zwei zweiten Verbindungsgliedern der mehreren zweiten Verbindungsglieder umgriffen. Anders gesagt sind gegenüberliegenden Gelenkköpfe eines einzelnen ersten Verbindungsglieds 48a der mehreren ersten Verbindungsglieder jeweils von einer Gelenkpfanne 62a zweier zweiter Verbindungsglieder der mehreren zweiten Verbindungsglieder umgriffen. Derart liegen zwei Gelenkpfannen zweier separater zweiter Verbindungsgliedern der mehreren zweiten Verbindungsgliedern an zwei Gelenkköpfen eines einzelnen ersten Verbindungsglieds 48a der mehreren ersten Verbindungsglieder an.

Ferner greifen zwei erste Verbindungsglieder stets von zwei gegenüberliegenden Seiten in ein zweites Verbindungsglied 50a der mehreren zweiten Verbindungsglieder ein. Anders gesagt greifen Gelenkköpfe zweier erster Verbindungsglieder der mehreren ersten Verbindungsglieder jeweils in eine der gegenüberliegenden Gelenkpfannen 62a eines zweiten Verbindungsglieds 50a der mehreren zweiten Verbindungsglieder ein. Derart liegen zwei Gelenkköpfe zweier separater erster Verbindungsgliedern der mehreren ersten Verbindungsgliedern an zwei Gelenkpfannen eines einzelnen zweiten Verbindungsglieds 50a der mehreren zweiten Verbindungsglieder an.

Lediglich die die Auslenkmechanik 46a abschließenden zweite Verbindungsglieder der mehreren zweiten Verbindungsglieder umgreifen nur ein einziges erstes Verbindungsglied 48a der mehreren ersten Verbindungsglieder. Anders gesagt greift nur ein Gelenkkopf 60a eines einzigen ersten Verbindungsgliedes 48a der mehreren ersten Verbindungsglieder jeweils in die einzige Gelenkpfanne 62a des die Auslenkungsmechanik abschließenden zweiten Verbindungsglieds 50a der mehreren zweiten Verbindungsglieder ein. Derart liegt nur ein einziger Gelenkköpfen eines ersten Verbindungsgliedes 48a der mehreren ersten Verbindungsgliedern in einem einzigen Gelenkkopfs 60a eines einzelnen dies Auslenkmechanik 46a abschließenden zweiten Verbindungsglieds 50a der mehreren zweiten Verbindungsglieder an.

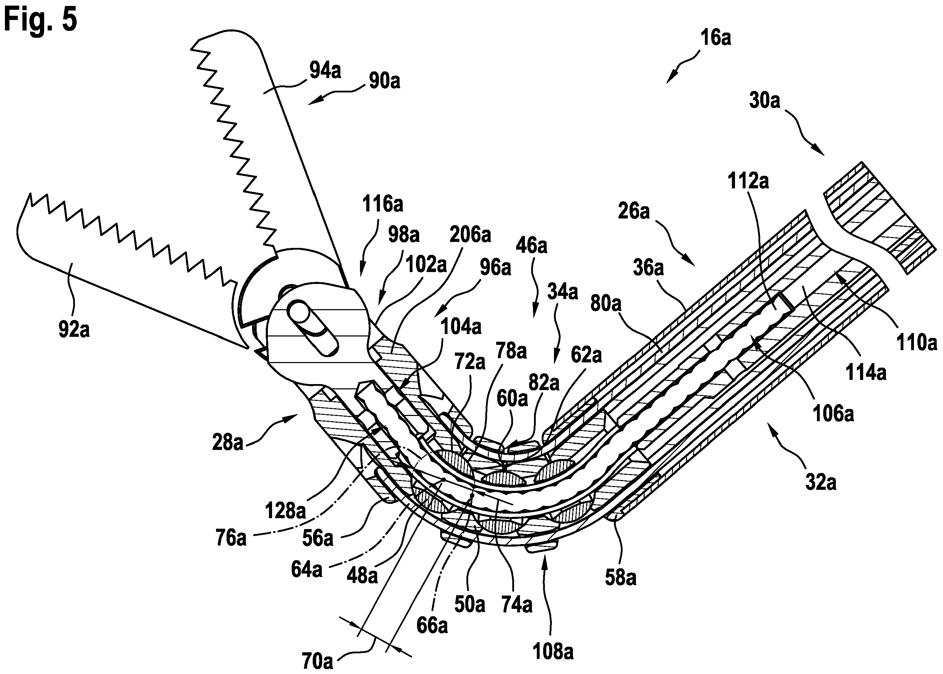

In der Geradenstellung, welche beispielsweise in den Fig. 2 und 4 dargestellt ist, stimmt eine erste Rotationssymmetrieachse 52a des ersten Verbindungsglieds 48a und zweite Rotationssymmetrieachse 54a des zweiten Verbindungsglieds 50a miteinander überein. In der Auslenkungsstellung, welche beispielsweise in den Figuren 3 und 5 dargestellt ist, sind die Haupterstreckungsrichtung des ersten Verbindungsglieds 48a und des zweiten Verbindungsglieds 50a winklig zueinander angeordnet. In der Auslenkungsstellung weist ein Winkel zwischen der ersten Rotationssymmetrieachse 52a des ersten Verbindungsglieds 48a und der zweiten Rotationssymmetrieachse 54a des zweiten Verbindungsglieds 50a zueinander höchstens 15° auf. Ein maximaler Winkel ist dabei dadurch beschränkt, dass zwei von den mehreren zweiten Verbindungsgliedern, welche ein erstes Verbindungsglied der mehreren ersten Verbindungsglieder umgreifen aneinander anschlagen.

Das erste Verbindungsglied 40a weist einen ersten geometrischen Mittelpunkt 64a auf. Ferner weist das zweite Verbindungsglied 50a einen zweiten geometrischen Mittelpunkt 66a auf. In der Geradenstellung sind der erste geometrischen Mittelpunkt 64a und der zweite geometrische Mittelpunkt 66a entlang der Längserstreckungsrichtung 38a des Schafts 26a versetzt zueinander angeordnet. In der Geradenstellung existiert ein Geradenstellungsabstand 68a zwischen dem ersten Verbindungsglied und zweiten Verbindungsglied. Der Geradenstellungsabstand 68a ist definiert durch eine kürzeste Verbindung zwischen dem ersten geometrischen Mittelpunkt 64a des ersten Verbindungsglieds 48a und dem zweiten geometrischen Mittelpunkt 66a des zweiten Verbindungsglieds 50a.

In der Auslenkungsstellung sind der erste geometrische Mittelpunkt 64a und der zweite geometrische Mittelpunkt 66a versetzt zueinander angeordnet. In der Auslenkungsstellung existiert ein Auslenkungsabstand 70a zwischen dem ersten Verbindungsglied 48a und zweiten Verbindungsglied 50a. In der Auslenkungsstellung ist der Auslenkungsabstand 70a definiert durch eine kürzeste Verbindung zwischen dem ersten geometrischen Mittelpunkt 64a des ersten Verbindungsglieds 48a und dem zweiten geometrischen Mittelpunkt 66a des zweiten Verbindungsglieds 50a. Der Auslenkungsstellungsabstand 70a in der Auslenkungsstellung ist im vorliegenden Ausführungsbeispiel gleich dem Geradenstellungsabstand 68a in der Geradenstellung. Alternativ könnte der Auslenkungsabstand, beispielsweise abhängig von einer Ausgestaltung der Verbindungsglieder, jedoch auch größer oder kleiner dem Geradenstellungsabstand 68a sein.

Das erste Verbindungsglied 40a weist wenigstens eine Außenkontur 72a auf. Die Außenkontur 72a bildet teilweise den Gelenkkopf 60a des ersten Verbindungsglieds 48a aus. Die Außenkontur 72a ist nach außen gewandt. Die Außenkontur 72a weist in Richtung einer Umgebung des Schafts 26a. Die Außenkontur 72a ist von konkav verschieden ausgebildet. Im vorliegenden Fall ist die Außenkontur 72a konvex ausgebildet. Die Außenkontur 72a entspricht einem Kreisbogen 76a. Alternativ könnte die Außenkontur wenigstens abschnittsweise eine von der Form eines Kreisbogens verschiedene Form aufweisen und zwar beispielsweise in Form einer Kreisevolvente, eines Zykloiden, eines Paraboloids und/oder eines Ellipsoids entsprechend ausgebildet sein.

Es existiert ein Durchmesser 74a eines kleinsten die Außenkontur 72a des ersten Verbindungsglieds 48a gerade noch vollständig einschließenden Kreisbogens 76a. In dem vorliegenden Ausführungsbeispiel entspricht dieser Durchmesser 74a im Wesentlichen einer maximalen Breite des ersten Verbindungsglieds. Dabei ist die Breite senkrecht zu der ersten Rotationssymmetrieachse 52a und/oder der Längserstreckungsrichtung 38a des Schafts 26a gemessen. Es ist jedoch auch denkbar, dass ein Durchmesser von einer Breite verschieden und beispielsweise größer als diese ist.

Das zweite Verbindungsglied 50a weist wenigstens eine Innenkontur 78a auf. Die Innenkontur 78a bildet wenigstens teilweise die Gelenkpfanne 62a des zweiten Verbindungsglieds 50a aus. Die Innenkontur 78a des zweiten Verbindungsglieds 48a ist zu einem Zusammenwirken mit der Außenkontur 72a des ersten Verbindungsglieds eingerichtet. Die Außenkontur 72a des ersten Verbindungsglieds 48a und die Innenkontur 78a des zweiten Verbindungsglieds 50a liegen einander gegenüber. Die Außenkontur 72a und die Innenkontur 78a liegen höchstens abschnittsweise aneinander an. Die Innenkontur 78a des zweiten Verbindungsglieds 50a ist zur Außenkontur 72a des ersten Verbindungsglieds 48a korrespondierend ausgebildet. Die Innenkontur 78a ist nach innen gewandt. Die Innenkontur 78a ist von konkav verschieden ausgebildet. Ferner ist im vorliegenden Fall die Innenkontur 78a gerade. Alternativ könnte eine Innenkontur wenigstens abschnittsweise einer insbesondere konvexen Form einer Kreisevolvente, eines Kreisbogens, eines Zykloiden, eines Paraboloids und/oder eines Ellipsoids entsprechend ausgebildet sein.

Die Auslenkmechanik 46a weist wenigstens einen Steuerstrang 80a auf. Im vorliegenden Fall weist die Auslenkmechanik 46a mehrere Steuerstränge 80a, beispielsweise wenigstens drei Steuerstränge, auf. Von den mehreren Steuersträngen ist der Übersichtlichkeit halber nur der Steuerstrang 80a mit einem Bezugszeichen versehen. Die mehreren Steuerstränge sind entlang eines Umfangs des Schafts 26a zueinander versetz angeordnet. Die mehreren Steuerstränge verlaufen im Wesentlichen parallel zueinander. Ferner sind die mehreren Steuerstränge wenigstens das erste Verbindungsglied oder sogar die mehreren ersten Verbindungsglieder koaxial umgebend angeordnet. Die mehreren Steuerstränge sind dabei im Wesentlichen identisch ausgebildet, sodass eine Beschreibung bezüglich des Steuerstrangs 80a auf die mehreren Steuerstränge übertragbar ist. Alternativ könnten die mehreren Steuerstränge auch voneinander zumindest teilweise verschieden ausgebildet sein.

Der Steuerstrang 80a ist zu einer Verstellung einer Auslenkung des auslenkbaren Abschnitts 42a des Schafft 26a eingerichtet. Der Steuerstrang 80a kann mittels einer Aktorik betätigt werden. Die Aktorik ist der Übersichtlichkeit halber hier nicht dargestellt. Dabei kann die Aktorik Teil der endoskopischen Vorrichtung 16a sein oder aber auch Teil des Chirurgieroboters 12a und zwar beispielsweise des Roboterarms 18a. Der Steuerstrang 80a erstreckt sich zumindest teilweise durch den Schaft 26a. Im vorliegenden Fall erstreckt sich der Steuerstrang 80a durch den gesamten Schaft 26a. Ferner erstreckt sich der Steuerstrang 80a sogar teilweise über den Schaft 26a hinaus, um beispielsweise mit einer Aktorik gekoppelt zu werden.

Der Steuerstrang 80a ist mit den Verbindungsgliedern 48a, 50a gekoppelt. Die Verbindungsglieder 48, 50a sind an dem Steuerstrang 80a aufgereiht. Der Steuerstrang 80a hält wenigstens in der Geradenstellung die Verbindungsglieder 48a, 50a unter Vorspannung. Alternativ oder zusätzlich könnte ein Steuerstrang zu einer Rotation eines Schafts eingerichtet sein.

Der Steuerstrang 80a ist biegeschlaff ausgebildet. Der Steuerstrang 80a ist im vorliegenden Fall als ein Draht ausgebildet. Der Steuerstrang 80a ist aus einer Litze, beispielsweise Metalllitze, ausgebildet. Der Steuerstrang 80a weist einen Durchmesser 74a auf. Der Durchmesser kann wenigstens 2,5% und/oder höchstens 25% eines Außendurchmessers des Schafts 26a betragen. Im vorliegenden Fall beträgt der Durchmesser 74a beispielsweise 0,36 mm

Der Steuerstrang 80a ist im Wesentlichen parallel zum Schaft 26a geführt. Der Steuerstrang 80a verläuft wenigstens abschnittsweise parallel zu einer Längserstreckungsrichtung 38a des Schafts 26a. Ferner ist der Steuerstrang 80a doppelt geführt. Der Steuerstrang 80a ist unterteilt in einen Abschnitt, welcher in Richtung des Endabschnitts 28a und von dem weiteren Endabschnitt 30a weggeführt ist und einen Abschnitt, welcher von dem Endabschnitt 28a weg und in Richtung des weiteren Endabschnitts 30a geführt ist.

Zur Führung des Steuerstrangs 80a weist das zweite Verbindungsglied 50a wenigstes eine Durchlassführung 82a auf. Die Durchlassführung 82a weist wenigstens trichterförmige oder zwei trichterförmige Öffnungen auf. Im vorliegenden Fall weist das zweite Verbindungsglied mehrere Durchlassführungen auf, von welchen der Übersichtlichkeit halber nur die Durchlassführung mit einem Bezugszeichen versehen ist. Die mehreren Durchlassführungen sind entlang eines Umfangs des zweiten Verbindungsglieds 50a zueinander versetz angeordnet. Die mehreren Durchlassführungen sind dabei im Wesentlichen identisch ausgebildet, so dass eine Beschreibung bezüglich der Durchlassführung 82a auf die mehreren Durchlassführungen übertragbar ist. Alternativ könnten die mehreren Durchlassführungen auch voneinander zumindest teilweise verschieden ausgebildet sein.

Jeweils zwei Durchlassführungen des zweiten Verbindungsglieds 50a führen einen Steuerstrang 80a. Eine Durchlassführung 82a des zweiten Verbindungsglieds 50a führt einen vom weiteren Endabschnitt 30a weg geführten Abschnitt des Steuerstrangs 80a und eine weitere Durchlassführung 82a des zweiten Verbindungsglieds 50a einen von dem Endabschnitt 28a weggeführten Abschnitt des Steuerstrangs 80a.

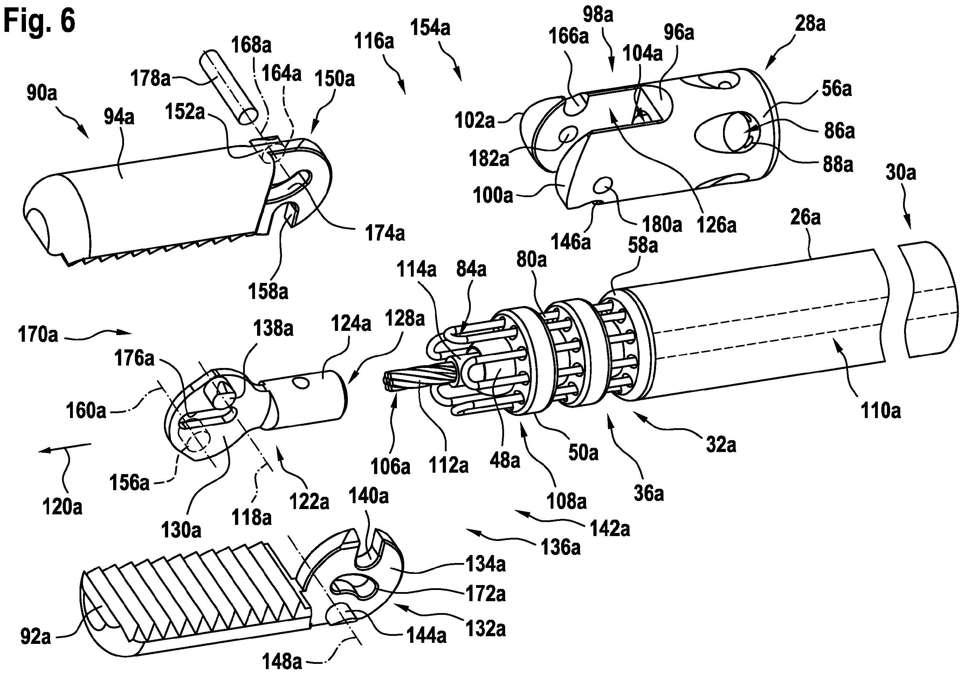

Fig. 6 zeigt eine schematische Darstellung eines Teils der endoskopischen Vorrichtung 16a in einem teilweise demontierten Zustand in einer perspektivischen Ansicht. Der Steuerstrang 80a ist mit dem Endabschnitt 28a des Schafts 26a verbunden. Ein Teil des Steuerstrangs 80a ist im Bereich des Endabschnitts 28a des Schafts 26a eine Umschlingung 84a ausbildend angeordnet.

Der Endabschnitt 28a des Schafts 26a weist wenigstens eine Strangaufnahme 86a auf. Die Strangaufnahme 86a ist an der Manschette 56a angeordnet. Der Steuerstrang 80a ist wenigstens teilweise in der Strangaufnahme 86a angeordnet. Der die Umschlingung 84a ausbildende Teil des Steuerstrangs 80a ist in der Strangaufnahme 86a angeordnet. Vor der Umschlingung 84a führt die Strangaufnahme 86a den Steuerstrang 80a in Richtung des Endabschnitts 28a des Schafts 26a. Nach der Umschlingung 84a führt die Strangaufnahme 86a den Steuerstrang 80a wieder entgegen dem Endabschnitt 28a des Schafts 26a. Zu wenigstens einem axialen Einfädeln des Steuerstrangs 80a weist die Strangaufnahme 86a wenigstens eine Durchlassführung 88a auf.

Im vorliegenden Fall weist die Strangaufnahme 86a mehrere Durchlassführungen auf. Der Übersichtlichkeit halber ist in den Durchlassführungen nur die Durchlassführung 88a mit einem Bezugszeichen versehen. Die Durchlassführungen sind an der Manschette 56a angeordnet. Die Durchlassführungen sind in Umfangsrichtung des Schafts 26a zueinander versetzt angeordnet. Jeweils zwei Durchlassführungen des Endabschnitts 28a führen einen Steuerstrang 80a. Alternativ könnten statt einem umgelenkten Steuerstrang auch zwei einzelne Steuerstränge verwendet werden. Eine Durchlassführung 82a des zweiten Verbindungsglieds 50a führt einen vom weiteren Endabschnitt 30a weg geführten Abschnitt des Steuerstrangs 80a und eine weitere Durchlassführung 88a des zweiten Verbindungsglieds 50a einen von dem Endabschnitt 28a weggeführten Abschnitt des Steuerstrangs 80a.

Die endoskopische Vorrichtung 16a weist wenigstens einen Endeffektor 90a auf. In den Fig. 2 und 4 ist der Endeffektor 90a in einem geschlossenen Betriebszustand gezeigt. In den Fig. 3 und 5 ist der Endeffektor 90a in einem geöffneten Betriebszustand gezeigt. Im vorliegenden Fall weist die endoskopische Vorrichtung 16a genau einen Endeffektor 90a auf. Der Endeffektor 90a ist an einem Endabschnitt 28a des Schafts 26a angeordnet. Der Endeffektor 90a ist wenigstens teilweise einstückig mit dem Endabschnitt 28a des Schafts 26a verbunden sein. Im vorliegenden Fall ist der Endeffektor 90a als eine Zange ausgebildet. Der Endeffektor 90a kann auch als eine Schere, eine Klemme, Zange, Skalpell, Koagulator, Stapler, Testhaken, oder dergleichen ausgebildet sein. Ein Endeffektor könnte dazu eingerichtet sein, elektrisch leitend zu sein, um vorteilhaft Strom zu übertragen. Ein Endeffektor könnte somit beispielsweise unipolar, bipolar oder dergleichen sein.

Der Endeffektor 90a umfasst wenigstens ein Werkzeugstück 92a. Im vorliegenden Fall weist der Endeffektor 90a wenigstens ein weiteres Werkzeugstück 94a auf. Das weitere Werkzeugstück 94a ist zu einem Zusammenwirken mit dem Werkzeugstück 92a eingerichtet. Das weitere Werkzeugstück 94a ist im Wesentlichen identisch zu dem Werkzeugstück 92a ausgebildet. Im vorliegenden Fall umfasst der Endeffektor 90a insgesamt zwei Werkzeugstücke 92a, 94a. Bei einem Werkzeugstück könnte es sich um ein Scherenblatt, eine Schneide, eine Elektrode oder ein anderes, insbesondere chirurgisches Werkzeugstück handeln. Im vorliegenden Fall bildet das Werkzeugstück 92a, 94a ein Maulteil aus. Bei dem Maulteil handelt es sich um eine Branche. Die Branche kann an einen spezifischen Anwendungszweck angepasst sein.

Der Endeffektor 90a weist einen Endeffektorkopf 96a auf. Der Endeffektorkopf 96a ist einstückig mit einem Endabschnitt 28a des Schafts 26a verbunden. Der Endeffektorkopf 96a ist einstückig mit der Manschette 56a ausgebildet. Ferner ist der Endeffektorkopf 96a mit dem die Auslenkmechanik 46a distal abschließenden zweiten Verbindungsgliedes einstückig verbunden.

Der Endeffektorkopf 96a weist eine Endeffektorgabel 98a auf. Die Endeffektorgabel 98a umfasst wenigstens einen Endeffektorschenkel 100a. Ferner umfasst die Endeffektorgabel 98a einen weiteren Endeffektorschenkel 102a. Der Endeffektorschenkel 100a und der weitere Endeffektorschenkel 102a sind einander gegenüberliegend angeordnet. Der Endeffektorschenkel 100a und der weitere Endeffektorschenkel 102a sind miteinander verbunden. Der Endeffektorschenkel 100a und der weitere Endeffektorschenkel 102a des Endeffektorkopfs 96a sind einstückig miteinander verbunden.

Der Endeffektorkopf 96a definiert eine Endeffektorbuchse 104a des Endeffektors 90a. In der Endeffektorbuchse 104a können weiter Komponenten der endoskopischen Vorrichtung 16a, beispielsweise ein Bewegungswandler 116a, angeordnet werden.

Die endoskopische Vorrichtung 16a weist wenigstens einen Betätigungsstrang 106a auf. Im vorliegenden Fall weist die endoskopische Vorrichtung 16a genau einen Betätigungsstrang 106a auf. Der Betätigungsstrang 106a ist zur Betätigung des Endeffektors 90a eingerichtet. Der Betätigungsstrang 106a kann mittels einer Aktorik betätigt werden. Dabei kann die Aktorik Teil der endoskopischen Vorrichtung 16a sein oder aber auch Teil des Chirurgieroboters 12a und zwar beispielsweise des Roboterarms 18a.

Der Betätigungsstrang 106a erstreckt sich zumindest teilweise durch den Schaft 26a. Der Betätigungsstrang 106a verläuft mittig durch den Schaft 26a. Im vorliegenden Fall erstreckt sich der Betätigungsstrang 106a durch den gesamten Schaft 26a. Ferner erstreckt sich der Betätigungsstrang 106a sogar teilweise über den Schaft 26a hinaus, um beispielsweise mit einer Aktorik gekoppelt zu werden.

Der Betätigungsstrang 106a ist wenigstens abschnittsweise flexibel ausgebildet. Der Betätigungsstrang 106a weist wenigstens einen flexiblen Abschnitt 108a auf. Der Betätigungsstrang 106a ist wenigstens abschnittsweise unflexibel ausgebildet. Ferner weist der Betätigungsstrang 106a wenigstens einen im unflexiblen Abschnitt 110a auf. Der unflexible Abschnitt 110a ist im Vergleich zum flexiblen Abschnitt 108 weniger flexibel. Der flexible Abschnitt 108a ist auf den unflexibleren Abschnitt 110a folgend angeordnet.

Der Betätigungsstrang 106a ist in dem Schaft 26a derart angeordnet, dass der flexible Abschnitt 108a des Betätigungsstrangs 106a kongruent ist mit dem auslenkbaren Abschnitt 42a des Schafts 26a. Im Bereich des auslenkbaren Abschnitts 42a des Schafts 26a ist der Betätigungsstrang 106a somit flexibel ausgebildet.

Der Betätigungsstrang 106aweist wenigstens eine Innentrosse 112a auf. Die Innentrosse 112a ist als eine Litze ausgebildet. Alternativ könnte die Innentrosse auch einen Volldraht aufweisen. Die Innentrosse 112a ist wenigstens zu einer mechanischen Kraftübertragung eingerichtet. Die Innentrosse 112a ist wenigstens abschnittsweise flexibel ausgebildet, beispielsweise im flexiblen Abschnitt des Betätigungsstrangs 106a. Im vorliegenden Fall ist die Innentrosse 112a über die gesamte Erstreckung des Betätigungsstrangs 106a flexibel ausgebildet.

Der Betätigungsstrang 106a weist zumindest eine Verstärkung 114a auf. Der Verstärkung 114a versteift den Betätigungsstrangs 106a wenigstens teilweise. Die Verstärkung 114a versteift den Betätigungsstrang 106a wenigstens in einem von dem flexiblen Abschnitt 108a verschiedenen Bereich des Schafts 26a. Der Verstärkung 114 versteift abschnittsweise die Innentrosse 112a. Der Verstärkung 114a ist die Innentrosse 112a koaxial umgebend angeordnet. Die Verstärkung 114a ist als ein Rohr ausgebildet. Die Verstärkung 114a ist wenigstens teilweise aus einen Metall ausgebildet. Alternativ oder zusätzlich kann die Verstärkung 114a wenigstens teilweise aus einem Kunststoff ausgebildet sein. Die Verstärkung 114a ist im unflexiblen Abschnitt 110a des Betätigungsstrangs 106a angeordnet. Der flexible Abschnitt 108a des Betätigungsdrangs 106a ist hingegen frei von einer Verstärkung 114a.

Die endoskopische Vorrichtung 16a weist wenigstens einen Bewegungswandler 116a auf. Im vorliegenden Fall weist die endoskopische Vorrichtung 16a genau einen Bewegungswandler 116a auf. Der Bewegungswandler 116a ist dazu eingerichtet, den Endeffektor 90a und den Betätigungsstrang 106a wenigstens mechanisch miteinander zu koppeln. Alternativ wäre denkbar, dass der Bewegungswandler den Endeffektor und den Betätigungsstrang auch elektrisch miteinander verbindet.

Der Bewegungswandler 116a ist zu einer Überführung einer Bewegung des Betätigungsstrangs 106a in eine Bewegung wenigstens eines Werkzeugstücks 92a eingerichtet. Die Bewegung des Betätigungsstrangs 106a ist eine Linearbewegung. Die Bewegung des Werkzeugstücks 92a ist eine Schwenkbewegung. Es wäre denkbar, dass das weitere Werkzeugstück 94a fest angeordnet oder mit anderen Worten nicht beweglich ist. Im vorliegenden Fall ist jedoch auch das weitere Werkzeugstück 94a über den Bewegungswandler 116a mit dem Betätigungsstrang 106a gekoppelt. Der Bewegungswandler 116a ist zu einer Überführung einer Bewegung des Betätigungsstrangs 106a in eine Bewegung des weiteren Werkzeugstücks 94a eingerichtet. Die Bewegung des weiteren Werkzeugstücks 94a ist eine Schwenkbewegung.

Unabhängig von einem Betriebszustand ist der Bewegungswandler 116a nichtaustretend innerhalb wenigstens eines Teils des Endeffektors 90a angeordnet. Im vorliegenden Fall ist der Bewegungswandler 116a unabhängig von einem Betriebszustand wenigstens zu einem Großteil in dem Endeffektorkopf 96a angeordnet. Der Bewegungswandler 106a ist unabhängig von einem Betriebszustand wenigstens zu einem Großteil in der Endeffektorbuchse 104a des Endeffektorkopfs 96a angeordnet. Unabhängig von einem Betriebszustand bedeckt der Endeffektorkopf 96a in einer Seitenansicht den Bewegungswandler 116a zumindest zu einem Großteil. Der Bewegungswandler 116a ist durch die Endeffektorgabel 98a seitlich bedeckt, indem dieser kongruent mit den Endeffektorschenkeln 100a, 102a der Endeffektorgabel 98a angeordnet ist. Im vorliegenden Fall bedeckt in einer Seitenansicht wenigstens ein Endeffektorschenkel 100a, 102a der Endeffektorgabel 98a des Endeffektorkopfs 96a den Bewegungswandler wenigstens zu einem Großteil.

Der Bewegungswandler 116a definiert wenigstens eine Schwenkachse 118a. Die Schwenkachse 118a ist zur Verschwenkung des Werkzeugstücks 92a eingerichtet. Die Schwenkachse 118a ist zumindest im Wesentlichen senkrecht zu einer Haupterstreckungsachse 120a des Endeffektors 90a orientiert. Die Schwenkachse 118a ist seitlich zu einer Haupterstreckungsachse 120a des Endeffektors 90a versetzt angeordnet. Anders gesagt schneiden sich die Haupterstreckungsachse 120a des Endeffektors 90a und die Schwenkachse 118a nicht. Ferner existiert eine gedachte Ebene, welche parallel zur Haupterstreckungsachse 120a des Endeffektors 90a ist und auf welcher die Schwenkachse 118a im Wesentlichen senkrecht orientiert ist.

Der Bewegungswandler 116a weist einen mechanischen Kraftweg auf. Über den mechanischen Kraftweg überträgt der Bewegungswandler 116a eine Kraft von dem Betätigungsstrang 106a wenigstens auf das Werkzeugstück 92a des Endeffektors 90a. Im vorliegenden Fall weist der Bewegungswandler 106a wenigstens einen weiteren mechanischen Kraftweg auf. Über den weiteren mechanisch Kraftweg überträgt der Bewegungswandler eine Kraft von dem Betätigungsstrang 106a auf das weitere Werkzeugstück 94a des Endeffektors 90a.

Der Bewegungswandler 116a umfasst wenigstens einen Schub- und/oder Zugkolben 122a. Im vorliegenden Fall umfasst der Bewegungswandler 116a genau einen Schub- und/oder Zugkolben 122a. Unabhängig von einem Betriebszustand ist der Schub- und/oder Zugkolben 122a wenigstens zu einem Großteil in der Endeffektorbuchse 104a angeordnet. In einer Seitenansicht ist der Schub- und/oder Zugkolben 122a von der Endeffektorgabel 98a beispielsweise von dem Endeffektorschenkel 100a und/oder dem weiteren Endeffektorschenkel 102a der Endeffektorgabel 98a verdeckt. Der Schub- und/oder Zugkolben 122a ist zumindest zur Kraftübertragung mit dem Betätigungsstrang 106a verbundenen. Ferner könnte der Schub-und/oder Zugkolben 122a elektrisch mit dem Betätigungsstrang 106a verbunden sein.

Der Schub- und/oder Zugkolben 122a ist linear geführt. Der Endeffektorkopf 96a weist eine Kolbenführung 126a auf. Die Kolbenführung 126a ist wenigstens zu einem Teil des Schub- und/oder Zugkolben 122a korrespondierend ausgebildet. Die Kolbenführung 126a ist zu einer linearen Führung des Schub- und/oder Zugkolbens 122a eingerichtet. Der Schub- und/oder Zugkolben 122a weist einen Bolzen 124a auf. Der Bolzen 124a weist eine zylindrische Form auf. Der Bolzen 124a ist in einer Kolbenführung 126a des Endeffektorkopfs 96a angeordnet.

Der Betätigungsstrang 106a und der Schub- und/oder Zugkolben 122a sind wenigstens form- und/oder kraftschlüssig miteinander verbunden. Im vorliegenden Fall sind der Schub- und/oder Zugkolben 122a sogar reibschlüssig miteinander verbunden. Der Betätigungsstrang 106a und der Schub- und/oder Zugkolben 122a sind durch eine plastische Verformung des Schub- und/oder Zugkolbens 122a und/oder des Betätigungsstrangs 106a miteinander verbunden. Der Schub- und/oder Zugkolben 122a und/oder des Betätigungsstrangs 106a sind miteinander vercrimpt. Im vorliegenden Fall ist der Bolzen 124a des Schub und/oder Zugkolbens 122a zu einer Verbindung mit dem Betätigungsstrang 106a ausgebildet.

Der Bolzen 124a des Schub und/oder Zugkolbens 122a definiert eine Betätigungsstrangaufnahme 128a. Der Betätigungsstrang 106a ist teilweise in die Betätigungsstrangaufnahme 128a eingesetzt. Der Bolzen 124a ist mit dem Betätigungsstrang 106a verpresst. Derart ist der Betätigungsstrang 106a in dem Bolzen 124a eingepresst. Alternativ oder zusätzlich könnten der Betätigungsstrang und der Schub- und/oder Zugkolben wenigstens stoffschlüssig miteinander verbunden sein. Beispielsweise könnten der Betätigungsstrang und der Schub- und/oder Zugkolben miteinander verlötet und/oder verklebt sein. Beispielsweise weist der Bolzen 124a Befüllungslöcher auf, in welche ein Adhäsiv oder Lötzinn zu einer stoffschlüssigen Verbindung in die Betätigungsstrangaufnahme eingeführt werden kann.

Der Schub- und/oder Zugkolben 122a weist einen Anker 130a auf. Der Anker 130a ist im Wesentlichen plattenförmig ausgebildet. Der Anker 130a weist die Form eines im Wesentlichen kreisförmigen Umrisses auf. Die Endeffektorgabel 98a bildet einen Anschlag für den Anker 130a aus. Der Anker 130a ist wenigstens in einer Dimension größer, als die Kolbenführungsaufnahme. Derart begrenzt der Anker 130a eine Linearbewegung des Schub- und/oder Zugkolbens 122a bzw. des Betätigungsstrangs 106a. Der Anker 130a ist in der Endeffektorbuchse 104a angeordnet. Der Anker 130a ist in einer Seitenansicht von der Endeffektorgabel 98a verdeckt, beispielsweise von dem Endeffektorschenkel 98a und/oder dem weiteren Endeffektorschenkel 102a der Endeffektorgabel 98a. Der Anker 130a ist mit dem Bolzen 124a verbunden.

Der Schub- und/oder Zugkolben 122a ist zumindest teilweise einstückig ausgebildet. Im vorliegenden Fall sind der Anker 130a und der Bolzen 124a des Schub- und/oder Zugkolbens 122a miteinander einstückig verbunden. Alternativ könnte der Schub- und/oder Zugkolben auch mehrteilig ausgebildet sein. Im vorliegen Fall sind der Anker 130a und der Bolzen 124a einstückig miteinander verbunden. Der Schub- und/oder Zugkolben 122a ist zumindest teilweise aus Metall ausgebildet. Beispielsweise kann es sich bei dem Schub-und/oder Zugkolben 122a auch um ein Spritzgussbauteil handeln.

Der Bewegungswandler 116a weist wenigstens einen Schwenkhebel 132a auf. Der Schwenkhebel 132a ist wenigstens mechanisch mit den Schub- und/oder Zugkolben 122a verbunden. Der Schwenkhebel 132a ist mit dem Endeffektor 90a verbunden. Der Schwenkhebel 132a ist mit dem Werkzeugstück 92a verbunden. Im vorliegenden Fall ist der Schwenkhebel 132a einstückig mit dem Werkzeugstück 92a verbunden. Der Schwenkhebel 132a ist wenigstens teilweise in der Endeffektorbuchse 104a angeordnet. Im vorliegenden Fall ist der Schwenkhebel 132a wenigstens teilweise in der Endeffektorbuchse 104a angeordnet. Der Schwenkhebel 132a ist in einer Seitenansicht von der Endeffektorgabel 98a verdeckt, beispielsweise von dem Endeffektorschenkel 100a und/oder dem weiteren Endeffektorschenkel 102a der Endeffektorgabel 98a. Der Schwenkhebel 132a liegt an dem Schub- und/oder Zugkolben 122a an und zwar beispielsweise an dem Anker 130a des Schub- und/oder Zugkolbens 122a.

Der Schwenkhebel 132a weist einen Schwenkhebelgrundkörper 134a auf. Der Schwenkhebelgrundkörper 134a ist im Wesentlichen plattenförmig ausgebildet. In einer Seitenansicht weist der Schwenkhebelgrundkörper 134a einen kreisförmigen Umriss auf. Der Schwenkhebelgrundkörper 134a ist einstückig mit dem Werkzeugstück 92a ausgebildet.

Der Bewegungswandler 116aweist einen Koppelmechanismus 136a auf. Der Koppelmechanismus 136a ist wenigstens zu einer mechanischen Kopplung des Schwenkhebels 132a und des Schub- und/oder Zugkolbens 122a eingerichtet. Der Koppelmechanismus 136a ist wenigstens teilweise von dem Schwenkhebel 132a ausgebildet. Ferner ist der Koppelmechanismus 136a wenigstens teilweise von dem Schub- und/oder Zugkolben 122a gebildet. Der Koppelmechanismus 136a weist wenigstens ein Koppelelement 138a auf. Der Koppelmechanismus 136a weist wenigstens ein korrespondierendes Koppelelement 140a auf. Das korrespondierende Koppelelement 140a ist zu dem Koppelelement 138a korrespondierend ausgebildet. Das Koppelelement 138a und das korrespondierende Koppelelement 140a definieren gemeinsam die Schwenkachse 118a des Bewegungswandlers 116a, welche zumindest im Wesentlichen senkrecht zu einer Haupterstreckungsachse 120a des Endeffektors 90a orientiert und zu dieser seitlich versetzt angeordnet ist.

Das Koppelelement 138a ist Teil des Schub- und/oder Zugkolben 122a. Das Koppelelement 138a ist an dem Anker 130a des Schub- und/oder Zugkolbens 122a angeordnet. Das Koppelelement 138a ist fest mit dem Anker 130a verbunden. Das Koppelelement 138a ist zu einem geometrischen Mittelpunkt 64a, 66a des Ankers 130a versetzt angeordnet. Das Koppelelement 138a ist zu der Haupterstreckungsachse 120a versetzt angeordnet. Im vorliegenden Fall ist das Koppelelement 138a als eine Nocke ausgebildet.

Das korrespondierende Koppelelement 140a ist Teil des Schwenkhebels 132a. Das korrespondierende Koppelelement 140a ist an dem Schwenkhebelgrundkörper 134a angeordnet bzw. mit diesem verbunden. Das korrespondierende Koppelelement 140a ist zu einem geometrischen Mittelpunkt 64a, 66a des Schwenkhebelgrundkörpers 134a versetzt angeordnet. Das korrespondierende Koppelelement 140a ist zu der Haupterstreckungsachse 120a des Endeffektors 120a versetzt angeordnet. Im vorliegenden Fall ist das korrespondierende Koppelelement 140a als ein Nockenmitenehmer, beispielsweise in Form einer seitlich geöffneten Ausnehmung des Schwenkhebels 132a, ausgebildet. Sind der Schub- und/oder Zugkolben 122a und der Schwenkhebel 132a mittels des Koppelmechanismus 136a miteinander gekoppelt, so greifen das Koppelelement 138a und das korrespondierende Koppelelement 140a ineinander ein und kontaktieren sich gegenseitig. Alternativ könnten die Ausgestaltungen des Koppelelements und des korrespondierenden Koppelelements auch miteinander vertauscht sein, beispielsweise könnte somit das Koppelelement als ein Nockenmitnehmer und das korrespondierende Komplement als eine Nocke ausgebildet sein.

Der Bewegungswandler 116aweist ein Drehlager 142a auf. Das Drehlager 142a ist wenigstens zu einer drehbaren Lagerung des Werkzeugstücks 92a relativ zu dem Endeffektorkopf 96a eingerichtet. Das Drehlager 142a ist wenigstens teilweise von dem Schwenkhebel 132a gebildet. Ferner ist das Drehlager 142a wenigstens teilweise von dem Endeffektorkopf 96a gebildet. Das Drehlager 142a weist wenigstens ein Lagerelement 144a auf. Das Drehlager 142a weist wenigstens ein korrespondierendes Lagerelement 146a auf. Das korrespondierende Lagerelement 146a ist korrespondierend zu dem Lagerelement 144a ausgebildet. Das Lagerelement 144a und das korrespondierende Lagerelement 146a definieren gemeinsam eine Drehachse 148a, um welche sich das Werkzeugstück 92a bei einer Betätigung des Werkzeugstücks 92a dreht. Die Drehachse 148a ist zumindest im Wesentlichen senkrecht zu einer Haupterstreckungsachse 120a des Endeffektors 90a orientiert und zu dieser seitlich versetzt angeordnet. Ferner ist die Drehachse 148a im Wesentlichen parallel zu der Schwenkachse 118a angeordnet. Bezüglich einer Haupterstreckungsachse 120a des Endeffektors 90a liegt die Drehachse 148a der Schwenkachse 118a gegenüber.

Das Lagerelement 144a ist Teil des Schwenkhebels 132a. Das Lagerelement 144a ist an dem Schwenkhebelgrundkörper 134a angeordnet bzw. mit diesem verbunden. Das Lagerelement 144a ist zu einem geometrischen Mittelpunkt 64a, 66a des Schwenkhebelgrundkörpers 134a versetzt angeordnet. Das Lagerelement 144a ist zu der Haupterstreckungsachse 120a des Endeffektors 90a versetzt angeordnet. Das Lagerelement 144a liegt dem korrespondierenden Koppelelement 140a gegenüber. Im vorliegenden Fall ist das Lagerelement 144a als eine Nocke ausgebildet.

Das korrespondierende Lagerelement 146a ist Teil des Endeffektorkopfs 96a. Das korrespondierende Lagerelement 146a ist an dem Endeffektorschenkel 100a der Endeffektorgabel 98a angeordnet bzw. verbunden. Das korrespondierende Lagerelement 146a ist zu einem geometrischen Mittelpunkt 64a, 66a des Endeffektorschenkels 100a versetzt angeordnet. Das korrespondierende Lagerelement 146a ist zu der Haupterstreckungsachse 120a des Endeffektors 90a versetzt angeordnet. Im vorliegenden Fall ist das korrespondierende Lagerelement 146a als ein Nockenmitnehmer, beispielsweise in Form einer seitlich geöffneten Ausnehmung des Endeffektorschenkels 100a, ausgebildet. Sind der Schwenkhebel 132a und der Endeffektorkopf 96a mittels des Drehlagers 142a miteinander drehbar gelagert, so greifen das Lagerelement 144a und das korrespondierende Koppelelement 140a ineinander ein und kontaktieren sich gegenseitig. Alternativ könnten die Ausgestaltungen des Lagerelements und des korrespondierenden Lagerelements auch miteinander vertauscht sein, beispielsweise könnte somit das Lagerelement als ein Nockenmitnehmer und das korrespondierende Lagerelement als eine Nocke ausgebildet sein.

Der Bewegungswandler 116a weist wenigstens einen weiteren Schwenkhebel 150a auf. Der weiteren Schwenkhebel 150a ist wenigstens mechanisch mit den Schub- und/oder Zugkolben 122a verbunden. Der weiteren Schwenkhebel 150a ist mit dem Endeffektor 90a verbunden. Der weiteren Schwenkhebel 150a ist mit dem weiteren Werkzeugstück 94a verbunden. Im vorliegenden Fall ist der weiteren Schwenkhebel 150a einstückig mit dem weiteren Werkzeugstück 94a verbunden. Der weitere Schwenkhebel 150a ist wenigstens teilweise in der Endeffektorbuchse 104a angeordnet. Im vorliegenden Fall ist der weiteren Schwenkhebel 150a wenigstens teilweise in der Endeffektorbuchse 104a angeordnet. Der weitere Schwenkhebel 150a ist in einer Seitenansicht von der Endeffektorgabel 98a verdeckt, beispielsweise von dem Endeffektorschenkel 100a und/oder dem weiteren Endeffektorschenkel 102a der Endeffektorgabel 98a. Der weiteren Schwenkhebel 150a liegt an dem Schub- und/oder Zugkolben 122a an und zwar beispielsweise an dem Anker 130a des Schub- und/oder Zugkolbens 122a. Der weiteren Schwenkhebel 150a liegt auf einer dem Schwenkhebel 132a gegenüberliegenden Seite an dem Schub- und/oder Zugkolben 122a an.

Der weiteren Schwenkhebel 150a weist einen weiteren Schwenkhebelgrundkörper 152a auf. Der weiteren Schwenkhebelgrundkörper 152a ist plattenförmig ausgebildet. In einer Seitenansicht weist der weiteren Schwenkhebelgrundkörper 152a einen kreisförmigen Umriss auf. Der weitere Schwenkhebelgrundkörper 152a ist einstückig mit dem weiteren Werkzeugstück 94a ausgebildet.

Der Bewegungswandler 116a weist einen weiteren Koppelmechanismus 154a auf. Der weitere Koppelmechanismus 154a ist wenigstens zu einer mechanischen Kopplung des weiteren Schwenkhebels 150a und des Schub- und/oder Zugkolbens 122a eingerichtet. Der weitere Koppelmechanismus 154a ist wenigstens teilweise von dem weiteren Schwenkhebel 150a ausgebildet. Ferner ist der weitere Koppelmechanismus 154a wenigstens teilweise von dem Schub- und/oder Zugkolben 122a gebildet. Der weitere Koppelmechanismus 154a weist wenigstens ein weiteres Koppelelement 156a auf. Der weitere Koppelmechanismus 154a weist wenigstens ein weiteres korrespondierendes Koppelelement 158a auf. Das weitere korrespondierende Koppelelement 158a ist zu dem Koppelelement 156a korrespondierend ausgebildet. Das weitere Koppelelement 156a und das weitere korrespondierende Koppelelement 158a definieren gemeinsam die weitere Schwenkachse 160a des Bewegungswandlers 116a, welche zumindest im Wesentlichen senkrecht zu einer Haupterstreckungsachse 120a des Endeffektors 90a orientiert und zu dieser seitlich versetzt angeordnet ist. Die weiteres Schwenkachse 160a liegt in Bezug zu der Haupterstreckungsachse 120a der Schwenkachse 118a gegenüber. Die weitere Schwenkachse 160a ist im Wesentlichen parallel zu der Schwenkachse 108a.

Das weitere Koppelelement 156a ist Teil des Schub- und/oder Zugkolben 122a. Das weitere Koppelelement 156a ist an dem Anker 130a des Schub- und/oder Zugkolbens 122a angeordnet. Das weitere Koppelelement 156a ist auf Seite des Ankers 130a angeordnet, welcher der Seite an welcher das Koppelelement 138a angeordnet ist, gegenüberliegt. Das weitere Koppelelement 156a ist fest mit dem Anker 130a verbunden. Das weitere Koppelelement 156a ist zu einem geometrischen Mittelpunkt 64a, 66a des Ankers 130a versetzt angeordnet. Das weitere Koppelelement 156a ist zu der Haupterstreckungsachse 120a versetzt angeordnet. Im vorliegenden Fall ist das weitere Koppelelement 156a als eine Nocke ausgebildet.

Das weitere korrespondierende Koppelelement 158a ist Teil des weiteren Schwenkhebels 150a. Das weitere korrespondierende Koppelelement 158a ist an dem weiteren Schwenkhebelgrundkörper 152a angeordnet bzw. mit diesem verbunden. Das weitere korrespondierende Koppelelement 158a ist zu einem geometrischen Mittelpunkt 64a, 66a des weiteren Schwenkhebelgrundkörpers 152a versetzt angeordnet. Das weitere korrespondierende Koppelelement 158a ist zu der Haupterstreckungsachse 120a des Endeffektors 90a versetzt angeordnet. Im vorliegenden Fall ist das weitere korrespondierende Koppelelement 158a als ein Nockenmitnehmer, beispielsweise in Form einer seitlich geöffneten Ausnehmung des weiteren Schwenkhebels 150a, ausgebildet. Sind der Schub- und/oder Zugkolben 122a und der weitere Schwenkhebel 150a mittels des weiteren Koppelmechanismus 154a miteinander gekoppelt, so greifen das weitere Koppelelement 156a und das korrespondierende Koppelelement 158a ineinander ein und kontaktieren sich gegenseitig. Alternativ könnten die Ausgestaltungen des weiteren Koppelelements und des weiteren korrespondierenden Koppelelements auch miteinander vertauscht sein, beispielsweise könnte somit das weitere Koppelelement als ein Nockenmitnehmer und das weitere korrespondierende Komplement als eine Nocke ausgebildet sein.

Der Bewegungswandler 116a weist ein weiteres Drehlager 162a auf. Das weitere Drehlager 162a ist wenigstens zu einer drehbaren Lagerung des weiteren Werkzeugstücks 94a relativ zu dem Endeffektorkopf 96a eingerichtet. Das weitere Drehlager 162a ist wenigstens teilweise von dem weiteren Schwenkhebel 150a gebildet. Ferner ist das weitere Drehlager 162a wenigstens teilweise von dem Endeffektorkopf 96a gebildet. Das weitere Drehlager 162a weist wenigstens ein weiteres Lagerelement 164a auf. Das weitere Drehlager 162a weist wenigstens ein weiteres korrespondierendes Lagerelement 166a auf. Das weitere korrespondierende Lagerelement 166a ist korrespondierend zu dem weiteren Lagerelement 164a ausgebildet. Das weitere Lagerelement 164a und das weitere korrespondierende Lagerelement 166a definieren gemeinsam eine weitere Drehachse 168a, um welche sich das weitere Werkzeugstück 94a bei einer Betätigung des weiteren Werkzeugstücks 94a dreht. Die weitere Drehachse 168a ist zumindest im Wesentlichen senkrecht zu einer Haupterstreckungsachse 120a des Endeffektors 90a orientiert und zu dieser seitlich versetzt angeordnet. Ferner ist die weitere Drehachse 168a im Wesentlichen parallel zu der weiteren Schwenkachse 160a angeordnet. Bezüglich eines Haupterstreckungsachse 120a des Endeffektors 90a liegt die weitere Drehachse 168a der weiteren Schwenkachse 160a gegenüber.

Das weitere Lagerelement 164a ist Teil des weiteren Schwenkhebels 150a. Das weitere Lagerelement 164a ist an dem weiteren Schwenkhebelgrundkörper 152a angeordnet bzw. mit diesem verbunden. Das weitere Lagerelement 164a ist zu einem geometrischen Mittelpunkt 64a, 66a des weiteren Schwenkhebelgrundkörpers 152a versetzt angeordnet. Das weitere Lagerelement 164a ist zu der Haupterstreckungsachse 120a des Endeffektors 90a versetzt angeordnet. Das weitere Lagerelement 164a liegt dem korrespondierenden weiteren Koppelelement 156a gegenüber. Im vorliegenden Fall ist das weitere Lagerelement 164a als eine Nocke ausgebildet.

Das weitere korrespondierende Lagerelement 166a ist Teil des Endeffektorkopfs 96a. Das weitere korrespondierende Lagerelement 166a ist an dem weiteren Endeffektorschenkel 102a der Endeffektorgabel 98a angeordnet bzw. verbunden. Das weitere korrespondierende Lagerelement 166a ist zu einem geometrischen Mittelpunkt 64a, 66a des weiteren Endeffektorschenkels 102a versetzt angeordnet. Das weitere korrespondierende Lagerelement 166a ist zu der Haupterstreckungsachse 120a des Endeffektors 90a versetzt angeordnet. Im vorliegenden Fall ist das weitere korrespondierende Lagerelement 166a als ein Nockenmitnehmer, beispielsweise in Form einer seitlich geöffneten Ausnehmung des weiteren Endeffektorschenkels 102a, ausgebildet. Sind der weitere Schwenkhebel 150a und der Endeffektorkopf 96a mittels des weiteren Drehlagers 162a miteinander drehbar gelagert, so greifen das weitere Lagerelement 164 und das weitere korrespondierende Koppelelement 158a ineinander ein und kontaktieren sich gegenseitig. Alternativ könnten die Ausgestaltungen des weiteren Lagerelements und des weiteren korrespondierenden Lagerelements auch miteinander vertauscht sein, beispielsweise könnte somit das weitere Lagerelements als ein Nockenmitnehmer und das weitere korrespondierende Lagerelement als eine Nocke ausgebildet sein.

Der Bewegungswandler 116a weist ein Führungslager 170a auf. Das Führungslager 170a ist dazu eingerichtet, Bestandteile des Bewegungswandlers 116a zu führen. Zur Führung des Schwenkhebels 132a weist das Führungslager 170a eine Kulissenführung 172a auf. Die Kulissenführung 172a ist in Form eines kurvigen Langlochs ausgebildet. Die Kulissenführung 172a ist durch den Schwenkhebel 132a definiert. Die Kulissenführung 172a erstreckt sich durch einen geometrischen Mittelpunkt 64a, 66a des Schwenkhebels 132a. Die Kulissenführung 172a ist von einer Ausnehmung des Schwenkhebelgrundkörpers 134a gebildet.

Zur Führung des weiteren Schwenkhebels 150a weist das Führungslager 170a eine weitere Kulissenführung 174a auf. Die weitere Kulissenführung 174a ist in Form eines kurvigen Langlochs ausgebildet. Wenigstens die weitere Kulissenführung 174a ist im Vergleich zu der Kulissenführung 172a um 180° gedreht. Die weitere Kulissenführung 174a ist durch den weiteren Schwenkhebel 150a definiert. Die weitere Kulissenführung 174a erstreckt sich durch einen geometrischen Mittelpunkt 64a, 66a des weiteren Schwenkhebels 150a. Die weitere Kulissenführung 174a ist von einer Ausnehmung des weiteren Schwenkhebelgrundkörpers 152a gebildet.

Zur Führung des Schub- und/oder Zugkolbens 122a weist das Führungslager 170a eine zusätzliche Kulissenführung 176a auf. Die zusätzliche Kulissenführung 176a ist in Form eines geraden Langlochs ausgebildet. Die zusätzliche Kulissenführung 176a ist durch das Schub- und/oder Zugkolbens 122a definiert. Die weitere Kulissenführung 174a erstreckt sich durch einen geometrischen Mittelpunkt 64a, 66a des Ankers 130a des Schub- und/oder Zugkolbens 122a. Die zusätzliche Kulissenführung 176a ist von einer Ausnehmung des weiteren Ankers 130a gebildet.

Ferner umfasst das Führungslager 170a einen Führungszapfen 178a auf. Der Führungszapfen 178a ist durch die Kulissenführung 172a erstreckend angeordnet. Zudem ist der Führungszapfen 178a durch die zusätzliche Kulissenführung 176a erstreckend angeordnet. Ferner ist der Führungszapfen 178a durch die weitere Kulissenführung 174a erstreckend angeordnet. Der Führungszapfen 178a ist mit dem Endeffektorkopf 96a und zwar beispielsweise mit der Endeffektorgabel 98a verbunden. Der Endeffektorschenkel 100a der Endeffektorgabel 98a weist eine Zapfenaufnahme 180a auf. Die Zapfaufnahme ist zu einer form- und/oder kraftschlüssigen Verbindung mit dem Führungszapfen 178a ausgebildet. Ferner weist der weitere Endeffektorschenkel 102a der Endeffektorgabel 98a eine weitere Zapfenaufnahme 182a auf. Die weitere Zapfaufnahme 182a ist zu einer form- und/oder kraftschlüssigen Verbindung mit dem Führungszapfen 178a ausgebildet. In einem montierten Zustand erstreckt sich der Führungszapfen 178a durch die Zapfaufnahme 180a, die Kulissenführung 172a, die zusätzliche Kulissenführung 176a, die weitere Kulissenführung 174a und die weitere Zapfenaufnahmen 182a. Der Führungszapfen 178a sichert den Schwenkhebel, den weiteren Schwenkhebel 150a und den Schub- und/oder Zugkolben 122a an dem Endeffektorkopf 96a.

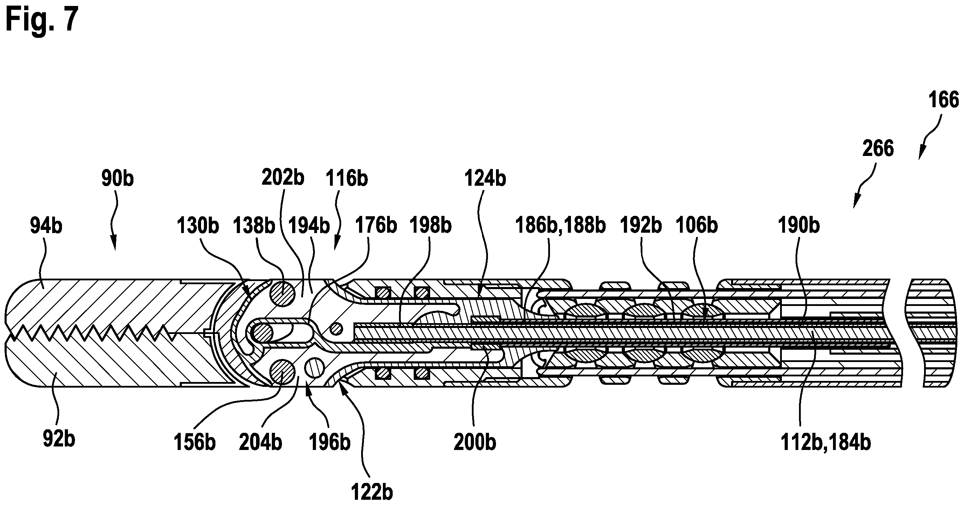

In den Figuren 7 bis 27 sind weitere offenbarungsgemäße Ausführungsbeispiele gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 6 verwiesen wird. Auch sollen sämtliche Kombinationen der hier genannten Ausführungsbeispiele als mit offenbart gelten. Zur Unterscheidung der Ausführungsbeispiele ist der Buchstabe a den Bezugszeichen des Ausführungsbeispiels in den Figuren 1 bis 6 nachgestellt. In den Ausführungsbeispielen der Figuren 7 bis 27 ist der Buchstabe a durch die Buchstaben b bis j ersetzt.

Fig. 7 zeigt eine schematische Darstellung eines weiteren Ausführungsbeispiels wenigstens eines Teils einer endoskopischen Vorrichtung 16b gemäß den Prinzipien der vorliegenden Offenbarung in einer Schnittansicht längs eines Schafts 26b der endoskopischen Vorrichtung 16b. Das vorliegende Ausführungsbeispiel unterscheidet sich von dem vorhergehenden im Wesentlichen durch eine Elektrifizierung der endoskopischen Vorrichtung 16b.

Die endoskopische Vorrichtung 16b weist einen Betätigungsstrang 106b auf. Der Betätigungsstrang 106b weist wenigstens einen elektrischen Polleiter 184b auf. Der elektrische Polleiter 184b ist zur Bereitstellung wenigstens eines elektrischen Potentials für wenigstens ein Werkzeugstück 92b eines Endeffektors 90b der endoskopischen Vorrichtung 16b eingerichtet. Der elektrische Polleiter 184b ist als ein Innenleiter ausgebildet. Der elektrische Polleiter 184b ist von einer Innentrosse 112b des Betätigungsstrangs 106b ausgebildet. Denkbar ist, dass der elektrische Polleiter zur Bereitstellung eines gleichen elektrischen Potentials für das Werkzeugstück und das weitere Werkzeugstück eingerichtet sein kann.

Der Betätigungsstrang 106b weist wenigstens einen weiteren elektrischen Polleiter 186b auf. Der weitere elektrische Polleiter 186b ist zur Bereitstellung wenigstens eines weiteren elektrischen Potentials für ein weiteres Werkzeugstück 94b des Endeffektors 90b der endoskopische Vorrichtung 16b eingerichtet. Der elektrische Polleiter 184b weist eine Haupterstreckung auf. Ferner weist der weitere elektrische Polleiter 186b eine weitere Haupterstreckung auf. Die Haupterstreckung des elektrischen Polleiters 184b ist größer als eine weitere Haupterstreckung des weiteren elektrischen Polleiters 186b. Der weiteren elektrischen Polleiter 186b ist von dem elektrischen Polleiter 184b separat ausgebildet. Der weiteren elektrischen Polleiter 186b ist zur Bereitstellung wenigstens eines weiteren elektrischen Potentials eingerichtet. Der weitere elektrische Polleiter 186b umgibt den elektrischen Polleiter 184b koaxial. Der weitere elektrische Polleiter 186b ist als ein Außenleiter ausgebildet. Der weitere elektrische Polleiter 186b ist schlauchförmig ausgebildet. Der weitere elektrische Polleiter 186b ist wenigstens teilweise von einem Geflecht ausgebildet. Der Betätigungsstrang 106b weist eine Außentrosse 188b auf. Die Außentrosse 188b ist die Innentrosse 112b umgebend angeordnet. Die Außentrosse 188b bildet den weiteren elektrischen Polleiter 186b aus.

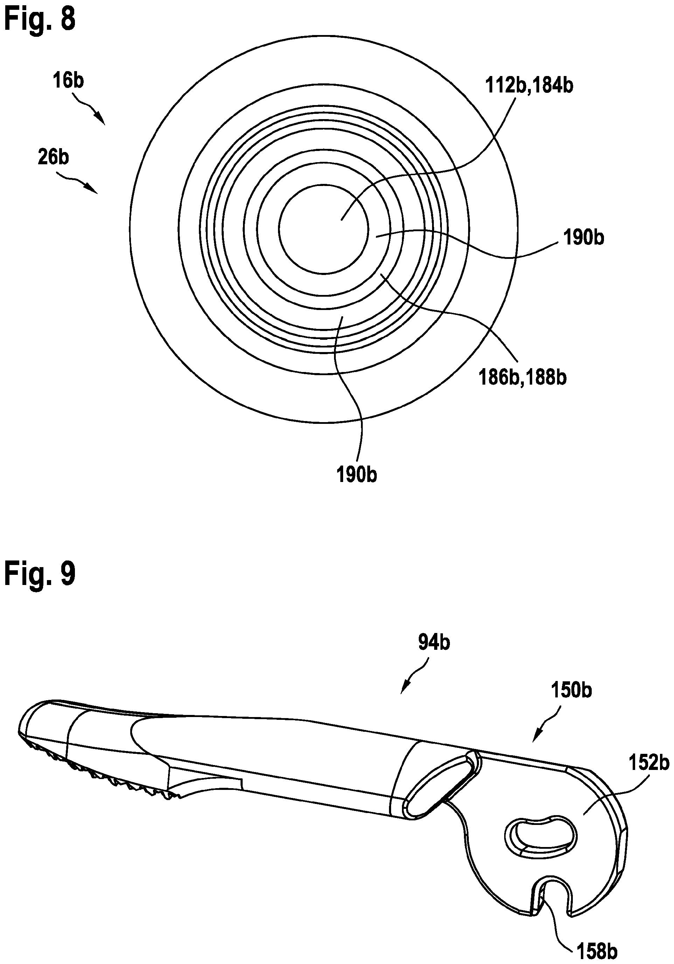

Fig. 8 zeigt eine schematische Darstellung wenigstens eines Teils der endoskopischen Vorrichtung 16b in einer Schnittansicht quer zum Schaft 16b. Der Betätigungsstrang 106b weist wenigstens einen elektrischen Isolator 190b auf. Der elektrische Isolator 190b ist wenigstens teilweise von einem Isolationsmaterial ausgebildet. Das Isolationsmaterial weist einen CTI Wert von wenigstens 150 auf. Im vorliegenden Fall weist das Isolationsmaterial sogar einen CTI Wert von mehr als 600 auf. Bei dem Isolationsmaterial kann es sich beispielsweise um Peek handeln. Im vorliegenden Fall ist das Isolationsmaterial ein Tetrafluorethylen-Hexafluorpropylen-Copolymer (FEP) oder ein Perfluoralkoxy-Polymer (PFA) Der Kunststoff kann flexibel und/oder elastisch sein. Der elektrische Isolator 190b umgibt den elektrischen Polleiter 184b koaxial. Der elektrische Isolator 190b ist zwischen dem elektrische Polleiter 184b und dem weiteren elektrischen Polleiter 186b angeordnet. Der Betätigungsstrang 106b weist wenigstens einen weiteren elektrischen Isolator 192b auf. Der weitere elektrische Isolator 192b umgibt den weiteren elektrischen Polleiter 186b koaxial.

Die endoskopische Vorrichtung 16b weist einen Bewegungswandler 116b auf (vgl. Fig. 7). Der Bewegungswandler 116b ist dazu eingerichtet, den Endeffektor 90b und den Betätigungsstrang 106b mechanisch zu koppeln. Im vorliegenden Ausführungsbeispiel ist der Bewegungswandler 116b zusätzlich dazu eingerichtet, den Endeffektor 90b und den Betätigungsstrang 106b elektrisch zu koppeln. Der Bewegungswandler 116b verbindet wenigstens den elektrischen Polleiter 184b mit dem Werkzeugstück 92b. Im vorliegenden Fall verbindet der Bewegungswandler 116b den elektrischen Polleiter 184b elektrisch mit dem Werkzeugstück 92b. Ferner verbindet der Bewegungswandler 116b den weiteren elektrischen Polleiter 186b elektrisch mit dem weiteren Werkzeugstück 94b.

Ein mechanischer Kraftweg des Bewegungswandlers 116b, über welchen eine Kraftübertragung von dem Betätigungsstrang 106b auf das Werkzeugstück 92b erfolgt, und ein elektrischer Leitweg des Bewegungswandlers 116b, über welchen eine Übertragung des elektrischen Potentials auf das Werkzeugstück 92b erfolgt, sind im vorliegenden Fall identisch. Ferner ist ein mechanischer Kraftweg des Bewegungswandlers 116b, über welchen eine Kraftübertragung von dem Betätigungsstrang 106b auf das weitere Werkzeugstück 94b erfolgt, und ein elektrischer Leitweg des Bewegungswandlers 116b, über welchen eine Übertragung des weiteren elektrischen Potentials auf das weitere Werkzeugstück 94b erfolgt, im vorliegenden Fall identisch.

Der Bewegungswandler 116b ist teilweise elektrisch leitend ausgebildet. Der Bewegungswandler 116b besteht dazu zumindest teilweise aus einem Metall. Der Bewegungswandler 116b ist teilweise aus einem weiteren Isolationsmaterial ausgebildet. Das weitere Isolationsmaterial weist einen CTI Wert von wenigstens 150 auf. Im vorliegenden Fall weist das weitere Isolationsmaterial sogar einen CTI Wert von mehr als 600 auf. Bei dem weiteren Isolationsmaterial kann es sich beispielsweise um Peek handeln. Im vorliegenden Fall ist das weitere Isolationsmaterial ein Cycloolefin-Copolymer (COC) und/oder Polymethylpenten. Lediglich Bauteile des Bewegungswandlers 116b, welche zu einer Bewegungsübertragung von dem Betätigungsstrang 106b auf das Werkzeugstück 92b eingerichtet sind, sind zur Durchleitung des elektrischen Potentials wenigstens teilweise frei von einem Isolationsmaterial. Lediglich Bauteile des Bewegungswandlers 116b, welche zu einer Bewegungsübertragung von dem Betätigungsstrang 106b auf das weitere Werkzeugstück 94b eingerichtet sind, sind zur Durchleitung des weiteren elektrischen Potentials wenigstens teilweise frei von einem Isolationsmaterial.

Zu einer elektrischen Verbindung weist ein Schub- und/oder Zugkolben 122b des Bewegungswandlers 116b weist wenigstens einen elektrischen Polleiterfortsatz 194a auf. Der elektrische Polleiterfortsatz 194b ist mit dem elektrischen Polleiter 184b des Betätigungsstrangs 106b elektrisch verbunden. Ferner ist der elektrische Polleiterfortsatz 194b mechanisch mit dem elektrischen Polleiter 184b des Betätigungsstrangs 106b verbunden.

Der elektrische Polleiterfortsatz 194b erstreckt sich teilweise durch einen Anker 130b des Schub- und/oder Zugkolbens 122b. Im Bereich des Ankers 130b ist der elektrische Polleiterfortsatz 194b mit einer weiteren Komponente des Bewegungswandlers 116b elektrisch und/oder mechanischen verbunden. Ferner erstreckt sich der elektrische Polleiterfortsatz 194b zumindest teilweise durch einen Bolzen 124b des Schub und/oder Zugkolbens 122b. Im Bereich des Bolzens 124b ist der elektrische Polleiterfortsatz 194b mit dem elektrischen Polleiter 184b elektrisch verbunden.

Der elektrische Polleiterfortsatz 194b weist einen elektronischen Polleiterfortsatzgrundkörper 202 auf. Der elektrische Polleiterfortsatz 194b weist eine Polleiterhülse 198b auf. Der elektrische Polleiterfortsatz 194b ist in der Polleiterhülse 198b eingefasst. Die Polleiterhülse 198b ist im Bereich des Bolzen 124b des Schub-und/oder Zugkolbens 122b angeordnet. Die Polleiterhülse 198b ist mit einem Polleiterfortsatzgrundkörper 202b des elektrischen Polleiterfortsatz 194b fest verbunden. Im vorliegenden Fall ist die Polleiterhülse 198b mit dem Polleiterfortsatzgrundkörper 202b verschweißt.

Der elektrische Polleiterfortsatz 194b ist wenigstens teilweise als ein flacher Streifen ausgebildet. Der Polleiterfortsatzgrundkörper 202b ist als ein flacher Streifen ausbildet. Der elektrische Polleiterfortsatz 194b ist wenigstens teilweise aus Metall ausgebildet. Bei dem Polleiterfortsatzgrundkörper 202b kann es sich beispielsweise um ein Blech handeln.

Der elektrische Polleiterfortsatz 194b ist in einer Seitenansicht hakenförmig ausgebildet. Dabei umgreift der elektrische Polleiterfortsatz 194b wenigstens teilweise eine zusätzliche Führungskulisse 176b des Schub-und/oder Zugkolbens 122b. Der elektrische Polleiterfortsatz 194b ist wenigstens teilweise als ein Blechbauteil, insbesondere Laserschneid-Blechbauteil ausgebildet. Der Polleiterfortsatzgrundkörper 202b ist ein Blechbauteil, insbesondere Laserschneid-Blechbauteil. Alternativ könnte es sich bei dem elektronischen Polleiterfortsatz um ein wenigstens teilweise generativ gefertigtes Bauteil handeln. Beispielsweise könnte der elektrische Polleiterfortsatz mittels eines Laserschmelz- und/oder Lasersinterverfahrens hergestellt sein.

Ferner weist der Schub- und/oder Zugkolben 122b wenigstens das weitere Isolationsmaterial auf. Der elektrische Polleiterfortsatz 194b ist zumindest teilweise mit dem weiteren Isolationsmaterial bedeckt. Im vorliegenden Fall ist der elektrische Polleiterfortsatz 194b sogar zu wenigstens einem Großteil mit dem weiteren Isolationsmaterial bedeckt. Das weitere Isolationsmaterial ummantelt im vorliegenden Fall den elektrischen Polleiterfortsatz 194b. Der mit dem weiteren Isolationsmaterial bedeckte elektronische Polleiterfortsatz194b bildet zumindest teilweise den Schub und/oder Zugkolben 122b aus.

Zu einer weiteren elektrischen Verbindung weist der Schub- und/oder Zugkolben 122b des Bewegungswandlers 116b wenigstens einen weiteren elektrischen Polleiterfortsatz 196b auf. Der weitere elektrische Polleiterfortsatz 196b ist mit dem weiteren elektrischen Polleiter 186b des Betätigungsstrangs 106b elektrisch verbunden. Ferner ist der weitere elektrische Polleiterfortsatz 196b mechanisch mit dem weiteren elektrischen Polleiter 186b des Betätigungsstrangs 106b verbunden.

Der weitere elektrische Polleiterfortsatz 196b erstreckt sich teilweise durch den Anker 130b des Schub-und/oder Zugkolbens 122b. Im Bereich des Ankers 130b ist der weitere elektrische Polleiterfortsatz 196b mit einer weiteren Komponente des Bewegungswandlers 116b elektrisch und/oder mechanischen verbunden. Ferner erstreckt sich der weitere elektrische Polleiterfortsatz 196b zumindest teilweise durch den Bolzen 124b des Schub und/oder Zugkolbens 122b. Im Bereich des Bolzens 122b ist der der weitere elektrische Polleiterfortsatz 196b mit dem weiteren elektrischen Polleiter 186b elektrisch verbunden.

Der weitere elektrische Polleiterfortsatz 196b weist einen weiteren Polleiterfortsatzgrundkörper 204b auf. Der weitere elektrische Polleiterfortsatz 196b weist eine weitere Polleiterhülse 198b auf. Der weitere elektrische Polleiter 186b ist in der weiteren Polleiterhülse 200b eingefasst. Die weitere Polleiterhülse 200b ist im Bereich des Bolzen 124b des Schub- und/oder Zugkolbens 122b angeordnet. Die weitere Polleiterhülse 200b ist mit einem weiteren Polleiterfortsatzgrundkörper 204b des weiteren elektrischen Polleiterfortsatzes 196b fest verbunden. Im vorliegenden Fall ist die weitere Polleiterhülse 200b mit dem weiteren Polleiterfortsatzgrundkörper 204b verschweißt.

Der weitere elektrische Polleiterfortsatz 196b ist wenigstens teilweise als ein flacher Streifen ausgebildet. Der weitere Polleiterfortsatzgrundkörper 204b ist als ein flacher Streifen ausbildet. Der weitere elektrische Polleiterfortsatz 196b ist wenigstens teilweise aus Metall ausgebildet. Bei dem weiteren Polleiterfortsatzgrundkörper 204b kann es sich beispielsweise um ein Blech handeln.

Der weitere elektrische Polleiterfortsatz 196b ist wenigstens teilweise als ein Blechbauteil, insbesondere ein Laserschneid-Blechbauteil ausgebildet. Der weitere Polleiterfortsatzgrundkörper 204b ist ein Blechbauteil, insbesondere ein Laserschneid-Blechbauteil. Alternativ könnte es sich bei dem weiteren elektrischen Polleiterfortsatz um ein wenigstens teilweise generativ gefertigtes Bauteil handeln. Beispielsweise könnte der weitere elektrische Polleiterfortsatz mittels eines Laserschmelz- und/oder Lasersinterverfahrens hergestellt sein.

Ferner weist der Schub- und/oder Zugkolben 122b wenigstens ein weiteres Isolationsmaterial auf. Im vorliegenden Fall handelt es sich um das vorbenannte weitere Isolationsmaterial. Der weitere elektrische Polleiterfortsatz 196b ist zumindest teilweise mit dem weiteren Isolationsmaterial bedeckt. Im vorliegenden Fall ist der weitere elektrische Polleiterfortsatz 196b sogar zu wenigstens einem Großteil mit dem weiteren Isolationsmaterial bedeckt. Das weitere Isolationsmaterial ummantelt im vorliegenden Fall den weiteren elektrischen Polleiterfortsatz 196b. Der mit dem weiteren Isolationsmaterial bedeckte weitere elektrische Polleiterfortsatz196b bildet zumindest teilweise den Schub und/oder Zugkolben 122b aus.

Der weitere elektrische Polleiterfortsatz 196b ist in einer Seitenansicht zu dem elektrischen Polleiterfortsatz 194b korrespondierend ausgebildet. Der weitere elektrische Polleiterfortsatz 196b erstreckt sich zumindest im Wesentlichen parallel zum elektrischen Polleiterfortsatz 194b. Der elektrische Polleiterfortsatz 194b und der weitere elektrische Polleiterfortsatz 196b sind in einer selben Ebene angeordnet. Bei der Ebene kann es sich um eine Symmetrieebene des Schub und/oder Zugkolbens 122b handeln. Der elektrische Polleiterfortsatz 194b umgreift wenigstens teilweise den weiteren elektrische Polleiterfortsatz 196b.

Im vorliegenden Fall ummantelt das weitere Isolationsmaterial gemeinsam den elektrischen Polleiterfortsatz 194b und den weiteren elektrischen Polleiterfortsatz 196b. Der elektrische Polleiterfortsatz 194b und der weitere elektrische Polleiterfortsatz 196b sind durch das weitere Isolationsmaterial elektrisch voneinander isoliert. Das weitere Isolationsmaterial, der elektrische Polleiterfortsatz 194b und der weitere Polleiterfortsatz 196b bilden den Schub- und/oder Zugkolben 122b zu wenigstens einem Großteil aus.

Der Bewegungswandler 116b weist wenigstens einen Schwenkhebel 132b auf. Der Schwenkhebel 132b ist elektrisch mit dem Schub- und/oder Zugkolben 122b verbunden. Der Schwenkhebel 132b ist elektrisch mit dem elektrischen Polleiterfortsatz 194b verbunden. Der Schwenkhebel 132b weist einen Schwenkhebelgrundkörper 134b auf. Der Schwenkhebelgrundkörper 134b ist wenigstens teilweise aus Metall ausgebildet. Der Schwenkhebelgrundkörper 134b ist elektrisch mit dem Werkzeugstück 92b verbunden. Der Schwenkhebel 132b weist wenigstens ein weiteres Isolationsmaterial auf. Im vorliegenden Fall handelt es sich um das vorbenannte weitere Isolationsmaterial. Der Schwenkhebelgrundkörper 134b ist wenigstens teilweise von dem weiteren Isolationsmaterial bedeckt. Im vorliegenden Fall ist der Schwenkhebelgrundkörper 134b wenigstens zu einem Großteil mit dem weiteren Isolationsmaterial bedeckt.

Der Bewegungswandler 116b umfasst wenigstens einen Koppelmechanismus 136b. Der Koppelmechanismus 136b weist wenigstens ein Koppelelement 138b auf. Das Koppelelement 138b ist Teil des Schub und/oder Zugkolbens 122b. Das Koppelelement 138b ist elektrisch leitend ausgebildet. Das Koppelelement 138b ist wenigstens teilweise aus Metall ausgebildet. Das Koppelelement 138b ist zumindest teilweise frei von dem weiteren Isolationsmaterial, welches den Schub- und/oder Zugkolben 122b umgibt. Ferner ist das Koppelelement 138b mit dem elektrischen Polleiterfortsatz 194b mechanisch wirkverbunden. Das Koppelelement 138b ist mit dem elektrischen Polleiterfortsatz 194b elektrisch wirkverbunden. Beispielsweise kann das Koppelelement 138b mit dem elektrischen Polleiterfortsatz 194b verschweißt sein.

Der Koppelmechanismus 136b weist wenigstens ein korrespondierendes Koppelelement 140b auf. Das korrespondierende Koppelelement 140b ist Teil eines Schwenkhebels 132b des Bewegungswandlers 116b. Das korrespondierende Koppelelement 140b ist mit einem Schwenkhebelgrundkörper 134b des Schwenkhebels 132b verbunden. Das korrespondierende Koppelelement 140b ist wenigstens teilweise frei von dem weiteren Isolationsmaterial. Das Koppelelement 138b und das korrespondierende Koppelelement 140b sind elektrisch miteinander wirkverbunden. Die aneinander anliegenden Flächen des Koppelements und des korrespondierenden Koppelelements 140b, welche vorteilhaft frei von dem weiteren Isolationsmaterial sind, bilden einen elektrischen Gleitkontakt aus.

Der Bewegungswandlers 116b weist wenigstens einen weiteren Schwenkhebel 150b auf (vgl. Fig. 9). Der weitere Schwenkhebel 150b ist elektrisch mit dem Schub- und/oder Zugkolben 122b verbunden. Der weitere Schwenkhebel 150b ist elektrisch mit dem weiteren elektrischen Polleiterfortsatz 196b verbunden. Der weitere Schwenkhebel 150b weist einen weiteren Schwenkhebelgrundkörper 152b auf. Der weitere Schwenkhebelgrundkörper 152b ist wenigstens teilweise aus Metall ausgebildet. Der weitere Schwenkhebelgrundkörper 152b ist elektrisch mit dem Werkzeugstück 92b verbunden. Der weitere Schwenkhebel 150b weist wenigstens ein weiteres Isolationsmaterial auf. Im vorliegenden Fall ist dies das vorbenannte weitere Isolationsmaterial. Der weitere Schwenkhebelgrundkörper 152b ist wenigstens teilweise von dem weiteren Isolationsmaterial bedeckt. Im vorliegenden Fall ist der weitere Schwenkhebelgrundkörper 152b wenigstens zu einem Großteil mit dem weiteren Isolationsmaterial bedeckt.

Der Koppelmechanismus 136b weist wenigstens ein weiteres Koppelelement 156b auf. Das weitere Koppelelement 156b ist Teil des Schub und/oder Zugkolben 122b. Das weitere Koppelelement 156b ist elektrisch leitend ausgebildet. Das weitere Koppelelement 156b ist wenigstens teilweise aus Metall ausgebildet. Das weitere Koppelelement 156b des Schub- und/oder Zugkolbens 122b ist zumindest teilweise frei von dem weiteren Isolationsmaterial. Das weitere Koppelelement 156b ist mit dem weiteren elektrischen Polleiterfortsatz 196b elektrisch wirkverbunden. Ferner ist das weitere Koppelelement 156b mit dem weiteren elektrischen Polleiterfortsatz 196b mechanisch wirkverbunden. Beispielsweise ist das weitere Koppelelement 156b mit dem weiteren elektrischen Polleiterfortsatz 196b verschweißt.

Der Koppelmechanismus 136b weist wenigstens ein weiteres korrespondierendes Koppelelement 158b auf. Das korrespondierendes Koppelelement 158b ist Teil des weiteren Schwenkhebels 150b. Das weitere korrespondierende Koppelelement 158b ist mit einem weiteren Schwenkhebelgrundkörper 152b des weiteren Schwenkhebels 150b verbunden. Das weitere korrespondierende Koppelelement 158b ist wenigstens teilweise frei von dem weiteren Isolationsmaterial. Das weitere Koppelelement 156b und das weitere korrespondierende Koppelelement 158b sind elektrisch miteinander wirkverbunden. Aneinander anliegende Flächen des weiteren Koppelements 156b und des weiteren korrespondierenden Koppelelements 158b, welche vorteilhaft frei von dem weiteren Isolationsmaterial sind, bilden einen elektrischen Gleitkontakt aus.

Ferner weist der Endeffektor 90b einen Endeffektorkopf 96b auf. Der Endeffektorkopf 96b ist wenigstens teilweise aus einem weiteren Isolationsmaterial, beispielsweise dem vorbenannten weiteren Isolationsmaterial, ausgebildet. Der Endeffektorkopf 96b weist einen Endeffektorgrundkörper 206b auf. Der Endeffektorgrundkörper 206b ist im vorliegenden Fall wenigstens teilweise aus einem Metall ausgebildet. Der Endeffektorgrundkörper 206b ist wenigstens zu einem Großteil mit dem weiteren Isolationsmaterial bedeckt. Im vorliegenden Fall ist der Endeffektorgrundkörper 206b vollständig mit dem weiteren Isolationsmaterial bedeckt.

Mit dem weiteren Isolationsmaterial bedeckte Komponenten der endoskopischen Vorrichtung 16b sind mit diesem fugenlos bedeckt. Dazu sind Grundkörper dieser Komponenten mit dem weiteren Isolationsmaterial umspritzt, wie beispielsweise der Endeffektorkopf, die Endeffektorgabel, der Schub- und/oder Zugkolben, der Schwenkhebel, der weitere Schwenkhebel oder dergleichen. Das weitere Isolationsmaterial schmiegt sich dabei bündig an weitere Komponenten, wie beispielsweise das Werkzeugstück an, sodass vorteilhaft Fugen vermieden werden können, in welchen sich Verunreinigungen ansammeln könnten.

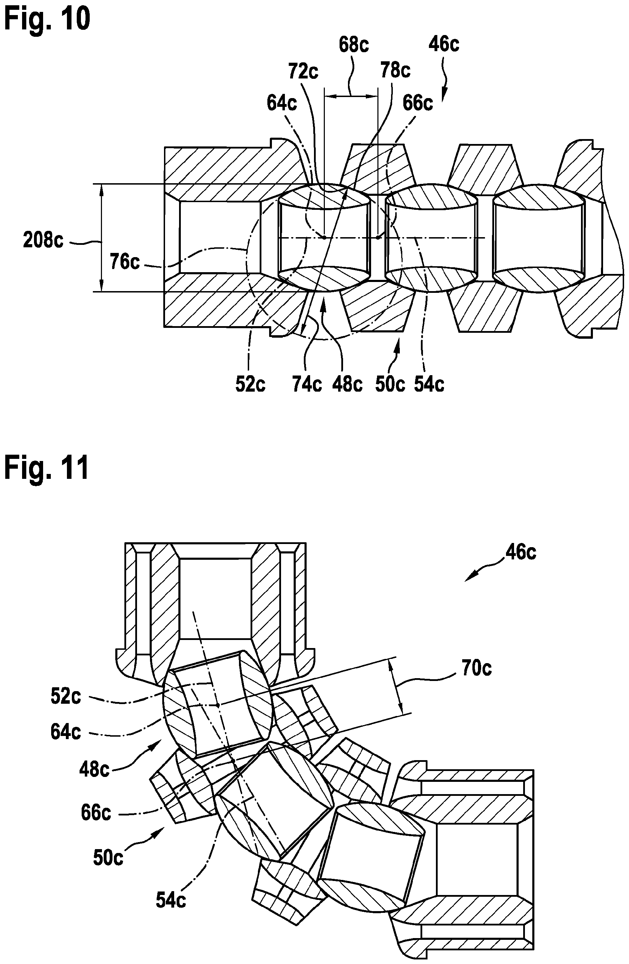

Fig. 10 zeigt eine schematische Darstellung wenigstens eines Teils einer alternativen endoskopischen Vorrichtung 16c in einer Schnittansicht längs eines Schafts 26c der endoskopischen Vorrichtung 16c gemäß den Prinzipien der vorliegenden Offenbarung in einer Schnittansicht längs eines Schafts 26c der endoskopischen Vorrichtung 16c in einer Geradenstellung. Ferner zeigt Fig. 11 eine schematische Darstellung wenigstens eines Teils der endoskopischen Vorrichtung 16c in einer Schnittansicht längs des Schafts 26c der endoskopischen Vorrichtung 16c in einer Auslenkungsstellung. Das vorliegende Ausführungsbeispiel der endoskopischen Vorrichtung 16c unterscheidet sich von dem Vorhergehenden im Wesentlichen durch eine Auslenkmechanik 46c der endoskopischen Vorrichtung 16c.

Die Auslenkmechanik 46c weist wenigstens ein erstes Verbindungsglied 48c auf. Im vorliegenden Fall weist die Auslenkmechanik 46c mehrere erste Verbindungsglieder auf. Ferner weist die Auslenkmechanik 46c wenigstens ein zweites Verbindungsglied 50c auf. Im vorliegenden Fall weist die Auslenkmechanik 46c mehrere zweite Verbindungsglieder auf.

In Fig. 10 ist die Auslenkmechanik 46c in einer Geradenstellung dargestellt. Das erste Verbindungsglied 48c und das zweite Verbindungsglied 50c sind relativ zueinander in einer Geradenstellung angeordnet. In der Geradenstellung sind eine erste Rotationssymmetrieachse 52c des ersten Verbindungsglieds 48c und eine zweite Rotationssymmetrieachse 54c des zweiten Verbindungsglieds 50c zumindest im Wesentlichen zueinander parallel ausgerichtet.

Das erste Verbindungsglied 48c weist einen ersten geometrischen Mittelpunkt 64c auf. Ferner weist das zweite Verbindungsglied 50c einen zweiten geometrischen Mittelpunkt 66c auf. In der Geradenstellung sind der erste geometrischen Mittelpunkt 64c und der zweite geometrischen Mittelpunkt 66c zueinander versetzt angeordnet.

Sind das erste Verbindungsglied 48c und das zweite Verbindungsglied 50c in der Geradenstellung angeordnet, existiert ein Geradenstellungsabstand 68c zwischen dem ersten Verbindungsglied 48c und zweiten Verbindungsglied 50c. In der Geradenstellung ist der Geradenstellungsabstand 68c definiert durch eine kürzeste Verbindung zwischen dem ersten geometrischen Mittelpunkt 64c und zweiten geometrischen Mittelpunkt 66c.

In Fig. 11 ist die Auslenkmechanik 46c in einer Auslenkungsstellung dargestellt. Das erste Verbindungsglied 48c und das zweite Verbindungsglied 50c sind relativ zueinander in einer Auslenkungsstellung angeordnet. In der Auslenkungsstellung sind die erste Rotationssymmetrieachse 52c des ersten Verbindungsglieds 48c und die zweite Rotationssymmetrieachse 54c des zweiten Verbindungsglieds 50c winklig zueinander angeordnet. In der Auslenkungsstellung weist ein Winkel zwischen der erste Rotationssymmetrieachse 52c und der zweite Rotationssymmetrieachse 54c zueinander wenigstens 10° auf. In der Auslenkungsstellung sind der erste geometrischen Mittelpunkt 64c und der zweite geometrischen Mittelpunkt 66c versetzt zueinander angeordnet.

Sind das erste Verbindungsglied 48c und das zweite Verbindungsglied 50c in der Auslenkungsstellung angeordnet, existiert ein Auslenkungsabstand 70c zwischen dem ersten Verbindungsglied 48c und zweiten Verbindungsglied 50c. In der Auslenkungsstellung ist der Auslenkungsabstand 70c definiert durch eine kürzeste Verbindung zwischen dem ersten geometrischen Mittelpunkt 64c und dem zweite geometrischen Mittelpunkt 66c. Der Auslenkungsstellungsabstand 70c ist größer als der Geradenstellungsabstand 68c.

Bei einer Auslenkung des ersten Verbindungsglieds 48c und des zweiten Verbindungsglieds 50c zueinander, wie dies beispielsweise bei einer Überführung der Verbindungsglieder von der Geradenstellung in die Auslenkungsstellung auftreten kann, sind diese dazu eingerichtet, dass deren geometrische Mittelpunkte 64c, 66c der pro Grad einer Auslenkung dieser aus der Geradenstellung heraus wenigstens um 0,3 µm zunimmt. In der Auslenkungsstellung ergibt sich eine Verlängerung der Auslenkmechanik 46c im Vergleich zur Geradenstellung. Stehen die Verbindungsglieder 48c, 50c unter Vorspannung, wie beispielsweise durch einen Steuerstrang der endoskopischen Vorrichtung 16c, so erhöht sich in der Auslenkungsstellung die Vorspannung im Vergleich zu einer Vorspannung, welche in der Geradenstellung auf die Verbindungsglieder einwirkt. Es kann eine Rückstellwirkung erzielt werden, wodurch die Verbindungsglieder automatisch in eine Geradenstellung zurückkehren.

Im vorliegenden Fall weist die Auslenkmechanik 46c drei erste Verbindungsglieder 48c auf. Ferner weist die Auslenkmechanik 46c vier zweite Verbindungsglieder 50c auf. Somit ergeben sich aufgrund der Anordnung der mehreren ersten Verbindungsglieder und der mehreren zweiten Verbindungsglieder insgesamt sechs zusammenwirkende Kombinationen aus einem ersten Verbindungsglied und einem zweiten Verbindungsglied.

Das erste Verbindungsglied 48c weist wenigstens eine Außenkontur 72c auf. Die Außenkontur 72c ist nach außen gewandt. Die Außenkontur 72c ist von konkav verschieden ausgebildet. Im vorliegenden Fall ist die Außenkontur 72c konvex. Die Außenkontur 72c beschreibt einen Kreisbogen 76c. Die Außenkontur 72c weist wenigstens abschnittsweise eine Form einer Kreisevolvente auf. Alternativ oder zusätzlich könnte die Außenkontur wenigstens abschnittsweise eine Form eines Kreisbogens, eines Zykloiden, eines Paraboloids und/oder eines Ellipsoids entsprechend ausgebildet sein.

Es existiert ein Durchmesser 74c eines kleinsten gedachten die Außenkontur 72c des ersten Verbindungsglieds 48c gerade noch vollständig einschließenden Kreisbogens 76c. Dieser Durchmesser 74c ist größer als eine maximale Verbindungsgliedbreite 208c des ersten Verbindungsglieds 48c. Die Verbindungsgliedbreite 208c ist zumindest im Wesentlichen senkrecht zur Längserstreckungsrichtung 38c eine Schafts 26c der endoskopischen Vorrichtung 16c gemessen.

Das zweite Verbindungsglied 50c weist wenigstens eine Innenkontur 78c auf. Die Innenkontur 78c ist nach innen gewandt. Die Innenkontur 78c ist von konkav verschieden ausgebildet. Ferner ist im vorliegenden Fall die Innenkontur 78c gerade. Die Innenkontur 78c ist zumindest abschnittsweise von einem Kreisbogen 76c verschieden ausgebildet. Alternativ oder zusätzlich könnte die Innenkontur wenigstens abschnittsweise eine Form eines Kreisbogens, einer Kreisevolvente, eines Zykloiden, eine Paraboloid und/oder eines Ellipsoids entsprechend ausgebildet sein.

Die Außenkontur 72c und die Innenkontur 78c liegen einander gegenüber. Die Innenkontur 78c des zweiten Verbindungsglieds 50c ist zu einem Zusammenwirken mit der Außenkontur 72c des ersten Verbindungsglieds 48c eingerichtet und umgekehrt. Die Außenkontur 72c und die Innenkontur 78c liegen höchstens abschnittsweise aneinander an.

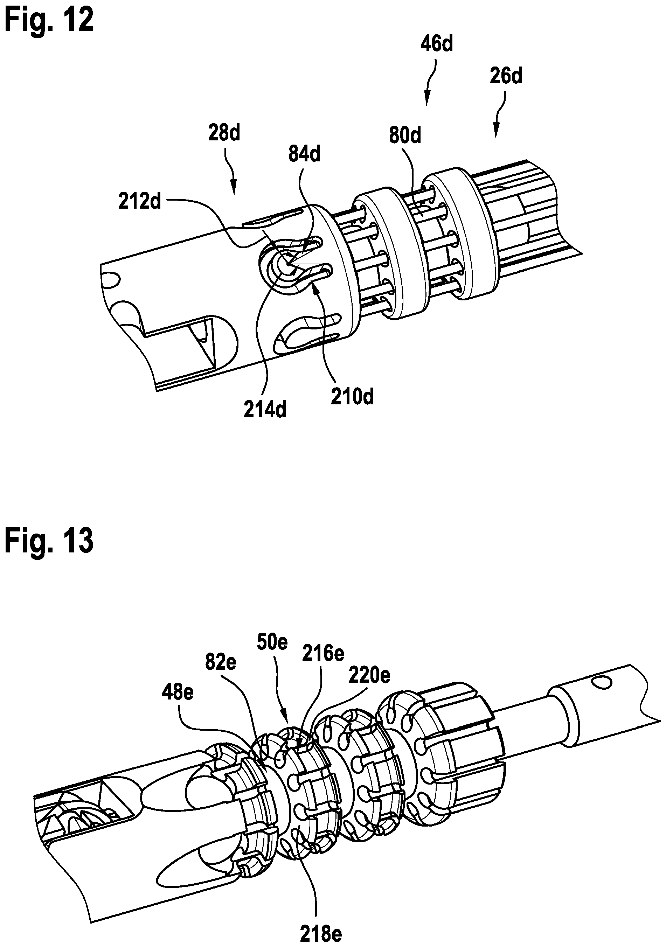

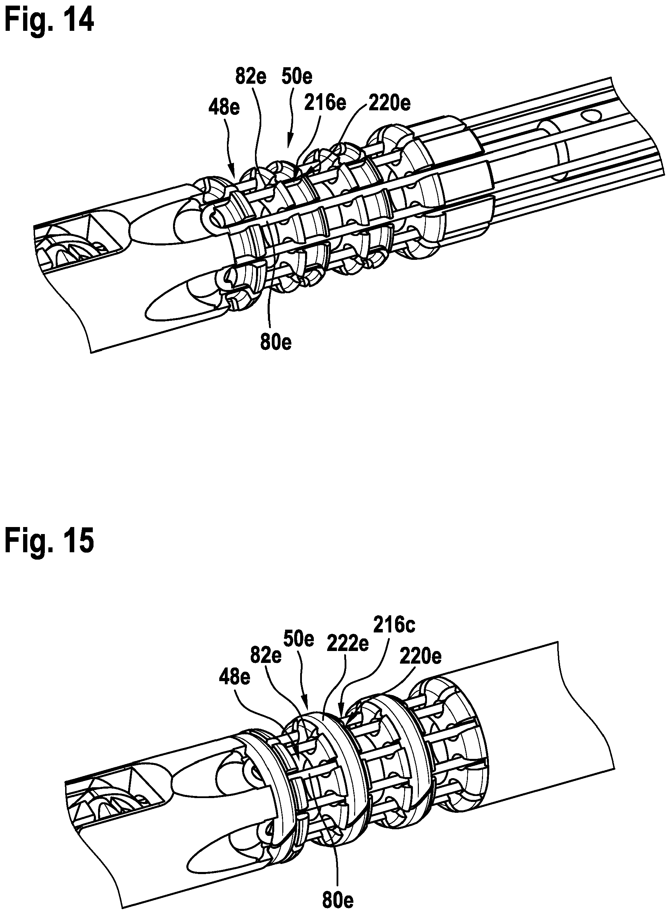

Fig. 12 zeigt eine schematische Darstellung wenigstens eines Teils eines weiteres Ausführungsbeispiel einer weiteren endoskopischen Vorrichtung 16d in einer perspektivischen Ansicht in einem Montagezustand gemäß den Prinzipien der vorliegenden Offenbarung. Ferner zeigen die Fig. 13 und 14 weitere Montagezustände der endoskopischen Vorrichtung 16d. Das vorliegende Ausführungsbeispiel der endoskopischen Vorrichtung 16d unterscheidet sich von dem Vorhergehenden im Wesentlichen durch eine Auslenkmechanik 46d der endoskopischen Vorrichtung 16d.

Die Auslenkmechanik 46d weist wenigstens einen Steuerstrang 80d auf. Der Steuerstrang 80d ist mit einem Endabschnitt 28d des Schafts 26d verbunden. Ein Teil des Steuerstrangs 80d ist im Bereich des Endabschnitts 28d des Schafts 26d eine Umschlingung 84d ausbildend angeordnet ist. Die Umschlingung 84d weist einen Umschlingungsradius 212d auf. Der Umschlingungsradius 212d ist größer als ein Durchmesser 74d des Steuerstrangs 80d. Der Umschlingungsradius 212d ist wenigstens doppelt so groß wie der Durchmesser 74d des Steuerstrangs 80d.

Der Endabschnitt 28d des Schafts 26d weist wenigstens eine Umschlingungsführung 210d auf. Der Steuerstrang 80d ist wenigstens teilweise in der Umschlingungsführung 210d angeordnet. Ein Umschlingung 84d ausbildender Abschnitt des Steuerstrangs 80d ist in der Umschlingungsführung 210d angeordnet. In einer Seitenansicht weist die Umschlingungsführung 210d eine schlüssellochartige Kontur auf. Vor der Umschlingung 84d führt eine Umschlingungsführung 210d den Steuerstrang 80d in Richtung des Endabschnitts 28d des Schafts 26d. Nach der Umschlingung 84d führt die Umschlingungsführung 210d den Steuerstrang 80d wieder entgegen dem Endabschnitts 28d des Schafts 26d.

Die Umschlingungsführung 210d führt den Steuerstrang 80d wenigstens abschnittsweise im Wesentlichen parallel zu einer Haupterstreckungsachse 120d des Schafts 26d. Es existiert ein kleinster Abstand zwischen einem zur Umschlingung 84d hingeführten Abschnitt und einem von der Umschlingung 84d zurückgeführten Abschnitt des Steuerstrangs 80d. Dieser kleinste Abstand ist kleiner als ein doppelter Umschlingungsradius 212d der Umschlingung 84d bzw. der Umschlingungsführung 210d.

Die Umschlingungsführung 210d weist einen Umfangserstreckungswinkel 214d auf. Der Umfangserstreckungswinkel 214d ist ein Winkel, welcher den radialen Winkelanteil der Umschlingung 84d beschreibt. Der Umfangserstreckungswinkel 214d beträgt mehr als 180°. Im vorliegenden Fall beträgt der Umfangserstreckungswinkel 214d wenigstens 210°.Ferner weist der Umfangserstreckungswinkel 214d einen Winkel von weniger als 360° auf. Im vorliegenden Fall beträgt der Umfangserstreckungswinkel 214d höchstens 340°.

Zu einem radialen Einlegen des Steuerstrangs 80d in die Umschlingungsführung 210d ist diese radial nach außen geöffnet. Alternativ könnte die Umschlingungsführung nach innen geöffnet sein. Auch ist denkbar, dass die Umschlingungsführung mittels einer Abdeckung radial nach außen bedeckt sein kann. Eine Abdeckung könnte dazu mit einem Endabschnitt eines Schafts koppelbar sein. Die Abdeckung bedeckt zumindest teilweise einen Endabschnitt 28d des Schafts 26d koppelbar sein, um die Umschlingungsführung 210d radial von außen zu verschließen.

Ferner weist der Endabschnitt 28d mehrere Umschlingungsführungen 210d auf, welche entlang des Umfangs des Schafts 26d zueinander versetzt angeordnet sind. Der Übersichtlichkeit halber ist von den mehreren Umschlingungsführungen nur die Umschlingungsführung 210d mit einem Bezugszeichen versehen. In den mehreren Umschlingungsführungen sind dabei mehrere Steuerstränge angeordnet. Dabei ist jeweils ein Steuerstrang 80d in einer der mehrere Umschlingungsführungen angeordnet.

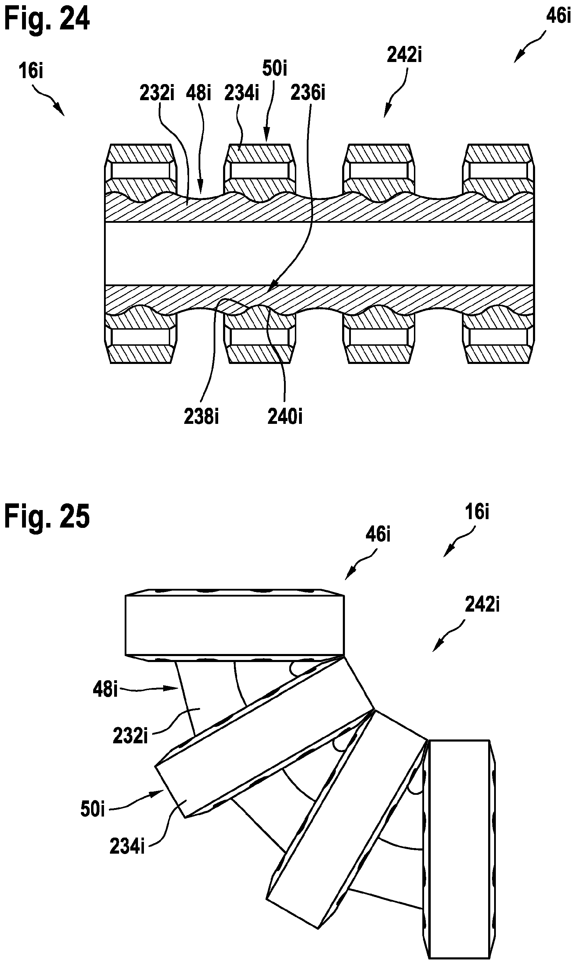

Fig. 13 zeigt eine schematische Darstellung wenigstens eines Teils einer zusätzlichen endoskopischen Vorrichtung 16e in einer perspektivischen Ansicht in einem Montagezustand gemäß den Prinzipien der vorliegenden Offenbarung. Fig. 14 zeigt eine schematische Darstellung des Teils der endoskopischen Vorrichtung 16e in einer perspektivischen Ansicht in einem zusätzlichen Montagezustand. Fig. 25 zeigt ferner eine schematische Darstellung wenigstens des Teils der weiteren endoskopischen Vorrichtung 16e in einer perspektivischen Ansicht in einem montierten Zustand. Das vorliegende Ausführungsbeispiel der weiteren endoskopischen Vorrichtung 16e unterscheidet sich von den Vorhergehenden im Wesentlichen durch eine Auslenkmechanik 46e der endoskopischen Vorrichtung 16e.

Die Auslenkmechanik 46e weist wenigstens ein erstes Verbindungsglied 48e auf. Ferner weist die Auslenkmechanik 46e wenigstens ein zweites Verbindungsglied 50e auf.

Das zweite Verbindungsglied 50e weist wenigstens ein Durchlassführung 82e auf. Ferner weist das zweite Verbindungsglied 50e wenigstens eine Radialöffnung 216e auf. Die Radialöffnung 216e ist mit der Durchlassführung 82e verbunden. Über die Radialöffnung 216e ist ein Steuerstrang 80e in die Durchlassführung 82e einlegbar.

Das zweite Verbindungsglied 50e weist wenigstens einen Verbindungsgliedgrundkörper 218e auf. Der Verbindungsgliedgrundkörper 218e weist die Radialöffnung 216e auf. Ferner weist der Verbindungsgliedgrundkörper 218e die Durchlassführung 82e. Der Verbindungsgliedgrundkörper 218e weist eine Verbindungsausnehmung 220e auf. Die Verbindungsausnehmung 220e verläuft wenigstens abschnittsweise radial. Im vorliegenden Fall verläuft die Verbindungsausnehmung 220e vollständig radial. Die Verbindungsausnehmung 220e des Verbindungsgliedgrundkörper 218e verbindet die Durchlassführung 82e und die Radialöffnung 216e miteinander.

Das zweite Verbindungsglied 50e weist zumindest ein Verschlusskörper 222e auf. Der Verschlusskörper 222e ist dazu eingerichtet, wenigstens in einem eingeführten Zustand des Steuerstrangs 80e die Radialöffnung 216e zu verschließen. Im vorliegenden Fall ist der Verschlusskörper 222e als ein Spannring ausgebildet. Der Verschlusskörper 222e ist mit dem Verbindungsgliedgrundkörper 218e verbindbar. Im vorliegenden Fall ist der Verschlusskörper 222e kraft- und/oder formschlüssig mit dem Verbindungsgliedgrundkörper 218e verbindbar. Ferner ist der Verschlusskörper 222e mit dem Verbindungsgliedgrundkörper 218e stoffschlüssig verbunden beziehungsweise verschweißt.

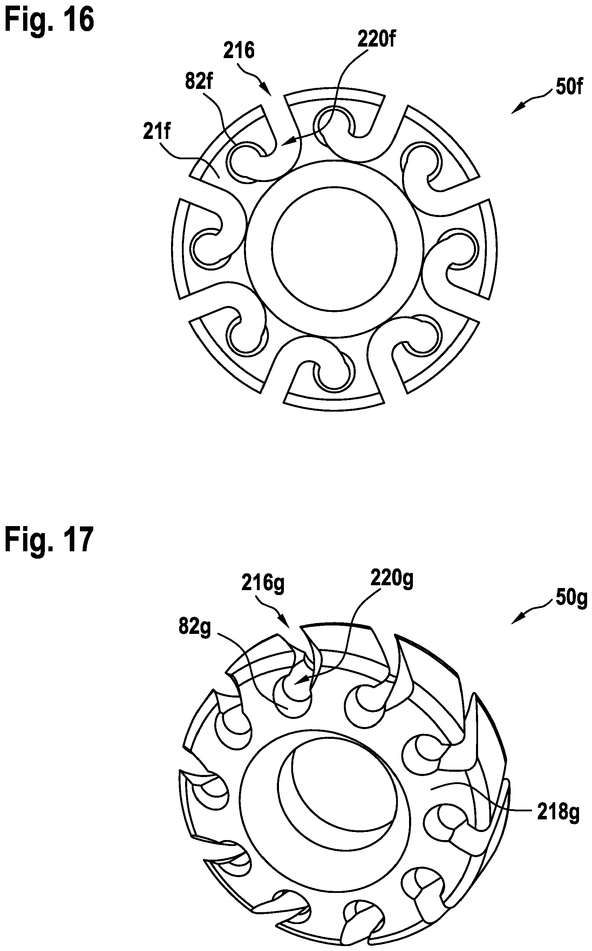

Fig. 16 zeigt eine schematische Darstellung wenigstens eines Teils einer alternativen endoskopischen Vorrichtung 16f in einer Draufsicht gemäß den Prinzipien der vorliegenden Offenbarung. Das vorliegende Ausführungsbeispiel der endoskopischen Vorrichtung 16f unterscheidet sich von dem Vorhergehenden im Wesentlichen durch eine Ausgestaltung einer Auslenkmechanik 46f der endoskopischen Vorrichtung 16f.

Ein zweites Verbindungsglied 50f der Auslenkmechanik 46f weist wenigstens einen Verbindungsgliedgrundkörper 218f auf. Der Verbindungsgliedgrundkörper 218f weist wenigstens eine Durchlassöffnung 82f auf. Ferner weist der Verbindungsgliedgrundkörper 218f wenigstens eine Radialöffnung 216f auf. Ferner weist der Verbindungsgliedgrundkörper 218f eine Verbindungsausnehmung 220f auf. Die Verbindungsausnehmung 220f verbindet die Radialöffnung 216f mit der Durchlassführung 82f.

Die Verbindungsausnehmung 220f verläuft im vorliegenden Fall abschnittsweise radial. Die Verbindungsausnehmung 220f beschreibt eine Kurvenbahn. Im vorliegenden Fall beschreibt die radial verlaufende Ausnehmung eine hakenförmige Kurvenbahn. Die Verbindungsausnehmung 220f weist die Form einer Kurvenbahn auf. Die Kurvenbahn weist einen Kurvenbahnwinkel von mehr als 90° auf. Im vorliegenden Fall weist die Kurvenbahn einen Kurvenbahnwinkel von mehr als 150° auf. Ferner weist die Kurvenbahnwinkel höchstens 180° auf. Vorteilhaft kann hier auf einen Verschlusskörper gemäß dem vorhergehenden Ausführungsbeispiel verzichtet werden.

Fig. 17 zeigt eine schematische Darstellung wenigstens eines Teils einer alternativen endoskopischen Vorrichtung 16g in perspektivischen Ansicht gemäß den Prinzipien der vorliegenden Offenbarung. Das vorliegende Ausführungsbeispiel unterscheidet sich von den Vorhergehenden im Wesentlichen durch eine Ausgestaltung einer Auslenkmechanik 46g der endoskopischen Vorrichtung 16g.

Ein zweites Verbindungsglied 50g der Auslenkmechanik 46g weist wenigstens einen Verbindungsgliedgrundkörper 218g auf. Der Verbindungsgliedgrundkörper 218g weist wenigstens ein Durchlassführung 82g auf. Ferner weist der Verbindungsgliedgrundkörper 218g wenigstens eine Radialöffnung 216g auf. Ferner weist der Verbindungsgliedgrundkörper 218g eine Verbindungsausnehmung 220g auf. Die Verbindungsausnehmung 220g verbindet die Radialöffnung 216g mit dem Führungsloch.

Die Radialöffnung 216g verläuft im vorliegenden Fall winklig zu einer Rotationssymmetrieachse des zweiten Verbindungsglieds. Ferner kann die Radialöffnung 216g einen kurvenbahnartigen verlauf aufweisen. Beispielsweise kann in einem solchen Verlauf ein kontinuierlicher Verlauf in etwa eine Cosinus-Welle entsprechen.

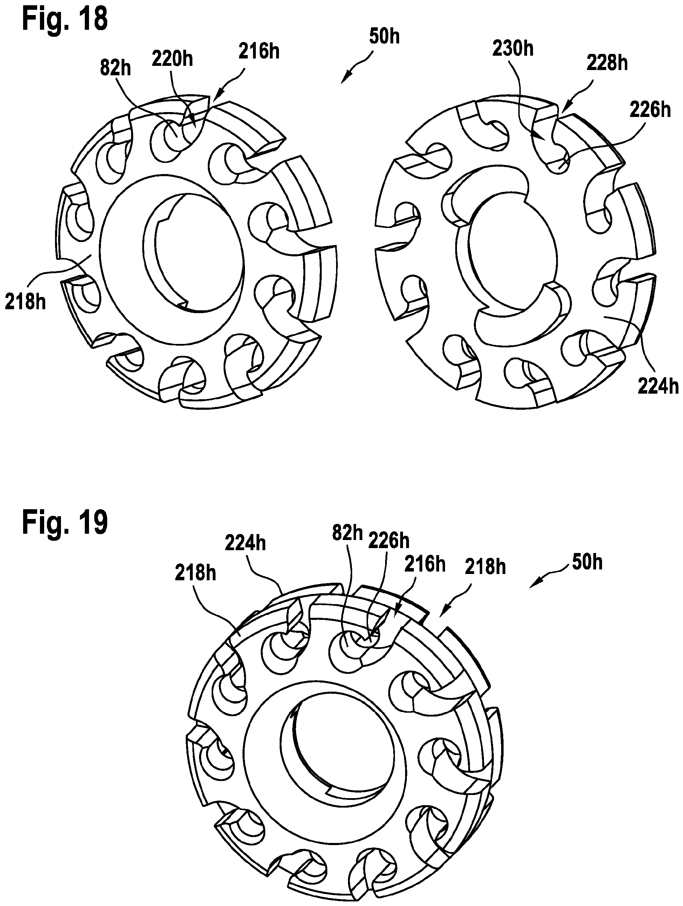

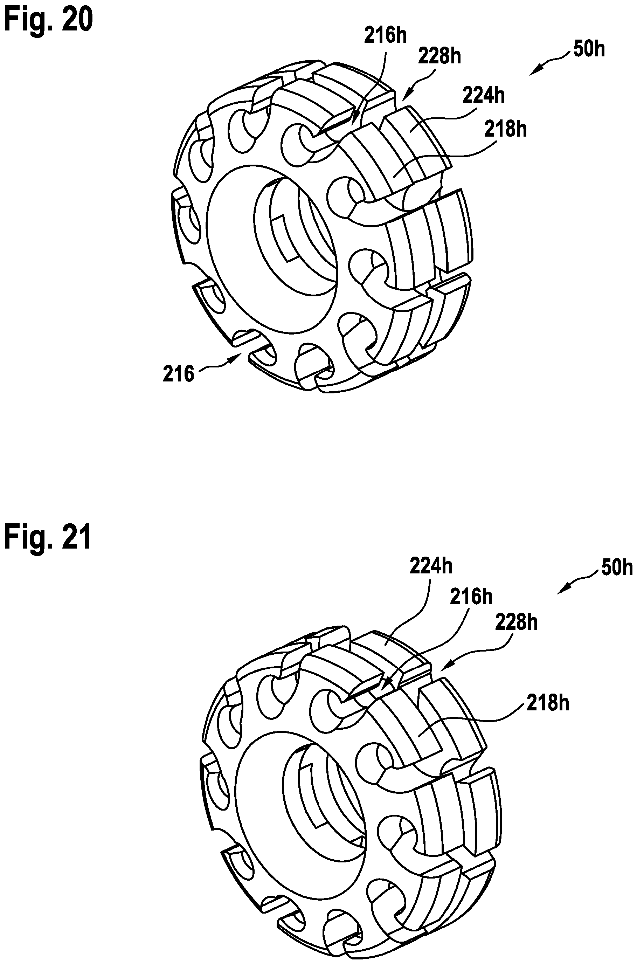

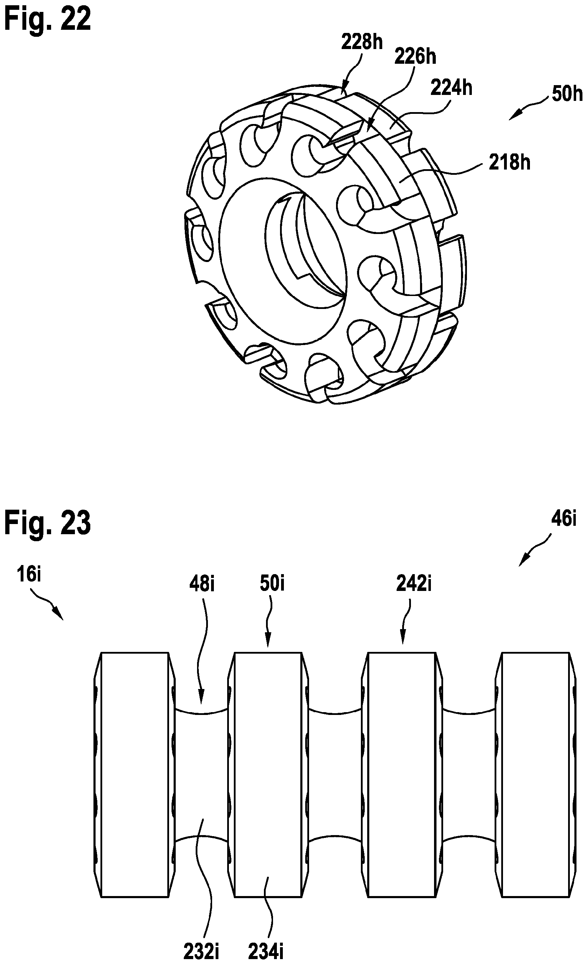

Fig. 18 zeigt eine schematische Darstellung wenigstens eines Teils einer alternativen endoskopischen Vorrichtung 16h in einer perspektivischen Ansicht in einem Montagezustand gemäß dem Prinzipien der vorliegenden Offenbarung. Fig. 19 zeigt eine schematische Darstellung des Teils der endoskopischen Vorrichtung 16h in einer perspektivischen Ansicht in einem montierten Zustand. Ferner zeigt Fig. 20 eine schematische Darstellung des Teils der endoskopischen Vorrichtung 16h in einer perspektivischen Ansicht in einem Montagezustand. Zudem zeigt Fig. 21 eine schematische Darstellung des Teils der endoskopischen Vorrichtung 16h in einer perspektivischen Ansicht in einem weiteren Montagezustand. In Fig. 22 ist eine schematische Darstellung wenigstens des Teils der endoskopischen Vorrichtung 16h in einer perspektivischen Ansicht in einem montierten Zustand gezeigt. Das vorliegende Ausführungsbeispiel der endoskopischen Vorrichtung 16h unterscheidet sich von den Vorhergehenden im Wesentlichen durch eine Ausgestaltung einer Auslenkmechanik 46h der endoskopischen Vorrichtung 16h.

Die Auslenkmechanik 46h weist ein zweites Verbindungsglied 50h auf. Das Verbindungsglied 50h umfasst wenigstens einen Verbindungsgliedgrundkörper 218h. Der Verbindungsgliedgrundkörper 218h weist wenigstens eine Durchlassführung 82h auf. Ferner weist der Verbindungsgliedgrundkörper 218h eine Radialöffnung 216h auf. Ferner umfasst der Verbindungsgliedgrundkörper 218 eine Verbindungsausnehmung 220h. Die Verbindungsausnehmung 220h verbindet die Radialöffnung 216h mit der Durchlassführung 82h.

Ein zweites Verbindungsglied weist wenigstens einen weiteren Verbindungsgliedgrundkörper 224h auf. Der weitere Verbindungsgliedgrundkörper 224h weist wenigstens eine weitere Durchlassführung 226h auf. Der Verbindungsgliedgrundkörper 218h und der weitere Verbindungsgliedgrundkörper 224h sind im vorliegenden Fall zumindest im Wesentlichen identisch zueinander ausgebildet. Ferner weist der weitere Verbindungsgliedgrundkörper 224h eine weitere Radialöffnung 228h auf. Ferner umfasst der weitere Verbindungsgliedgrundkörper 224h eine weitere Verbindungsausnehmung 230h. Die weitere Verbindungsausnehmung 230h verbindet die weitere Radialöffnung 228h mit der weiteren Durchlassführung 226h.

Der Verbindungsgliedgrundkörper 218h und der weitere Verbindungsgliedgrundkörper 224h sind miteinander koppelbar. Der Verbindungsgliedgrundkörper 218h und der weitere Verbindungsgliedgrundkörper 224h sind kraft- und/oder formschlüssig miteinander verbindbar. In einer Stellung, in welcher eine Radialöffnung 216h des Verbindungsgliedgrundkörpers 218h und die weitere Radialöffnung 228h des weiteren Verbindungsgliedgrundkörper 224h miteinander kongruent sind, sind der Verbindungsgliedgrundkörper 218h und der weitere Verbindungsgliedgrundkörper 224h voneinander getrennt.

In einer weiteren Stellung, in welcher die Durchlassführung 82h des Verbindungsgliedgrundkörper 218h und die weitere Durchlassführung 226h des weiteren Verbindungsgliedgrundkörper 224h miteinander kongruent sind, sind der Verbindungsgliedgrundkörper 218h und der weitere Verbindungsgliedgrundkörper 224h miteinander verbindbar sind. Ein Steuerstrang 80e der Auslenkmechanik 46h hält in einem montierten Zustand den Verbindungsgliedgrundkörper 218h und den weitere Verbindungsgliedgrundkörper 224h unter Vorspannung, sodass diese zusammenpresst sind. Alternativ oder zusätzlich könnten die Verbindungsgliedgrundkörper mittels eines Schnellverbinders 248h, wie beispielsweise einem Bajonettverschluss, einem Schraubverschluss oder dergleichen verbindbar sein.

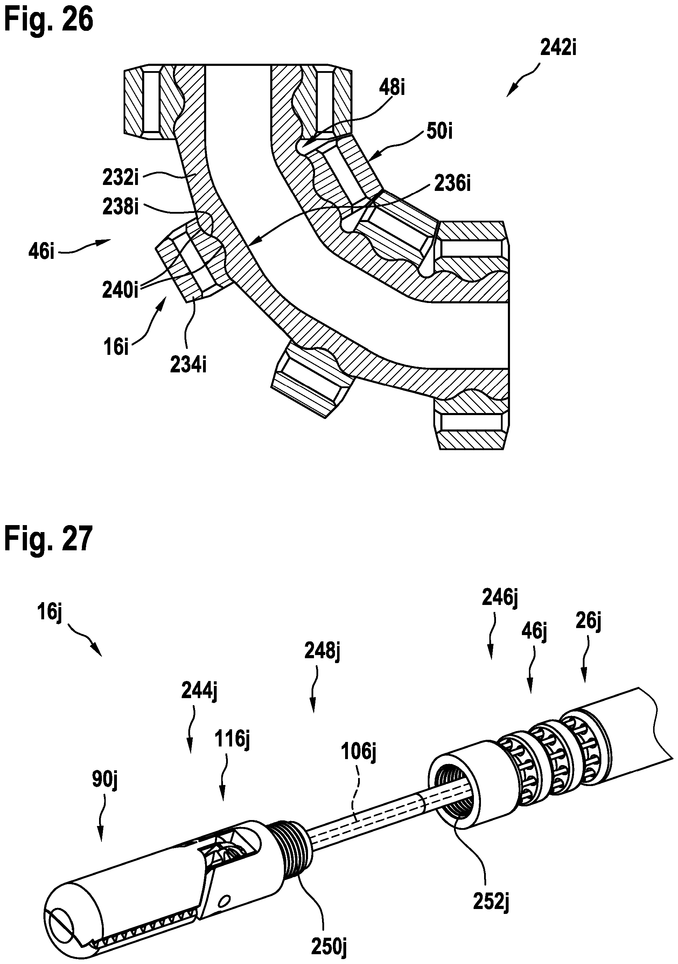

Fig. 23 zeigt eine schematische Darstellung wenigstens eines Teils einer alternativen endoskopischen Vorrichtung 16i in einer Seitenansicht in einer Geradenstellung gemäß den Prinzipien der vorliegenden Offenbarung. Ferner ist der Fig. 24 eine schematische Darstellung des Teils der endoskopischen Vorrichtung 16i aus Fig. 23 in einer Schnittansicht längs eines Schafts 26i der endoskopischen Vorrichtung 16i in der Geradestellung gezeigt. In Fig. 25 ist eine schematische Darstellung des Teils der endoskopischen Vorrichtung 16i in einer Seitenansicht in einer Auslenkungsstellung gezeigt. In Fig. 26 ist eine schematische Darstellung des Teils der endoskopischen Vorrichtung 16i in einer Schnittansicht längs des Schafts 26i der endoskopischen Vorrichtung 16i in der Auslenkungsstellung gezeigt. Das vorliegende Ausführungsbeispiel der endoskopischen Vorrichtung 16i unterscheidet sich von dem Vorhergehenden im Wesentlichen durch eine Auslenkmechanik 46i der endoskopischen Vorrichtung 16i.

Die Auslenkmechanik 46i weist wenigstens ein erstes Verbindungsglied 48i auf. Im vorliegenden Fall weist die Auslenkmechanik 46i mehrere erste Verbindungsglieder auf. Ferner weist die Auslenkmechanik 46i wenigstens ein zweites Verbindungsglied 50i auf. Im vorliegenden Fall weist die Auslenkmechanik 46i mehrere zweite Verbindungsglieder auf.

Das erste Verbindungsglied 48i ist zumindest teilweise aus einem ersten Material 232i ausgebildet. Das erste Material 232i ist der Stoffgruppe der Kunststoffe zugeordnet. Im vorliegenden Fall ist das erste Material 232i ein Elastomer. Das erste Material 232i weist eine erste Elastizität auf.

Das zweite Verbindungsglied 50i ist zumindest teilweise aus einem zweiten Material 234i ausgebildet. Das zweite Material 234i ist der Stoffgruppe der Kunststoffe zugeordnet. Das zweite Material 234i ist ein Thermoplast. Alternativ könnte es sich bei dem zweiten Material auch um ein Metall, eine Keramik oder dergleichen handeln.

Das zweite Material 234i weist eine zweite Elastizität auf. Die zweite Elastizität des zweiten Materials 234i unterscheidet sich von der ersten Elastizität des ersten Materials 232i. Im vorliegenden Fall ist eine Elastizität des ersten Materials 232i größer als eine Elastizität des zweiten Materials 234i.

Das zweite Verbindungsglied 50i ist das erste Verbindungsglied 48i zumindest teilweise koaxial umgebend angeordnet. Das erste Verbindungsglied 48i ist schlauchartig ausgebildet. Das zweite Verbindungsglied 50i ringartig ausgebildet.

Das erste Verbindungsglied 48i und das zweite Verbindungsglied 50i sind wenigstens formschlüssig miteinander verbunden. Das erste Verbindungsglied 48i und das zweite Verbindungsglied 50i greifen in einem Eingriffsbereich 236i zumindest teilweise ineinander ein. Das erste Verbindungsglied 48i weist zur Verbindung dieser mit dem zweiten Verbindungsglied 50i eine erste Profilierung 238i auf. Im vorliegenden Fall weist die Profilierung 238i die Form einer Undulation auf. Das zweite Verbindungsglied 50i weist zur Verbindung mit dem ersten Verbindungsglied 48i eine zweite Profilierung 240i. Die zweite Profilierung 240i ist korrespondierend zur ersten Profilierung 238i ausgebildet. Zu einer wenigstens formschlüssigen Verbindung des ersten Verbindungsglieds 48i und des zweiten Verbindungslieds 50i, greifen die erste Profilierung 238i und die zweite Profilierung 240i ineinander ein und bilden den Eingriffsbereich 236i aus.

Ferner sind das erste Verbindungsglied 48i und das zweite Verbindungsglied 50i wenigstens stoffschlüssig miteinander verbunden. Beispielsweise könnten das erste Verbindungsglied 48i und das zweite Verbindungsglied 50i miteinander verklebt sein. Im vorliegenden Fall sind das erste Verbindungsglied 48i und das zweite Verbindungsglied 50i jedoch miteinander umspritzt. Derart bilden zumindest das erste Verbindungsglied 48i und das zweite Verbindungsglied 50i wenigstens teilweise eine Mehrkomponenten-Spritzguss Baugruppe 242i der endoskopischen Vorrichtung 16i aus.

Im vorliegenden Fall sind die mehreren ersten Verbindungsglieder miteinander einstückig ausgebildet. Die mehreren ersten Verbindungsglieder bilden gemeinsam einen Schlauch aus. Die Haupterstreckung des Schlauchs entspricht zumindest im Wesentlichen einer Haupterstreckung einer Auslenkmechanik 46i der endoskopischen Vorrichtung 16i. Um den Schlauch sind dann die mehreren zweiten Verbindungsglieder jeweils versetzt zueinander angeordnet. Zusammen bilden die mehreren ersten Verbindungsglieder und die mehreren zweiten Verbindungsglieder somit die Mehrkomponenten-Spritzguss Baugruppe 242i aus.

Fig. 27 zeigt eine schematische Darstellung wenigstens eines Teils einer weiteren endoskopischen Vorrichtung 16j in einer perspektivischen Ansicht gemäß den Prinzipien der vorliegenden Offenbarung Das vorliegende Ausführungsbeispiel der endoskopischen Vorrichtung 16j unterscheidet sich von den vorhergehenden im Wesentlichen durch einen modularen Aufbau der endoskopischen Vorrichtung 16j.

Die endoskopische Vorrichtung 16j weist wenigstens ein Endeffektormodul 244j auf. Das Endeffektormodul 244j umfasst wenigstens einen Endeffektor 90j. Ferner weist das Endeffektormodul 244j einen Betätigungsstrang 106j auf. Zudem weist das Endeffektormodul 244j einen Bewegungswandler 116j auf. Das Endeffektormodul 244j ist als ein Mehrweg-Modul ausgebildet ist. Beispielsweise ist das Endeffektormodul 244j autoklavierbar eingerichtet, sodass es nach einem Eingriff gereinigt und somit mehrfach verwendet werden kann. Alternativ könnte das Endeffektormodul als ein Einweg-Modul ausgebildet sein. Beispielsweise könnte das Endeffektormodul nicht für einen Autoklaviervorgang ausgelegt sein. Denkbar wäre es, dass das Einweg-Modul bei dem Versuch einer Mehrfachverwendung gezielt einen Defekt aufweist, welcher dessen Funktion behindert oder eine Mehrfachverwendung erkennt und indiziert.

Die endoskopische Vorrichtung 16j umfasst ferner wenigstens ein Schaftmodul 246j. Das Schaftmodul 246j weist wenigstens den Schaft 26j auf. Ferner weist das Schaftmodul 246j eine Auslenkmechanik 46j auf. Das Schaftmodul 246j ist als ein Einweg-Modul ausgebildet. Beispielsweise könnte das Schaftmodul 246j nicht für einen Autoklaviervorgang ausgelegt sein. Denkbar wäre es, dass das Einweg-Modul bei dem Versuch einer Mehrfachverwendung gezielt einen Defekt aufweist, welcher dessen Funktion behindert oder eine Mehrfachverwendung erkennt und indiziert. Alternativ könnte das Schaftmodul als ein Mehrweg-Modul ausgebildet sein. Beispielsweise ist das Schaftmodul autoklavierbar eingerichtet, so dass es nach einem Eingriff gereinigt und somit mehrfach verwendet werden kann. Ferner kann das Schaftmodul 246j alle Komponenten der endoskopischen Vorrichtung 16j aufweisen, welche nicht bereits dem Endeffektormodul 244j zugeordnet sind.

Das Endeffektormodul 244j und das Schaftmodul 246j sind austauschbar miteinander verbindbar. Die endoskopische Vorrichtung 16j umfasst wenigstens einen Schnellverbinder 248j. Im vorliegenden Fall ist der Schnellverbinder 248j als ein Schraubverbinder ausgebildet. Alternativ könnte es sich bei dem Schnellverbinder auch um eine Schnappverbindung, Klemmverbindung, eine Bajonettverbindung oder dergleichen handeln.

Der Schnellverbinder 248j weist ein Schnellverbinderstück 250j auf. Ferner weist der Schnellverbinder 248j ein zu dem Schnellverbinderstück 250j korrespondierendes Schnellverbinderstück 252j auf. Das Schnellverbinderstück 250j ist im vorliegenden Fall ein Gewindestück. Das Schnellverbinderstück 250j weist ein Innengewinde auf. Das korrespondierende Schnellverbinderstück 252j ist im vorliegenden Fall ein korrespondierendes Gewindestück. Das korrespondierende Schnellverbinderstück 252j weist ein Außengewinde auf.

Der Schnellverbinder 248j ist zumindest teilweise von dem Endeffektor 90j einstückig verbunden. Ein Endeffektorkopf 96j des Endeffektors 90j ist einstückig mit dem Schnellverbinder 248j ausgebildet. Der Endabschnitt 28j des Schafts 26j weist im vorliegenden Fall das korrespondierende Schnellverbinderstück 252j auf. Ferner ist der Schnellverbinder 248j zumindest teilweise von einem Endeffektorkopf 96j des Endeffektors 90j ausgebildet ist. Der Endeffektorkopf 96j weist im vorliegenden Fall das korrespondierende Schnellverbinderstück 252j auf.

Um eine Austauschbarkeit und somit eine variable Einsetzbarkeit zu erzielen, weist die endoskopische Vorrichtung 16j wenigstens ein oder mehrere weitere Endeffektormodule auf. Ferner kann die endoskopische Vorrichtung 16j wenigstens ein oder mehrere weitere Schaftmodule 246j auf.

| | | | |
|---|---|---|---|
| 10 | Chirurgiesystem | 66 | Zweiter geometrischer Mittelpunkt |
| 12 | Chirurgieroboter | 68 | Geradenstellungsabstand |
| 14 | Steuergerät | 70 | Auslenkungsstellungsabstand |
| 16 | Endoskopische Vorrichtung | 72 | Außenkontur |
| 18 | Roboterarm | 74 | Durchmesser |
| 20 | Endoskopisches Instrument | 76 | Kreisbogen |
| 22 | Endoskop | 78 | Innenkontur |
| 26 | Schaft | 80 | Steuerstrang |
| 28 | Endabschnitt | 82 | Durchlassführung |
| 30 | Weiterer Endabschnitt | 84 | Umschlingung |
| 32 | Mittelabschnitt | 86 | Strangaufnahme |
| 34 | Grundgerüst | 88 | Durchlassführung |
| 36 | Schaftmantel | 90 | Endeffektor |
| 38 | Längserstreckungsrichtung | 92 | Werkzeugstück |
| 40 | Längserstreckung | 94 | Weiteres Werkzeugstück |
| 42 | Auslenkbarer Abschnitt | 96 | Endeffektorkopf |
| 44 | Ebene | 98 | Endeffektorgabel |
| 46 | Auslenkmechanik | 100 | Endeffektorschenkel |
| 48 | Erstes Verbindungsglied | 102 | Weiterer Endeffektorschenkel |
| 50 | Zweites Verbindungsglied | 104 | Endeffektorbuchse |
| 52 | Erste Rotationssymmetrieachse | 106 | Betätigungsstrang |
| 54 | zweite Rotationssymmetrieachse | 108 | Flexibler Abschnitt |
| 56 | Manschette | 110 | Unflexibler Abschnitt |
| 58 | Weitere Manschette | 112 | Innentrosse |
| 60 | Gelenkkopf | 114 | Verstärkung |
| 62 | Gelenkpfanne | 116 | Bewegungswandler |
| 64 | erster geometrischer Mittelpunkt | 118 | Schwenkachse |
| 120 | Haupterstreckungsachse | 174 | Weitere Kulissenführung |
| 122 | Schub- und/oder Zugkolben | 176 | Zusätzliche Kulissenführung |
| 124 | Bolzen | 178 | Führungszapfen |
| 126 | Kolbenführung | 180 | Zapfenaufnahme |
| 128 | Betätigungsstrangaufnahme | 182 | weitere Zapfenaufnahme |
| 130 | Anker | 184 | Elektrischer Polleiter |
| 132 | Schwenkhebel | 186 | Weiterer elektrischer Polleiter |
| 134 | Schwenkhebelgrundkörper | 188 | Außentrosse |
| 136 | Koppelmechanismus | 190 | Elektrischer Isolator |
| 138 | Koppelelement | 192 | weiterer elektrischer Isolator |
| 140 | Korrespondierendes Koppelelement | 194 | Elektrische Polleiterfortsatz |
| 142 | Drehlager | 196 | Weiterer elektrischer Polleiterfortsatz |
| 144 | Lagerelement | 198 | Polleiterhülse |
| 146 | Korrespondierendes Lagerelement | 200 | Weitere Polleiterhülse |
| 148 | Drehachse | 202 | Polleiterfortsatzgrundkörper |
| 150 | Weiterer Schwenkhebel | 204 | Weiterer Polleiterfortsatzgrundkörper |
| 152 | Weiterer Schwenkhebelgrundkörper | 206 | Endeffektorgrundkörper |
| 154 | Weiterer Koppelmechanismus | 208 | Verbindungsgliedbreite |
| 156 | Weiteres Koppelelement | 210 | Umschlingungsführung |
| 158 | Weiteres korrespondierendes Koppelelement | 212 | Umschlingungsradius |
| | | 214 | Umfangserstreckungswinkel |
| 160 | Weitere Schwenkachse | 216 | Radialöffnung |
| 162 | Weiteres Drehlager | 218 | Verbindungsgliedgrundkörper |
| 164 | Weiteres Lagerelement | 220 | Verbindungsausnehmung |
| 166 | Weiteres Korrespondierendes Lagerelement | 222 | Verschlusskörper |
| 168 | Weiteres Drehachse | 224 | Weiterer Verbindungsgliedgrundkörper |
| 170 | Führungslager | 226 | Weitere Durchlassführung |
| 172 | Kulissenführung | 228 | Weitere Radialöffnung |
| 230 | Weitere Verbindungsausnehmung | 244 | Endeffektormodul |
| 232 | Erstes Material | 246 | Schaftmodul |
| 234 | Zweites Material | 248 | Schnellverbinder |
| 236 | Eingriffsbereich | 250 | Schnellverbinderstück |
| 238 | Erste Profilierung | 252 | Korrespondierendes Schnellverbinderstück |
| 240 | Zweite Profilierung | | |
| 242 | Mehrkomponenten-Spritzguss Baugruppe | | |

## Patentansprüche

1. Endoskopische Vorrichtung (16a-j) mit wenigstens einem Schaft (26a; 26b; 26c; 26d; 26i; 26j), welcher wenigstens einen in zumindest einer Ebene (44a) auslenkbaren Abschnitt (42a) aufweist, und mit wenigstens einer Auslenkmechanik (46a-j), welche zur Auslenkung des auslenkbaren Abschnitts (42a) eingerichtet ist und in Reihe angeordnet zumindest ein erstes Verbindungsglied (48a; 48c; 48e; 48i) und zumindest ein zu einer Auslenkung mit dem ersten Verbindungsglied (48a; 48c; 48e; 48i) zusammenwirkendes zweites Verbindungsglied (50a; 50c; 50e; 50f; 50g; 50h; 50i) umfasst und wenigstens einen Steuerstrang (80a; 80d; 80e), welcher zu einer Verstellung einer Auslenkung des auslenkbaren Abschnitts (42a) mit den Verbindungsgliedern (48a; 48c; 48e; 48i; 50a; 50c; 50e; 50f; 50g; 50h; 50i) gekoppelt und mit einem Endabschnitt (28a; 28d; 28j) des Schafts (26a; 26b; 26c; 26d; 26i; 26j) verbunden ist,
**dadurch gekennzeichnet, dass**
wenigstens ein Teil des Steuerstrangs (80a; 80d; 80e) im Bereich des Endabschnitts (28a; 28d; 28j) des Schafts (26a; 26b; 26c; 26d; 26i; 26j) eine Umschlingung (84a; 84d) ausbildend angeordnet ist, der Endabschnitt (28a; 28d; 28j) des Schafts (26a; 26b; 26c; 26d; 26i; 26j) wenigstens eine Strangaufnahme (86a) aufweist, in welcher wenigstens ein die Umschlingung (84a; 84d) ausbildender Teil des Steuerstrangs (80a; 80d; 80e) angeordnet ist und die Strangaufnahme (86a) eine hufeisenartige und/oder schlüssellochartige Kontur aufweist.

2. Endoskopische Vorrichtung (16a-j) nach Anspruch 1, **dadurch gekennzeichnet, dass** ein Umschlingungsradius (212d) der Umschlingung (84a; 84d) größer ist als ein Durchmesser (74a; 74c) des Steuerstrangs (80a; 80d; 80e).

3. Endoskopische Vorrichtung (16a-j) nach Anspruch 1 oder 2, **dadurch gekennzeichnet, dass** die Strangaufnahme (86a) einen Umfangserstreckungswinkel (214d) von mehr als 180° und/oder von weniger als 360° beschreibt.

4. Endoskopische Vorrichtung (16a-j) nach einem der vorhergehenden Ansprüche, **dadurch gekennzeichnet, dass** der Steuerstrang (80a) mittels der Strangaufnahme (86a) wenigstens abschnittsweise im Wesentlichen parallel zu einer Längserstreckungsrichtung des Schaftes (26a; 26b; 26c; 26d; 26i; 26j) geführt ist.

5. Endoskopische Vorrichtung (16a-j) nach einem der vorhergehenden Ansprüche, **dadurch gekennzeichnet, dass** der Steuerstrang (80a; 80d; 80e) mittels der Strangaufnahme (86a) in Richtung des Endabschnitts (28a; 28d; 28j) des Schafts (26a; 26b; 26c; 26d; 26i; 26j) geführt ist und nach der Umschlingung (84a; 84d) wieder entgegen dem Endabschnitts (28a; 28d; 28j) des Schafts (26a; 26b; 26c; 26d; 26i; 26j) geführt ist.

6. Endoskopische Vorrichtung (16a-j) nach einem der vorhergehenden Ansprüche, **dadurch gekennzeichnet, dass** ein Abstand zwischen einem hingeführten Abschnitt und einem zurückgeführten Abschnitt des Steuerstrangs (80a; 80d; 80e) existiert, welcher kleiner ist als ein doppelter Umschlingungsradius (212d) der Umschlingung (84a; 84d).

7. Endoskopische Vorrichtung (16a-j) nach einem der vorhergehenden Ansprüche, **dadurch gekennzeichnet, dass** die Strangaufnahme (86a) zu einem radialen Einlegen des Steuerstrangs (80a; 80d; 80e) zumindest teilweise radial nach außen geöffnet ist.

8. Endoskopische Vorrichtung (16a-j) nach einem der vorhergehenden Ansprüche, **gekennzeichnet, durch** wenigstens eine mit dem Endabschnitt (28a; 28d; 28j) des Schafts (26a; 26b; 26c; 26d; 26i; 26j) koppelbare Abdeckung, welche in einem gekoppelten Zustand die Strangaufnahme (86a) radial von außen zumindest teilweise bedeckt.

9. Endoskopische Vorrichtung (16a-j) nach einem der vorhergehenden Ansprüche, **gekennzeichnet durch** wenigstens einen weiteren Steuerstrang (80a; 80d; 80e), welcher parallel und entlang eins Umfangs des Schafts (26a; 26b; 26c; 26d; 26i; 26j) versetzt zum Steuerstrang (80a; 80d; 80e) angeordnet ist und im Bereich des Endabschnitts (28a; 28d; 28j) des Schafts (26a; 296b; 26c; 26d; 26i; 26j) wenigstens eine weitere Umschlingung (84a; 84d) ausbildend angeordnet ist.

10. Endoskop (22a) mit wenigstens einer endoskopischen Vorrichtung (16a-j) nach einem der Ansprüche 1 bis 9.

11. Endoskopisches Instrument (20a) mit wenigstens einer endoskopischen Vorrichtung (16a-j) nach einem der Ansprüche 1 bis 9.

12. Chirurgiesystem (10a) mit wenigstens einer endoskopischen Vorrichtung (16a-j) nach einem der Ansprüche 1 bis 9 und mit wenigstens einem Chirurgieroboter (12a).

13. Verfahren zur Herstellung einer endoskopischen Vorrichtung (16a-j) nach einem der Ansprüche 1 bis 9.

## Claims

1. Endoscopic device (16a-j) having at least one shaft (26a; 26b; 26c; 26d; 26i; 26j), which has at least one portion (42a) deflectable in at least one plane (44a), and having at least one deflection mechanism (46a-j), which is configured to deflect the deflectable portion (42a) and comprises, arranged in series, at least one first connection member (48a; 48c; 48e; 48i) and at least one second connection member (50a; 50c; 50e; 50f; 50g; 50h; 50i) interacting for a deflection with the first connection member (48a; 48c; 48e; 48i), and at least one control train (80a; 80d; 80e) which, in order to adjust a deflection of the deflectable portion (42a), is coupled to the connection members (48a; 48c; 48e; 48i; 50a; 50c; 50e; 50f; 50g; 50h; 50i) and is connected to an end portion (28a; 28d; 28j) of the shaft (26a; 26b; 26c; 26d, 26i; 26j), **characterized in that** at least one part of the control train (80a; 80d; 80e) in the region of the end portion (28a; 28d; 28j) of the shaft (26a; 26b; 26c; 26d; 26i; 26j) is arranged to form a loop-back (84a; 84d), the end portion (28a; 28d; 28j) of the shaft (26a; 26b; 26c; 26d; 26i; 26j) has at least one train receptacle (86a) in which at least one part of the control train (80a; 80d; 80e) forming the loop-back (84a; 84d) is arranged, and the train receptacle (86a) has a horseshoe-shaped and/or keyhole-shaped contour.

2. Endoscopic device (16a-j) according to Claim 1, **characterized in that** a loop-back radius (212d) of the loop-back (84a; 84d) is greater than a diameter (74a; 74c) of the control train (80a; 80d; 80e).

3. Endoscopic device (16a-j) according to Claim 1 or 2, **characterized in that** the train receptacle (86a) describes an angle of circumferential extent (214d) of more than 180° and/or of less than 360°.

4. Endoscopic device (16a-j) according to one of the preceding claims, **characterized in that** the control train (80a) is guided by means of the train receptacle (86a) at least in part substantially parallel to a direction of longitudinal extent of the shaft (26a; 26b; 26c; 26d; 26i; 26j).

5. Endoscopic device (16a-j) according to one of the preceding claims, **characterized in that** the control train (80a; 80d; 80e) is guided by means of the train receptacle (86a) in the direction of the end portion (28a; 28d; 28j) of the shaft (26a; 26b; 26c; 26d; 26i; 26j) and, after the loop-back (84a; 84d), is guided back from the end portion (28a; 28d; 28j) of the shaft (26a; 26b; 26c; 26d; 26i; 26j).

6. Endoscopic device (16a-j) according to one of the preceding claims, **characterized in that** a distance is present between a guided-in portion and a guided-back portion of the control train (80a; 80d; 80e), which distance is smaller than twice the loop-back radius (212d) of the loop-back (84a; 84d).

7. Endoscopic device (16a-j) according to one of the preceding claims, **characterized in that** the train receptacle (86a) is opened at least in part radially outwards in order to permit radial insertion of the control train (80a; 80d; 80e).

8. Endoscopic device (16a-j) according to one of the preceding claims, **characterized by** at least one covering which can be coupled to the end portion (28a; 28d; 28j) of the shaft (26a; 26b; 26c; 26d; 26i; 26j) and which, in a coupled state, at least partially covers the train receptacle (86a) radially from the outside.

9. Endoscopic device (16a-j) according to one of the preceding claims, **characterized by** at least one further control train (80a; 80d; 80e) which is arranged in parallel and along a circumference of the shaft (26a; 26b; 26c; 26d; 26i; 26j), offset in relation to the control train (80a; 80d; 80e), and is arranged in the region of the end portion (28a; 28d; 28j) of the shaft (26a; 26b; 26c; 26d; 26i; 26j) to form at least one further loop-back (84a; 84d).

10. Endoscope (22a) having at least one endoscopic device (16a-j) according to one of Claims 1 to 9.

11. Endoscopic instrument (20a) having at least one endoscopic device (16a-j) according to one of Claims 1 to 9.

12. Surgical system (10a) having at least one endoscopic device (16a-j) according to one of Claims 1 to 9 and having at least one surgical robot (12a).

13. Method for producing an endoscopic device (16a-j) according to one of Claims 1 to 9.

## Revendications

1. Dispositif endoscopique (16a-j) avec au moins une tige (26a ; 26b ; 26c ; 26d ; 26i ; 26j), qui présente au moins une section (42a) pouvant être déviée dans au moins un plan (44a), et avec au moins un mécanisme de déviation (46a-j), qui est adapté pour dévier la section pouvant être déviée (42a) et qui comprend, agencés en série, au moins un premier élément de liaison (48a ; 48c ; 48e ; 48i) et au moins un deuxième élément de liaison (50a ; 50c ; 50e ; 50f ; 50g ; 50h ; 50i) coopérant avec le premier élément de liaison (48a ; 48c ; 48e ; 48i) pour une déviation et au moins un brin de commande (80a ; 80d ; 80e) qui est couplé aux éléments de liaison (48a ; 48c ; 48e ; 48i ; 50a ; 50c ; 50e ; 50f ; 50g ; 50h ; 50i) pour un réglage d'une déviation de la section pouvant être déviée (42a) et qui est relié à une section d'extrémité (28a ; 28d ; 28j) de la tige (26a ; 26b ; 26c ; 26d ; 26i ; 26j),
**caractérisé en ce que**
au moins une partie du brin de commande (80a ; 80d ; 80e) est agencée dans la zone de la section d'extrémité (28a ; 28d ; 28j) de la tige (26a ; 26b ; 26c ; 26d ; 26i ; 26j) en formant un enroulement (84a ; 84d), la section d'extrémité (28a ; 28d ; 28j) de la tige (26a ; 26b ; 26c ; 26d ; 26i ; 26j) présente au moins un logement de brin (86a) dans lequel est agencée au moins une partie du brin de commande (80a ; 80d ; 80e) formant l'enroulement (84a ; 84d) et le logement de brin (86a) présente un contour en forme de fer à cheval et/ou en forme de trou de serrure.

2. Dispositif endoscopique (16a-j) selon la revendication 1, **caractérisé en ce qu'**un rayon d'enroulement (212d) de l'enroulement (84a ; 84d) est supérieur à un diamètre (74a ; 74c) du brin de commande (80a ; 80d ; 80e).

3. Dispositif endoscopique (16a-j) selon la revendication 1 ou 2, **caractérisé en ce que** le logement de brin (86a) décrit un angle d'extension périphérique (214d) supérieur à 180° et/ou inférieur à 360°.

4. Dispositif endoscopique (16a-j) selon l'une quelconque des revendications précédentes, **caractérisé en ce que** le brin de commande (80a) est guidé au moyen du logement de brin (86a), au moins par sections, essentiellement parallèlement à une direction d'extension longitudinale de la tige (26a ; 26b ; 26c ; 26d ; 26i ; 26j).

5. Dispositif endoscopique (16a-j) selon l'une quelconque des revendications précédentes, **caractérisé en ce que** le brin de commande (80a ; 80d ; 80e), au moyen du logement de brin (86a), est guidé en direction de la section d'extrémité (28a ; 28d ; 28j) de la tige (26a ; 26b ; 26c ; 26d ; 26i ; 26j) et, après l'enroulement (84a ; 84d), est à nouveau guidé à l'encontre de la section d'extrémité (28a ; 28d ; 28j) de la tige (26a ; 26b ; 26c ; 26d ; 26i ; 26j).

6. Dispositif endoscopique (16a-j) selon l'une quelconque des revendications précédentes, **caractérisé en ce qu'**une distance existe entre une section avancée et une section rétractée du brin de commande (80a ; 80d ; 80e), qui est inférieure au double du rayon d'enroulement (212d) de l'enroulement (84a ; 84d).

7. Dispositif endoscopique (16a-j) selon l'une quelconque des revendications précédentes, **caractérisé en ce que** le logement de brin (86a) est au moins partiellement ouvert radialement vers l'extérieur pour une insertion radiale du brin de commande (80a ; 80d ; 80e) .

8. Dispositif endoscopique (16a-j) selon l'une quelconque des revendications précédentes, **caractérisé par** au moins un couvercle pouvant être couplé à la section d'extrémité (28a ; 28d ; 28j) de la tige (26a ; 26b ; 26c ; 26d ; 26i ; 26j), qui, dans un état couplé, recouvre au moins partiellement le logement de brin (86a) radialement de l'extérieur.

9. Dispositif endoscopique (16a-j) selon l'une quelconque des revendications précédentes, **caractérisé par** au moins un autre brin de commande (80a ; 80d ; 80e) qui est agencé parallèlement à une périphérie de la tige (26a ; 26b ; 26c ; 26d ; 26i ; 26j) et le long de celle-ci, en décalage par rapport au brin de commande (80a ; 80d ; 80e) et qui est agencé dans la zone de la section d'extrémité (28a ; 28d ; 28j) de la tige (26a ; 296b ; 26c ; 26d ; 26i ; 26j) en formant au moins un autre enroulement (84a ; 84d).

10. Endoscope (22a) avec au moins un dispositif endoscopique (16a-j) selon l'une quelconque des revendications 1 à 9.

11. Instrument endoscopique (20a) avec au moins un dispositif endoscopique (16a-j) selon l'une quelconque des revendications 1 à 9.

12. Système chirurgical (10a) avec au moins un dispositif endoscopique (16a-j) selon l'une quelconque des revendications 1 à 9 et avec au moins un robot chirurgical (12a).

13. Procédé de fabrication d'un dispositif endoscopique (16a-j) selon l'une quelconque des revendications 1 à 9.
